(19) Europäisches Patentamt
European Patent Office
Office européen des brevets

(11) EP 0 648 765 B1

(12) **EUROPÄISCHE PATENTSCHRIFT**

(45) Veröffentlichungstag und Bekanntmachung des Hinweises auf die Patenterteilung:
**12.07.2000 Patentblatt 2000/28**

(51) Int. Cl.[7]: **C07D 405/04**, C07D 307/83, C07D 333/22, C07D 307/91, C08K 5/15

(21) Anmeldenummer: **94810521.8**

(22) Anmeldetag: **08.09.1994**

(54) **Verfahren zur Herstellung von 3-Arylbenzofuranonen**
Process for the production of 3-arylbenzofuranones
Procédé pour la préparation de 3-arylebenzofuranones

(84) Benannte Vertragsstaaten:
**AT BE CH DE ES FR GB IT LI NL SE**

(30) Priorität: **17.09.1993 CH 281293**

(43) Veröffentlichungstag der Anmeldung:
**19.04.1995 Patentblatt 1995/16**

(73) Patentinhaber:
**Ciba Specialty Chemicals Holding Inc.**
**4057 Basel (CH)**

(72) Erfinder:
- **Nesvadba, Peter, Dr.**
  **CH-1723 Marly (CH)**
- **Evans, Samuel, Dr.**
  **CH-1723 Marly (CH)**
- **Schmitt, Ralf, Dr.**
  **CH-4450 Sissach (CH)**

(56) Entgegenhaltungen:
**EP-A- 0 589 839** **US-A- 4 325 863**
**US-A- 4 338 244** **US-A- 5 175 312**

- **CHEM. BER. ZINCKE Bd. 10, Seite 996**
- **FUSON ET AL: "", J.AMER.CHEM.SOC., , 1944, Band 66, Nr. , Seiten 1274 - 1275**
- **VOL.813: 'Ullmann's Encyclopedia of Industrial Chemistry', 1989, VCH, BRD * Seite 524 - Seite 525 ***

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

**Beschreibung**

Die vorliegende Erfindung betrifft ein neues Verfahren zur Herstellung von 3-Aryl-benzofuranonen, die zum Stabilisieren von organischen Materialien gegen oxidativen, thermischen oder lichtinduzierten Abbau geeignet sind.

Einzelne Benzofuran-2-one sind in der Literatur bekannt, und wurden beispielsweise in Beilstein 18, 17 und Beilstein E III/IV, 18, 154-166 erwähnt oder von Th. Kappe et al., Monatshefte für Chemie 99, 990 (1968); J. Morvan et al., Bull. Soc. Chim. Fr. 1979, 583; L. F. Clarke et al., J. Org. Chem. 57, 362 (1992); M. Julia et al., Bull. Soc. Chim. Fr. 1965, 2175 oder H. Sterk et al., Monatshefte für Chemie 99, 2223 (1968) beschrieben. In keiner Publikation werden diese Verbindungen als Stabilisatoren für organische Materialien verwendet.

Die Verwendung von einigen 3-Phenyl-3H-benzofuran-2-onen als Stabilisatoren für organische Polymere ist beispielsweise aus US-A-4 325 863; US-A-4 338 244 und US-A-5 175 312 bekannt.

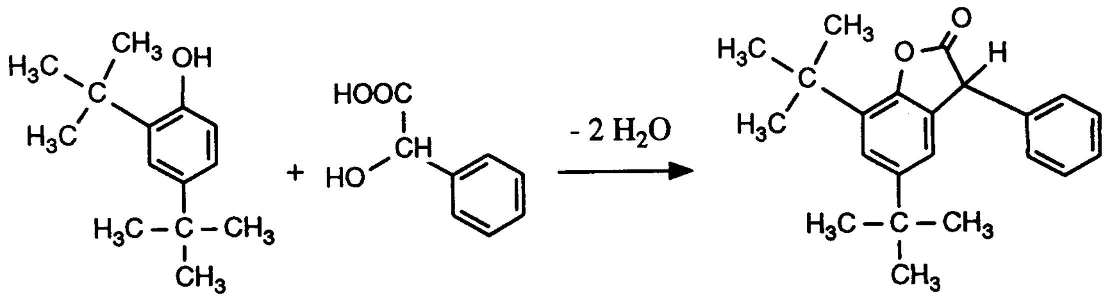


(A) (B) (C)

Das bis jetzt bevorzugte Verfahren zur Herstellung von 3-Phenyl-3H-benzofuran-2-onen, wie beispielsweise 5,7-Di-tert-butyl-3-phenyl-3H-benzofuran-2-on der Formel C, ist dadurch gekennzeichnet, dass das 2,4-Di-tert-butyl-phenol der Formel A mit der Mandelsäure der Formel B unter Abspaltung von Wasser zur Reaktion gebracht wird (siehe US-A-4 325 863, Beispiel 1, Kolonne 8, Zeilen 35-45).

Für die Synthese von 3-Phenyl-3H-benzofuran-2-onen, die am 3-Phenylring substituiert sind oder die Herstellung von 3H-benzofuran-2-onen, die in 3-Stellung mit einem Heterocyclus substituiert sind, hat das Verfahren den Nachteil, dass am Phenylring substituierte Mandelsäuren oder heterocyclische Mandelsäuren verwendet werden müssen. Von diesen sind jedoch nicht sehr viele in der Literatur bekannt und die bekannten Synthesen zur Herstellung von diesen Mandelsäuren sind relativ aufwendig.

Die vorliegende Erfindung betrifft daher ein Verfahren zur Herstellung von Verbindungen der Formel I

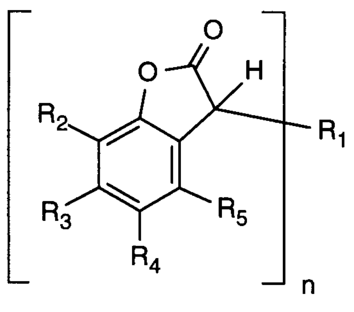

(I)

worin, wenn n 1 ist,

$R_1$ unsubstituiertes oder durch Chlor, Amino, Hydroxy, $C_1$-$C_{18}$-Alkyl, $C_1$-$C_{18}$-Alkoxy, $C_1$-$C_{18}$-Alkylthio, $C_3$-$C_4$-Alkenyloxy, $C_3$-$C_4$-Alkinyloxy, $C_1$-$C_4$-Alkylamino, Di-($C_1$-$C_4$-Alkyl)amino, Phenyl, Benzyl, Benzoyl oder Benzoyloxy substituiertes Phenyl, Naphthyl, Phenanthryl, Anthryl, 5,6,7,8-Tetrahydro-2-naphthyl, Thienyl, Benzo[b]thienyl, Naphtho[2,3-b]thienyl, Thiathrenyl, Furyl, Benzofuryl, Isobenzofuryl, Dibenzofuryl, Chromenyl, Xanthenyl, Phenoxathiinyl, Pyrrolyl, Imidazolyl, Pyrazolyl, Pyrazinyl, Pyrimidinyl, Pyridazinyl, Indolizinyl, Isoindolyl, Indolyl,

Indazolyl, Purinyl, Chinolizinyl, Isochinolyl, Chinolyl, Phtalazinyl, Naphthyridinyl, Chinoxalinyl, Chinazolinyl,Cinnolinyl, Pteridinyl, Carbazolyl, β-Carbolinyl, Phenanthridinyl, Acridinyl, Perimidinyl, Phenanthrolinyl, Phenazinyl, Isothiazolyl, Phenothiazinyl, Isoxazolyl, Furazanyl, Biphenyl, Tetralinyl, Fluorenyl oder Phenoxazinyl darstellt, oder $R_1$ einen Rest der Formel V oder VI

(V) 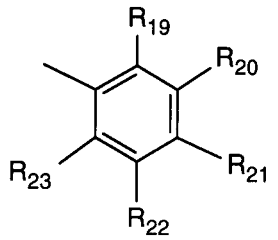

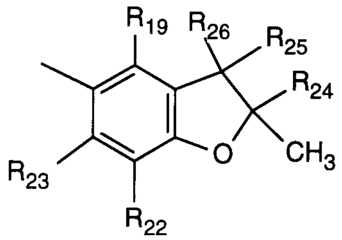 (VI)

bedeutet,
wenn n 2 ist,
$R_1$ unsubstituiertes oder mit $C_1$-$C_4$-Alkyl oder Hydroxy substituiertes Phenylen oder Naphthylen; oder $-R_6$-X-$R_7$- darstellt,
$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{18}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>N-R_8$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich $-(CH_2)_p-COR_9$ oder $-(CH_2)_qOH$ darstellt, oder wenn $R_3$ und $R_5$ Wasserstoff sind, $R_4$ zusätzlich einen Rest der Formel II

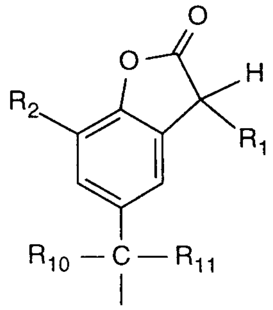 (II)

bedeutet, worin $R_1$ wie oben für n = 1 angegeben, definiert ist,
$R_6$ und $R_7$ unabhängig voneinander unsubstituiertes oder durch $C_1$-$C_4$-Alkyl substituiertes Phenylen oder Naphthylen darstellen,
$R_8$ Wasserstoff oder $C_1$-$C_8$-Alkyl ist,
$R_9$ Hydroxy, $[-O^{\theta} \frac{1}{r} M^{r+}]$, $C_1$-$C_{18}$-Alkoxy oder

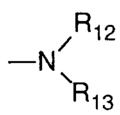

bedeutet,
$R_{10}$ und $R_{11}$ unabhängig voneinander Wasserstoff, $CF_3$, $C_1$-$C_{12}$-Alkyl oder Phenyl darstellen, oder $R_{10}$ und $R_{11}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden:

$R_{12}$ und $R_{13}$ unabhängig voneinander Wasserstoff oder $C_1$-$C_{18}$-Alkyl darstellen,

$R_{14}$ Wasserstoff oder $C_1$-$C_{18}$-Alkyl bedeutet,

$R_{19}$, $R_{20}$, $R_{21}$, $R_{22}$ und $R_{23}$ unabhängig voneinander Wasserstoff, Halogen, Hydroxy, $C_1$-$C_{25}$-Alkyl, durch Sauerstoff, Schwefel oder >N—$R_8$ unterbrochenes $C_2$-$C_{25}$-Alkyl;

$C_1$-$C_{25}$-Alkoxy, durch Sauerstoff, Schwefel oder >N—$R_8$ unterbrochenes $C_2$-$C_{25}$-Alkoxy; $C_1$-$C_{25}$-Alkylthio, $C_3$-$C_{25}$-Alkenyl, $C_3$-$C_{25}$-Alkenyloxy, $C_3$-$C_{25}$-Alkinyl, $C_3$-$C_{25}$-Alkinyloxy, $C_7$-$C_9$-Phenylalkyl, $C_7$-$C_9$-Phenylalkoxy, unsubstituiertes oder durch $C_1$-$C_4$-Alkyl substituiertes Phenyl; unsubstituiertes oder durch $C_1$-$C_4$-Alkyl substituiertes Phenoxy; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes $C_5$-$C_8$-Cycloalkoxy; $C_1$-$C_4$-Alkylamino, Di-($C_1$-$C_4$-alkyl)amino, $C_1$-$C_{25}$-Alkanoyl, durch Sauerstoff, Schwefel oder >N—$R_8$ unterbrochenes $C_3$-$C_{25}$-Alkanoyl; $C_1$-$C_{25}$-Alkanoyloxy, durch Sauerstoff, Schwefel oder >N-$R_8$ unterbrochenes $C_3$-$C_{25}$-Alkanoyloxy; $C_1$-$C_{25}$-Alkanoylamino, $C_3$-$C_{25}$-Alkenoyl, durch Sauerstoff, Schwefel oder >N—$R_8$ unterbrochenes $C_3$-$C_{25}$-Alkenoyl;

$C_3$-$C_{25}$-Alkenoyloxy, durch Sauerstoff, Schwefel oder >N—$R_8$ unterbrochenes $C_3$-$C_{25}$-Alkenoyloxy; $C_6$-$C_9$-Cycloalkylcarbonyl, $C_6$-$C_9$-Cycloalkylcarbonyloxy, Benzoyl oder durch $C_1$-$C_{12}$-Alkyl substituiertes Benzoyl; Benzoyloxy oder durch $C_1$-$C_{12}$-Alkyl substituiertes Benzoyloxy;

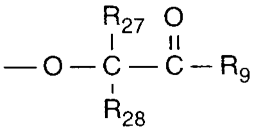 , 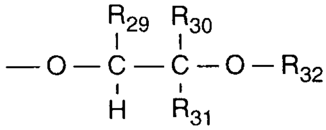

darstellen, oder ferner in Formel V die Reste $R_{19}$ und $R_{20}$ oder die Reste $R_{20}$ und $R_{21}$ zusammen mit den Kohlenstoffatomen, an die sie gebunden sind, einen Benzoring bilden,

$R_{24}$ Wasserstoff, $C_1$-$C_4$-Alkyl, unsubstituiertes oder durch $C_1$-$C_4$-Alkyl substituiertes Phenyl bedeutet,

$R_{25}$ und $R_{26}$ Wasserstoff, $C_1$-$C_4$-Alkyl oder Phenyl bedeuten, mit der Bedingung, dass mindestens einer der Reste $R_{25}$ und $R_{26}$ Wasserstoff ist,

$R_{27}$ und $R_{28}$ unabhängig voneinander Wasserstoff, $C_1$-$C_4$-Alkyl oder Phenyl darstellen,

$R_{29}$ Wasserstoff oder $C_1$-$C_4$-Alkyl ist,

$R_{30}$ Wasserstoff, unsubstituiertes oder durch $C_1$-$C_4$-Alkyl substituiertes Phenyl; $C_1$-$C_{25}$-Alkyl, durch Sauerstoff, Schwefel oder >N—$R_8$ unterbrochenes $C_2$-$C_{25}$-Alkyl; unsubstituiertes oder am Phenylrest durch 1 bis 3 $C_1$-$C_4$-Alkyl substituiertes $C_7$-$C_9$-Phenylalkyl; durch Sauerstoff, Schwefel oder >N-$R_8$ unterbrochenes unsubstituiertes oder am Phenylrest durch 1 bis 3 $C_1$-$C_4$-Alkyl substituiertes $C_7$-$C_{25}$-Phenylalkyl bedeutet, oder ferner die Reste $R_{29}$ und $R_{30}$ zusammen mit den Kohlenstoffatomen, an die sie gebunden sind, einen unsubstituierten oder durch 1 bis 3 $C_1$-$C_4$-Alkyl substituierten $C_5$-$C_{12}$-Cycloalkylenring bilden;

$R_{31}$ Wasserstoff oder $C_1$-$C_4$-Alkyl darstellt,

$R_{32}$ Wasserstoff, $C_1$-$C_{25}$-Alkanoyl, $C_3$-$C_{25}$-Alkenoyl, durch Sauerstoff, Schwefel oder >N—$R_8$ unterbrochenes $C_3$-$C_{25}$-Alkanoyl; durch eine Di($C_1$-$C_6$-alkyl)phosphonatgruppe substituiertes $C_2$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl;

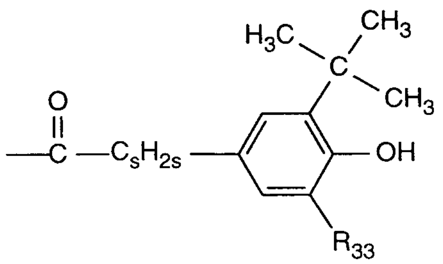 ,

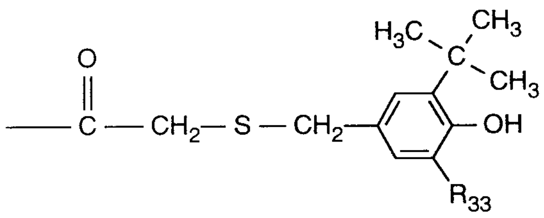 , 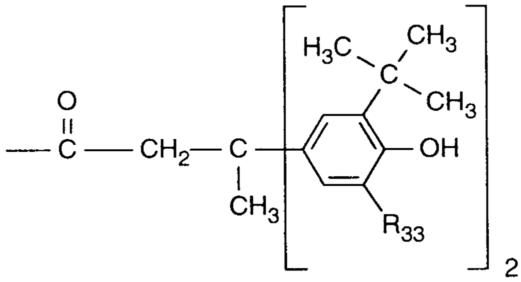

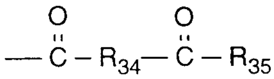 oder 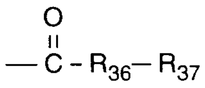

bedeutet,

$R_{33}$ Wasserstoff oder $C_1$-$C_8$-Alkyl darstellt,

$R_{34}$ eine direkte Bindung, $C_1$-$C_{18}$-Alkylen, durch Sauerstoff, Schwefel oder >N—$R_8$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

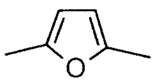 oder 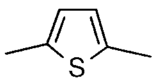

darstellt,

$R_{35}$ Hydroxy, $[-O^{\theta}\ \frac{1}{r}\ M^{r+}]$ , $C_1$-$C_{18}$-Alkoxy oder

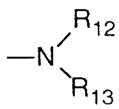

bedeutet,

$R_{36}$ Sauerstoff, -NH- oder

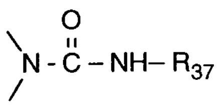

darstellt,

$R_{37}$ $C_1$-$C_{18}$-Alkyl oder Phenyl ist,

M ein r-wertiges Metallkation ist,

X eine direkte Bindung, Sauerstoff, Schwefel oder -$NR_{14}$- darstellt,

n 1 oder 2 ist,

p 0, 1 oder 2 bedeutet,

q 1, 2, 3, 4, 5 oder 6 darstellt,

r 1, 2 oder 3 ist, und

s 0, 1 oder 2 bedeutet,

dadurch gekennzeichnet, dass eine Verbindung der Formel III

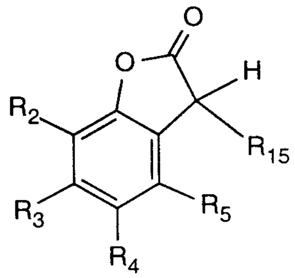 (III)

worin

$R_{15}$ Halogen oder $-OR'_{15}$ darstellt,

$R'_{15}$ Wasserstoff, $C_1$-$C_{25}$-Alkanoyl, $C_3$-$C_{25}$-Alkenoyl, durch Sauerstoff, Schwefel oder $>N—R_8$ unterbrochenes $C_3$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl; Naphthoyl oder durch $C_1$-$C_{12}$-Alkyl substituiertes Naphthoyl; $C_1$-$C_{25}$-Alkansulfonyl, durch Fluor substituiertes $C_1$-$C_{25}$-Alkansulfonyl; Phenylsulfonyl oder durch $C_1$-$C_{12}$-Alkyl substituiertes Phenylsulfonyl;

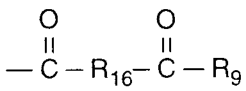 oder 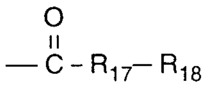

bedeutet,

$R_{16}$ eine direkte Bindung, $C_1$-$C_{18}$-Alkylen, durch Sauerstoff, Schwefel oder $>N—R_8$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

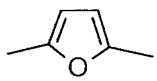 oder 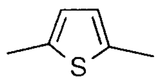

darstellt,

$R_{17}$ Sauerstoff, -NH- oder

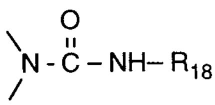

bedeutet, und

$R_{18}$ $C_1$-$C_{18}$-Alkyl oder Phenyl ist,

mit einer Verbindung der Formel IV

$$[H]_n\text{-}R_1 \qquad \text{(IV)}$$

umgesetzt wird.

Halogen bedeutet beispielsweise Chlor, Brom oder Iod. Bevorzugt ist Chlor.

Alkanoyl mit bis zu 25 Kohlenstoffatomen bedeutet einen verzweigten oder unverzweigten Rest wie beispielsweise Formyl, Acetyl, Propionyl, Butanoyl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Heptadecanoyl, Octadecanoyl, Eicosanoyl oder Docosanoyl. $R'_{15}$ als Alkanoyl hat vorzugsweise 2 bis 18, insbesondere 2 bis 12, z.B. 2 bis 6 Kohlenstoffatome. Besonders bevorzugt ist Acetyl.

Durch eine Di($C_1$-$C_6$-alkyl)phosphonatgruppe substituiertes $C_2$-$C_{25}$-Alkanoyl bedeutet beispielsweise

$(CH_3CH_2O)_2POCH_2CO$-, $(CH_3O)_2POCH_2CO$-, $(CH_3CH_2CH_2CH_2O)_2POCH_2CO$-,$(CH_3CH_2O)_2POCH_2CH_2CO$-, $(CH_3O)_2POCH_2CH_2CO$-,$(CH_3CH_2CH_2CH_2O)_2POCH_2CH_2CO$-, $(CH_3CH_2O)_2PO(CH_2)_4CO$-, $(CH_3CH_2O)_2PO(CH_2)_8CO$- oder $(CH_3CH_2O)_2PO(CH_2)_{17}CO$-.

Alkanoyloxy mit bis zu 25 Kohlenstoffatomen bedeutet einen verzweigten oder unverzweigten Rest wie beispielsweise Formyloxy, Acetoxy, Propionyloxy, Butanoyloxy, Pentanoyloxy, Hexanoyloxy, Heptanoyloxy, Octanoyloxy, Nonanoyloxy, Decanoyloxy, Undecanoyloxy, Dodecanoyloxy, Tridecanoyloxy, Tetradecanoylxoy, Pentadecanoyloxy, Hexadecanoyloxy, Heptadecanoyloxy, Octadecanoyloxy, Eicosanoyloxy oder Docosanoyloxy. Bevorzugt ist Alkanoyloxy mit 2 bis 18, insbesondere 2 bis 12, z.B. 2 bis 6 Kohlenstoffatomen. Besonders bevorzugt ist Acetoxy.

Alkenoyl mit 3 bis 25 Kohlenstoffatomen bedeutet einen verzweigten oder unverzweigten Rest wie beispielsweise Propenoyl, 2-Butenoyl, 3-Butenoyl, Isobutenoyl, n-2,4-Pentadienoyl, 3-Methyl-2-butenoyl, n-2-Octenoyl, n-2-Dodecenoyl, iso-Dodecenoyl, Oleoyl, n-2-Octadecenoyl oder n-4-Octadecenoyl. Bevorzugt ist Alkenoyl mit 3 bis 18, insbesondere 3 bis 12, z.B. 3 bis 6, vor allem 3 bis 4 Kohlenstoffatomen.

Durch Sauerstoff, Schwefel oder $>N—R_8$ unterbrochenes $C_3$-$C_{25}$-Alkenoyl bedeutet beispielsweise $CH_3OCH_2CH_2CH=CHCO$- oder $CH_3OCH_2CH_2OCH=CHCO$-.

Alkenoyloxy mit 3 bis 25 Kohlenstoffatomen bedeutet einen verzweigten oder unverzweigten Rest wie beispielsweise Propenoyloxy, 2-Butenoyloxy, 3-Butenoyloxy, Isobutenoyloxy, n-2,4-Pentadienoyloxy, 3-Methyl-2-butenoyloxy, n-2-Octenoyloxy, n-2-Dodecenoyloxy, iso-Dodecenoyloxy, Oleoyloxy, n-2-Octadecenoyloxy oder n-4-Octadecenoyloxy. Bevorzugt ist Alkenoyloxy mit 3 bis 18, insbesondere 3 bis 12, z.B. 3 bis 6, vor allem 3 bis 4 Kohlenstoffatomen.

Durch Sauerstoff, Schwefel oder $>N—R_8$ unterbrochenes $C_3$-$C_{25}$-Alkenoyloxy bedeutet beispielsweise $CH_3OCH_2CH_2CH=CHCOO$- oder $CH_3OCH_2CH_2OCH=CHCOO$-.

Durch Sauerstoff, Schwefel oder $>N—R_8$ unterbrochenes $C_3$-$C_{25}$-Alkanoyl bedeutet beispielsweise $CH_3$-O-$CH_2CO$-, $CH_3$-S-$CH_2CO$-, $CH_3$-NH-$CH_2CO$-, $CH_3$-N($CH_3$)-$CH_2CO$-, $CH_3$-O-$CH_2CH_2$-O-$CH_2CO$-, $CH_3$-(O-$CH_2CH_2$-$)_2$O-$CH_2CO$-,$CH_3$-(O-$CH_2CH_2$-$)_3$O-$CH_2CO$-oder $CH_3$-(O-$CH_2CH_2$-$)_4$O-$CH_2CO$-.

Durch Sauerstoff, Schwefel oder $>N—R_8$ unterbrochenes $C_3$-$C_{25}$-Alkanoyloxy bedeutet beispielsweise $CH_3$-O-$CH_2COO$-, $CH_3$-S-$CH_2COO$-, $CH_3$-NH-$CH_2COO$-, $CH_3$-N($CH_3$)-$CH_2COO$-, $CH_3$-O-$CH_2CH_2$-O-$CH_2COO$-, $CH_3$-(O-$CH_2CH_2$-$)_2$O-$CH_2COO$-, $CH_3$-(O-$CH_2CH_2$-$)_3$O-$CH_2COO$- oder $CH_3$-(O-$CH_2CH_2$-$)_4$O-$CH_2COO$-.

$C_6$-$C_9$-Cycloalkylcarbonyl bedeutet beispielsweise Cyclopentylcarbonyl, Cyclohexylcarbonyl, Cycloheptylcarbonyl oder Cyclooctylcarbonyl. Cyclohexylcarbonyl ist bevorzugt.

$C_6$-$C_9$-Cycloalkylcarbonyloxy bedeutet beispielsweise Cyclopentylcarbonyloxy, Cyclohexylcarbonyloxy, Cycloheptylcarbonyloxy oder Cyclooctylcarbonyloxy. Cyclohexylcarbonyloxy ist bevorzugt.

Durch $C_1$-$C_{12}$-Alkyl substituiertes Benzoyl, das vorzugsweise 1 bis 3, insbesondere 1 oder 2 Alkylgruppen trägt, bedeutet beispielsweise o-, m- oder p-Methylbenzoyl, 2,3-Dimethylbenzoyl, 2,4-Dimethylbenzoyl, 2,5-Dimethylbenzoyl, 2,6-Dimethylbenzoyl, 3,4-Dimethylbenzoyl, 3,5-Dimethylbenzoyl, 2-Methyl-6-ethylbenzoyl, 4-tert-Butylbenzoyl, 2-Ethylbenzoyl, 2,4,6-Trimethylbenzoyl, 2,6-Dimethyl-4-tert-butylbenzoyl oder 3,5-Di-tert-butylbenzoyl. Bevorzugte Substituenten sind $C_1$-$C_8$-Alkyl, insbesondere $C_1$-$C_4$-Alkyl.

Durch $C_1$-$C_{12}$-Alkyl substituiertes Benzoyloxy, das vorzugsweise 1 bis 3, insbesondere 1 oder 2 Alkylgruppen trägt, bedeutet beispielsweise o-, m- oder p-Methylbenzoyloxy, 2,3-Dimethylbenzoyloxy, 2,4-Dimethylbenzoyloxy, 2,5-Dimethylbenzoyloxy, 2,6-Dimethylbenzoyloxy, 3,4-Dimethylbenzoyloxy, 3,5-Dimethylbenzoyloxy, 2-Methyl-6-ethylbenzoyloxy, 4-tert-Butylbenzoyloxy, 2-Ethylbenzoyloxy, 2,4,6-Trimethylbenzoyloxy, 2,6-Dimethyl-4-tert-butylbenzoyloxy oder 3,5-Di-tert-butylbenzoyloxy. Bevorzugte Substituenten sind $C_1$-$C_8$-Alkyl, insbesondere $C_1$-$C_4$-Alkyl.

Durch $C_1$-$C_{12}$-Alkyl substituiertes Naphthoyl, das 1-Naphthoyl oder 2-Naphthoyl bedeutet und das vorzugsweise 1 bis 3, insbesondere 1 oder 2 Alkylgruppen enthält, bedeutet beispielsweise 1-, 2-, 3-, 4-, 5-, 6-, 7- oder 8-Methyl-naphthoyl, 1-, 2-, 3-, 4-, 5-, 6-, 7- oder 8-Ethyl-naphthoyl, 4-tert-Butyl-naphthoyl oder 6-tert-Butyl-naphthoyl. Besonders bevorzugte Substituenten sind $C_1$-$C_8$-Alkyl, insbesondere $C_1$-$C_4$-Alkyl.

$C_1$-$C_{25}$-Alkansulfonyl bedeutet einen verzweigten oder unverzweigten Rest wie beispielsweise Methansulfonyl, Ethansulfonyl, Propansulfonyl, Butansulfonyl, Pentansulfonyl, Hexansulfonyl, Heptansulfonyl, Oktansulfonyl, Nonansulfonyl oder Docosansulfonyl. Bevorzugt ist Alkansulfonyl mit 1 bis 18, insbesondere 1 bis 12, z.B. 2 bis 6 Kohlenstoffatomen. Besonders bevorzugt ist Methansulfonyl.

Durch Fluor substituiertes $C_1$-$C_{25}$-Alkansulfonyl bedeutet beispielsweise Trifluormethansulfonyl.

Durch $C_1$-$C_{12}$-Alkyl substituiertes Phenylsulfonyl, das vorzugsweise 1 bis 3, insbesondere 1 oder 2 Alkylgruppen trägt, bedeutet beispielsweise o-, m- oder p-Methylphenylsulfonyl, p-Ethylphenylsulfonyl, p-Propylphenylsulfonyl oder p-Butylphenylsulfonyl. Bevorzugte Substituenten sind $C_1$-$C_8$-Alkyl, insbesondere $C_1$-$C_4$-Alkyl. Besonders bevorzugt ist p-Methylphenylsulfonyl.

Alkyl mit bis zu 25 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl,1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl. Eine der bevorzugten Bedeutungen von $R_2$ und $R_4$ ist beispielweise $C_1$-$C_{18}$-Alkyl. Eine besonders bevorzugte Bedeutung von $R_4$ ist $C_1$-$C_4$-Alkyl.

Alkenyl mit 3 bis 25 Kohlenstoffatomen bedeutet einen verzweigten oder unverzweigten Rest wie beispielsweise Propenyl, 2-Butenyl, 3-Butenyl, Isobutenyl, n-2,4-Pentadienyl, 3-Methyl-2-butenyl, n-2-Octenyl, n-2-Dodecenyl, iso-Dodecenyl, Oleyl, n-2-Octadecenyl oder n-4-Octadecenyl. Bevorzugt ist Alkenyl mit 3 bis 18, insbesondere 3 bis 12, z.B. 3 bis 6, vor allem 3 bis 4 Kohlenstoffatomen.

Alkenyloxy mit 3 bis 25 Kohlenstoffatomen bedeutet einen verzweigten oder unverzweigten Rest wie beispielsweise Propenyloxy, 2-Butenyloxy, 3-Butenyloxy, Isobutenyloxy, n-2,4-Pentadienyloxy, 3-Methyl-2-butenyloxy, n-2-Octenyloxy, n-2-Dodecenyloxy, iso-Dodecenyloxy, Oleyloxy, n-2-Octadecenyloxy oder n-4-Octadecenyloxy. Bevorzugt ist Alkenyloxy mit 3 bis 18, insbesondere 3 bis 12, z.B. 3 bis 6, vor allem 3 bis 4 Kohlenstoffatomen.

Alkinyl mit 3 bis 25 Kohlenstoffatomen bedeutet einen verzweigten oder unverzweigten Rest wie beispielsweise Propinyl( $-CH_2-C\equiv CH$ ), 2-Butinyl, 3-Butinyl, n-2-Octinyl, oder n-2-Dodecinyl. Bevorzugt ist Alkinyl mit 3 bis 18, insbesondere 3 bis 12, z.B. 3 bis 6, vor allem 3 bis 4 Kohlenstoffatomen.

Alkinyloxy mit 3 bis 25 Kohlenstoffatomen bedeutet einen verzweigten oder unverzweigten Rest wie beispielsweise Propinyloxy ( $-OCH_2-C\equiv CH$ ), 2-Butinyloxy, 3-Butinyloxy, n-2-Octinyloxy, oder n-2-Dodecinyloxy. Bevorzugt ist Alkinyloxy mit 3 bis 18, insbesondere 3 bis 12, z.B. 3 bis 6, vor allem 3 bis 4 Kohlenstoffatomen.

Durch Sauerstoff, Schwefel oder $>N-R_8$ unterbrochenes $C_2$-$C_{25}$-Alkyl bedeutet beispielsweise $CH_3$-O-$CH_2$-, $CH_3$-S-$CH_2$-, $CH_3$-NH-$CH_2$-, $CH_3$-N($CH_3$)-$CH_2$-, $CH_3$-O-$CH_2CH_2$-O-$CH_2$-, $CH_3$-(O-$CH_2CH_2$-)$_2$O-$CH_2$-, $CH_3$-(O-$CH_2CH_2$-)$_3$O-$CH_2$- oder $CH_3$-(O-$CH_2CH_2$-)$_4$O-$CH_2$-.

$C_7$-$C_9$-Phenylalkyl bedeutet beispielsweise Benzyl, α-Methylbenzyl, α,α-Dimethylbenzyl oder 2-Phenylethyl. Benzyl und a,a-Dimethylbenzyl ist bevorzugt.

Unsubstituiertes oder am Phenylrest durch 1 bis 3 $C_1$-$C_4$-Alkyl substituiertes $C_7$-$C_9$-Phenylalkyl bedeutet beispielsweise Benzyl, α-Methylbenzyl, α,α-Dimethylbenzyl, 2-Phenylethyl, 2-Methylbenzyl, 3-Methylbenzyl, 4-Methylbenzyl, 2,4-Dimethylbenzyl, 2,6-Dimethylbenzyl oder 4-tert-Butylbenzyl. Benzyl ist bevorzugt.

Durch Sauerstoff, Schwefel oder $>N-R_8$ unterbrochenes unsubstituiertes oder am Phenylrest durch 1 bis 3 $C_1$-$C_4$-Alkyl substituiertes $C_7$-$C_{25}$-Phenylalkyl bedeutet einen verzweigten oder unverzweigten Rest wie beispielsweise Phenoxymethyl, 2-Methyl-phenoxymethyl, 3-Methyl-phenoxymethyl, 4-Methyl-phenoxymethyl, 2,4-Dimethyl-phenoxymethyl, 2,3-Dimethyl-phenoxymethyl, Phenylthiomethyl, N-Methyl-N-phenyl-methyl, N-Ethyl-N-phenyl-methyl, 4-tert-Butyl-phenoxymethyl, 4-tert-Butyl-phenoxyethoxymethyl, 2,4-Di-tert-butyl-phenoxymethyl, 2,4-Di-tert-butyl-phenoxyethoxymethyl, Phenoxyethoxyethoxyethoxymethyl, Benzyloxymethyl, Benzyloxyethoxymethyl, N-Benzyl-N-ethyl-methyl oder N-Benzyl-N-isopropyl-methyl.

$C_7$-$C_9$-Phenylalkoxy bedeutet beispielsweise Benzyloxy, α-Methylbenzyloxy, α,α-Dimethylbenzyloxy oder 2-Phenylethoxy. Benzyloxy ist bevorzugt.

Durch $C_1$-$C_4$-Alkyl substituiertes Phenyl, das vorzugsweise 1 bis 3, insbesondere 1 oder 2 Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-Butylphenyl, 2-Ethylphenyl oder 2,6-Diethylphenyl.

Durch $C_1$-$C_4$-Alkyl substituiertes Phenoxy, das vorzugsweise 1 bis 3, insbesondere 1 oder 2 Alkylgruppen enthält, bedeutet beispielsweise o-, m- oder p-Methylphenoxy, 2,3-Dimethylphenoxy, 2,4-Dimethylphenoxy, 2,5-Dimethylphenoxy, 2,6-Dimethylphenoxy, 3,4-Dimethylphenoxy, 3,5-Dimethylphenoxy, 2-Methyl-6-ethylphenoxy, 4-tert-Butylphenoxy, 2-Ethylphenoxy oder 2,6-Diethylphenoxy.

Unsubstituiertes oder durch $C_1$-$C_4$-Alkyl substituiertes $C_5$-$C_8$-Cycloalkyl bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl oder Cyclooctyl. Bevorzugt ist Cyclohexyl und tert-Butylcyclohexyl.

Unsubstituiertes oder durch $C_1$-$C_4$-Alkyl substituiertes $C_5$-$C_8$-Cycloalkoxy bedeutet beispielsweise Cyclopentoxy, Methylcyclopentoxy, Dimethylcyclopentoxy, Cyclohexoxy, Methylcyclohexoxy, Dimethylcyclohexoxy, Trimethylcyclohexoxy, tert-Butylcyclohexoxy, Cycloheptoxy oder Cyclooctoxy. Bevorzugt ist Cyclohexoxy und tert-Butylcyclohexoxy.

Alkoxy mit bis zu 25 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy. Bevorzugt ist Alkoxy mit 1 bis 12, insbesondere 1 bis 8, z.B. 1 bis 6 Kohlenstoffatomen.

Durch Sauerstoff, Schwefel oder $>N-R_8$ unterbrochenes $C_2$-$C_{25}$-Alkoxy bedeutet beispielsweise $CH_3$-O-$CH_2CH_2$O-, $CH_3$-S-$CH_2CH_2$O-, $CH_3$-NH-$CH_2CH_2$O-, $CH_3$-N($CH_3$)-$CH_2CH_2$O-, $CH_3$-O-$CH_2CH_2$-O-$CH_2CH_2$O-, $CH_3$-(O-$CH_2CH_2$-)$_2$O-$CH_2CH_2$O-, $CH_3$-(O-$CH_2CH_2$-)$_3$O-$CH_2CH_2$O- oder $CH_3$-(O-$CH_2CH_2$-)$_4$O-$CH_2CH_2$O-.

Alkylthio mit bis zu 25 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio, Hexylthio,

Heptylthio, Octylthio, Decylthio, Tetradecylthio, Hexadecylthio oder Octadecylthio. Bevorzugt ist Alkylthio mit 1 bis 12, insbesondere 1 bis 8, z.B. 1 bis 6 Kohlenstoffatomen.

Alkylamino mit bis zu 4 Kohlenstoffatomen bedeutet einen verzweigten oder unverzweigten Rest wie beispielsweise Methylamino, Ethylamino, Propylamino, Isopropylamino, n-Butylamino, Isobutylamino oder tert-Butylamino.

Di-($C_1$-$C_4$-alkyl)amino bedeutet auch, dass die beiden Reste unabhängig voneinander verzweigt oder unverzweigt sind wie beispielsweise Dimethylamino, Methylethylamino, Diethylamino, Methyl-n-propylamino, Methylisopropylamino, Methyl-n-butylamino, Methylisobutylamino, Ethylisopropylamino, Ethyl-n-butylamino, Ethylisobutylamino, Ethyl-tert-butylamino, Diethylamino, Diisopropylamino, Isopropyl-n-butylamino, Isopropylisobutylamino, Di-n-butylamino oder Di-isobutylamino.

Alkanoylamino mit bis zu 25 Kohlenstoffatomen bedeutet einen verzweigten oder unverzweigten Rest wie beispielsweise Formylamino, Acetylamino, Propionylamino, Butanoylamino, Pentanoylamino, Hexanoylamino, Heptanoylamino, Octanoylamino, Nonanoylamino, Decanoylamino, Undecanoylamino, Dodecanoylamino, Tridecanoylamino, Tetradecanoylamino, Pentadecanoylamino, Hexadecanoylamino, Heptadecanoylamino, Octadecanoylamino, Eicosanoylamino oder Docosanoylamino. Bevorzugt ist Alkanoylamino mit 2 bis 18, insbesondere 2 bis 12, z.B. 2 bis 6 Kohlenstoffatomen.

$C_1$-$C_{18}$-Alkylen bedeutet einen verzweigten oder unverzweigten Rest wie beispielsweise Methylen, Ethylen, Propylen, Trimethylen, Tetramethylen, Pentamethylen, Hexamethylen, Heptamethylen, Octamethylen, Decamethylen, Dodecamethylen oder Octadecamethylen. Bevorzugt ist $C_1$-$C_{12}$-Alkylen, insbesondere $C_1$-$C_8$-Alkylen.

Durch $C_1$-$C_4$-Alkyl substituierter $C_5$-$C_{12}$-Cycloalkylenring, der vorzugsweise 1 bis 3, insbesondere 1 oder 2 verzweigte oder unverzweigte Alkylgruppen-Reste enthält, bedeutet beispielsweise Cyclopentylen, Methylcyclopentylen, Dimethylcyclopentylen, Cyclohexylen, Methylcyclohexylen, Dimethylcyclohexylen, Trimethylcyclohexylen, tert-Butylcyclohexylen, Cycloheptylen, Cyclooctylen oder Cyclodecylen. Bevorzugt ist Cyclohexylen und tert-Butylcyclohexylen.

Durch Sauerstoff, Schwefel oder $>N-R_8$ unterbrochenes $C_2$-$C_{18}$-Alkylen bedeutet beispielsweise $-CH_2-O-CH_2-$, $-CH_2-S-CH_2-$, $-CH_2-NH-CH_2-$, $-CH_2-N(CH_3)-CH_2-$, $-CH_2-O-CH_2CH_2-O-CH_2-$, $-CH_2-(O-CH_2CH_2-)_2O-CH_2-$, $-CH_2-(O-CH_2CH_2-)_3O-CH_2-$, $-CH_2-(O-CH_2CH_2-)_4O-CH_2-$ oder $-CH_2CH_2-S-CH_2CH_2-$.

$C_2$-$C_{18}$-Alkenylen bedeutet beispielsweise Vinylen, Methylvinylen, Octenylethylen oder Dodecenylethylen. Bevorzugt ist $C_2$-$C_8$-Alkenylen.

Alkyliden mit 2 bis 20 Kohlenstoffatomen bedeutet beispielsweise Ethyliden, Propyliden, Butyliden, Pentyliden, 4-Methylpentyliden, Heptyliden, Nonyliden, Tridecyliden, Nonadecyliden, 1-Methylethyliden, 1-Ethylpropyliden oder 1-Ethylpentyliden. Bevorzugt ist $C_2$-$C_8$-Alkyliden.

Phenylalkyliden mit 7 bis 20 Kohlenstoffatomen bedeutet beispielsweise Benzyliden, 2-Phenylethyliden oder 1-Phenyl-2-hexyliden. Bevorzugt ist $C_7$-$C_9$-Phenylalkyliden.

$C_5$-$C_8$-Cycloalkylen bedeutet eine gesättigte Kohlenwasserstoffgruppe mit zwei freien Valenzen und mindestens einer Ringeinheit und ist beispielsweise Cyclopentylen, Cyclohexylen, Cycloheptylen oder Cyclooctylen. Bevorzugt ist Cyclohexylen.

$C_7$-$C_8$-Bicycloalkylen bedeutet beispielsweise Bicycloheptylen oder Bicyclooctylen.

Unsubstituiertes oder durch $C_1$-$C_4$-Alkyl substituiertes Phenylen oder Naphthylen bedeutet beispielsweise 1,2-, 1,3-, 1,4-Phenylen, 1,2-, 1,3-, 1,4-, 1,6-, 1,7-, 2,6-oder 2,7-Naphthylen. 1,4-Phenylen ist bevorzugt.

Durch $C_1$-$C_4$-Alkyl substituierter $C_5$-$C_8$-Cycloalkylidenring, der vorzugsweise 1 bis 3, insbesondere 1 oder 2 verzweigte oder unverzweigte Alkylgruppen-Reste enthält, bedeutet beispielsweise Cyclopentyliden, Methylcyclopentyliden, Dimethylcyclopentyliden, Cyclohexyliden, Methylcyclohexyliden, Dimethylcyclohexyliden, Trimethylcyclohexyliden, tert-Butylcyclohexyliden, Cycloheptyliden oder Cyclooctyliden. Bevorzugt ist Cyclohexyliden und tert-Butylcyclohexyliden.

Ein ein-, zwei- oder drei-wertiges Metallkation ist vorzugsweise ein Alkalimetall-, Erdalkalimetall- oder Aluminium-Kation, beispielsweise $Na^+$, $K^+$, $Mg^{++}$, $Ca^{++}$ oder $Al^{+++}$.

Als Substituenten für den aromatischen Rest $R_1$ (für n = 1) kommen beispielsweise solche in Betracht, wie sie nachstehend für die Reste $R_{19}$ bis $R_{23}$ definert werden. Solche Substituenten sind insbesondere Chlor, Amino, Hydroxy, $C_1$-$C_{18}$-Alkyl, $C_1$-$C_{18}$-Alkoxy, $C_1$-$C_{18}$-Alkylthio, $C_3$-$C_4$-Alkenyloxy, $C_3$-$C_4$-Alkinyloxy, $C_1$-$C_4$-Alkylamino. Di-($C_1$-$C_4$-Alkyl)amino, Phenyl, Benzyl, Benzoyl oder Benzoyloxy, vor allem Chlor, Amino, Hydroxy, $C_1$-$C_4$-Alkyl, $C_1$-$C_4$-Alkoxy, $C_1$-$C_4$-Alkylthio, $C_1$-$C_4$-Alkylamino oder Di($C_1$-$C_4$-alkyl)amino.

Ebenfalls von besonderem Interesse ist ein Verfahren zur Herstellung von Verbindungen der Formel I, worin, wenn n 2 ist,

$R_1$ $-R_6-X-R_7-$ darstellt,
$R_6$ und $R_7$ Phenylen bedeuten,
X Sauerstoff oder $-NR_{14}-$ darstellt, und
$R_{14}$ $C_1$-$C_4$-Alkyl bedeutet.

Speziell von besonderem Interesse ist ein Verfahren zur Herstellung von Verbindungen der Formel I, worin, wenn n 1 ist,

$R_1$ Phenanthryl, Thienyl, Dibenzofuryl, unsubstituiertes oder mit $C_1$-$C_4$-Alkyl substituiertes Carbazolyl; oder Fluorenyl darstellt, oder $R_1$ einen Rest der Formel V oder VI

(V) 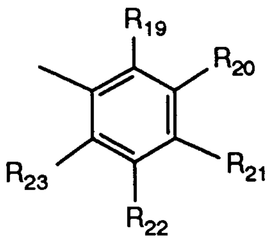 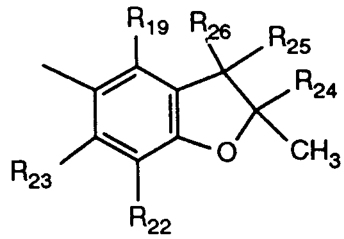 (VI)

bedeutet, worin

$R_{19}$, $R_{20}$, $R_{21}$, $R_{22}$ und $R_{23}$ unabhängig voneinander Wasserstoff, Halogen, Hydroxy, $C_1$-$C_{18}$-Alkyl, durch Sauerstoff, Schwefel oder $\rangle N—R_8$ unterbrochenes $C_2$-$C_{18}$-Alkyl;

$C_1$-$C_{18}$-Alkoxy, durch Sauerstoff, Schwefel oder $\rangle N—R_8$ unterbrochenes $C_2$-$C_{18}$ -Alkoxy; $C_1$-$C_{18}$-Alkylthio, $C_3$-$C_{18}$-Alkenyl, $C_3$-$C_{18}$-Alkenyloxy, $C_3$-$C_{18}$-Alkinyl, $C_3$-$C_{18}$-Alkinyloxy, $C_7$-$C_9$-Phenylalkyl, $C_7$-$C_9$-Phenylalkoxy, unsubstituiertes oder durch $C_1$-$C_4$-Alkyl substituiertes Phenyl; unsubstituiertes oder durch $C_1$-$C_4$-Alkyl substituiertes Phenoxy; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes $C_5$-$C_8$-Cycloalkoxy; $C_1$-$C_4$-Alkylamino, Di-($C_1$-$C_4$-alkyl)amino, $C_1$-$C_{18}$-Alkanoyl, durch Sauerstoff, Schwefel oder $\rangle N—R_8$ unterbrochenes $C_3$-$C_{18}$-Alkanoyl; $C_1$-$C_{18}$-Alkanoyloxy, durch Sauerstoff, Schwefel oder $\rangle N—R_8$ unterbrochenes $C_3$-$C_{18}$-Alkanoyloxy; $C_1$-$C_{18}$-Alkanoylamino, $C_3$-$C_{18}$-Alkenoyl, durch Sauerstoff, Schwefel oder $\rangle N—R_8$ unterbrochenes $C_3$-$C_{18}$-Alkenoyl;

$C_3$-$C_{18}$-Alkenoyloxy, durch Sauerstoff, Schwefel oder $\rangle N—R_8$ unterbrochenes $C_3$-$C_{18}$-Alkenoyloxy; $C_6$-$C_9$-Cycloalkylcarbonyl, $C_6$-$C_9$-Cycloalkylcarbonyloxy, Benzoyl oder durch $C_1$-$C_{12}$-Alkyl substituiertes Benzoyl; Benzoyloxy oder durch $C_1$-$C_8$-Alkyl substituiertes Benzoyloxy;

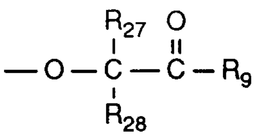 , 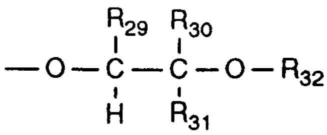

darstellen, oder ferner in Formel V die Reste $R_{19}$ und $R_{20}$ oder die Reste $R_{20}$ und $R_{21}$ zusammen mit den Kohlenstoffatomen, an die sie gebunden sind, einen Benzoring bilden,

$R_{24}$ Wasserstoff, $C_1$-$C_4$-Alkyl, unsubstituiertes oder durch $C_1$-$C_4$-Alkyl substituiertes Phenyl bedeutet,

$R_{25}$ und $R_{26}$ Wasserstoff, $C_1$-$C_4$-Alkyl oder Phenyl bedeuten, mit der Bedingung, dass mindestens einer der Reste $R_{25}$ und $R_{26}$ Wasserstoff ist,

$R_{27}$ und $R_{28}$ unabhängig voneinander Wasserstoff, $C_1$-$C_4$-Alkyl oder Phenyl darstellen,

$R_{29}$ Wasserstoff oder $C_1$-$C_4$-Alkyl ist,

$R_{30}$ Wasserstoff, unsubstituiertes oder durch $C_1$-$C_4$-Alkyl substituiertes Phenyl; $C_1$-$C_{18}$-Alkyl, durch Sauerstoff, Schwefel oder $\rangle N—R_8$ unterbrochenes $C_2$-$C_{18}$-Alkyl; unsubstituiertes oder am Phenylrest durch 1 bis 3 $C_1$-$C_4$-Alkyl substituiertes $C_7$-$C_9$-Phenylalkyl; durch Sauerstoff, Schwefel oder $\rangle N—R_8$ unterbrochenes unsubstituiertes oder am Phenylrest durch 1 bis 3 $C_1$-$C_4$-Alkyl substituiertes $C_7$-$C_{18}$-Phenylalkyl bedeutet, oder ferner die Reste $R_{29}$ und $R_{30}$ zusammen mit den Kohlenstoffatomen, an die sie gebunden sind, einen unsubstituierten oder durch 1 bis 3 $C_1$-$C_4$-Alkyl substituierten $C_5$-$C_9$-Cycloalkylenring bilden;

$R_{31}$ Wasserstoff oder $C_1$-$C_4$-Alkyl darstellt,

$R_{32}$ Wasserstoff, $C_1$-$C_{18}$-Alkanoyl, $C_3$-$C_{18}$-Alkenoyl, durch Sauerstoff, Schwefel oder $\rangle N—R_8$ unterbrochenes $C_3$-$C_{18}$-Alkanoyl; durch eine Di($C_1$-$C_6$-alkyl)phosphonatgruppe substituiertes $C_2$-$C_{18}$-Alkanoyl; $C_6$-$C_9$-Cycloalkylcarbonyl, Thenoyl, Furoyl, Benzoyl oder durch $C_1$-$C_8$-Alkyl substituiertes Benzoyl;

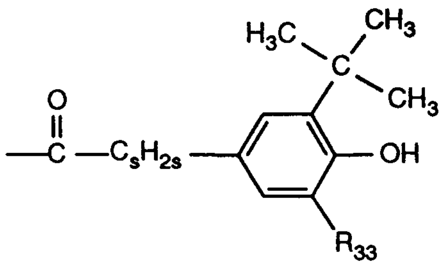,

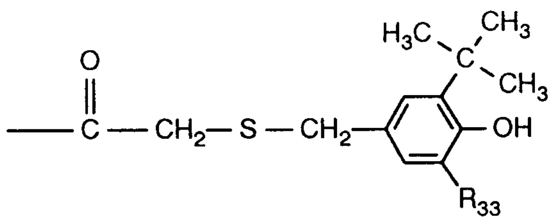, 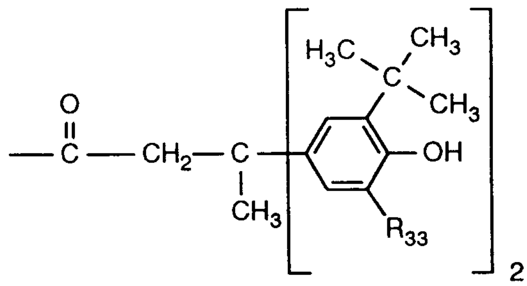,

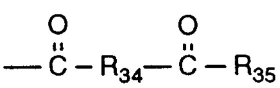 oder 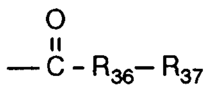

bedeutet,

$R_{33}$ Wasserstoff oder $C_1$-$C_8$-Alkyl darstellt,

$R_{34}$ eine direkte Bindung, $C_1$-$C_{18}$-Alkylen, durch Sauerstoff, Schwefel oder $>N—R_8$ unterbrochenes $C_2$-$C_{12}$-Alkylen; $C_2$-$C_{12}$-Allcenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

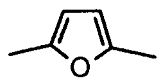 oder 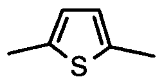

darstellt,

$R_{35}$ Hydroxy, $[-O^{\theta}\ \frac{1}{r}\ M^{r+}]$, $C_1$-$C_{18}$-Alkoxy oder

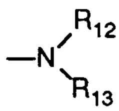

bedeutet,

$R_{36}$ Sauerstoff, -NH- oder

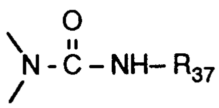

darstellt,

$R_{37}$ $C_1$-$C_{12}$-Alkyl oder Phenyl ist, und

s 0, 1 oder 2 bedeutet.

Bevorzugt ist ein Verfahren zur Herstellung von Verbindungen der Formel I, worin

$R_{19}$, $R_{20}$, $R_{21}$, $R_{22}$ und $R_{23}$ unabhängig voneinander Wasserstoff, Chlor, Brom, Hydroxy, $C_1$-$C_{18}$-Alkyl, durch Sauerstoff oder Schwefel unterbrochenes $C_2$-$C_{18}$-Alkyl; $C_1$-$C_{18}$-Alkoxy, durch Sauerstoff oder Schwefel unterbrochenes $C_2$-$C_{18}$-Alkoxy; $C_1$-$C_{18}$-Alkylthio, $C_3$-$C_{12}$-Alkenyloxy, $C_3$-$C_{12}$-Alkinyloxy, $C_7$-$C_9$-Phenylalkyl, $C_7$-$C_9$-Phenylalkoxy, unsubstituiertes oder durch $C_1$-$C_4$-Alkyl substituiertes Phenyl; Phenoxy, Cyclohexyl, $C_5$-$C_8$-Cycloalkoxy; $C_1$-$C_4$-Alkylamino, Di-($C_1$-$C_4$-alkyl)amino, $C_1$-$C_{12}$-Alkanoyl, durch Sauerstoff oder Schwefel unterbrochenes $C_3$-$C_{12}$-Alkanoyl; $C_1$-$C_{12}$-Alkanoyloxy, durch Sauerstoff oder Schwefel unterbrochenes $C_3$-$C_{12}$-Alkanoyloxy; $C_1$-$C_{12}$-Alkanoylamino, $C_3$-$C_{12}$-Alkenoyl, $C_3$-$C_{12}$-Alkenoyloxy, Cyclohexylcarbonyl, Cyclohexylcarbonyloxy, Benzoyl oder durch $C_1$-$C_4$-Alkyl substituiertes Benzoyl; Benzoyloxy oder durch $C_1$-$C_4$-Alkyl substituiertes Benzoyloxy;

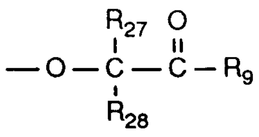 , 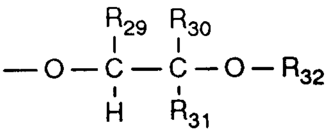

darstellen, oder ferner in Formel V die Reste $R_{19}$ und $R_{20}$ oder die Reste $R_{20}$ und $R_{21}$ zusammen mit den Kohlenstoffatomen, an die sie gebunden sind, einen Benzoring bilden,
$R_{24}$ Wasserstoff oder $C_1$-$C_4$-Alkyl bedeutet,
$R_{25}$ und $R_{26}$ Wasserstoff oder $C_1$-$C_4$-Alkyl bedeuten, mit der Bedingung, dass mindestens einer der Reste $R_{25}$ und $R_{26}$ Wasserstoff ist,
$R_{27}$ und $R_{28}$ unabhängig voneinander Wasserstoff oder $C_1$-$C_4$-Alkyl darstellen,
$R_{29}$ Wasserstoff ist,
$R_{30}$ Wasserstoff, Phenyl, $C_1$-$C_{18}$-Alkyl, durch Sauerstoff oder Schwefel unterbrochenes $C_2$-$C_{18}$-Alkyl; $C_7$-$C_9$-Phenylalkyl, durch Sauerstoff oder Schwefel unterbrochenes unsubstituiertes oder am Phenylrest durch 1 bis 3 $C_1$-$C_4$-Alkyl substituiertes $C_7$-$C_{18}$-Phenylalkyl bedeutet, oder ferner die Reste $R_{29}$ und $R_{30}$ zusammen mit den Kohlenstoffatomen, an die sie gebunden sind, einen unsubstituierten oder durch 1 bis 3 $C_1$-$C_4$-Alkyl substituierten Cyclohexylenring bilden;
$R_{31}$ Wasserstoff oder $C_1$-$C_4$-Alkyl darstellt,
$R_{32}$ Wasserstoff, $C_1$-$C_{18}$-Alkanoyl, $C_3$-$C_{12}$-Alkenoyl, durch Sauerstoff oder Schwefel unterbrochenes $C_3$-$C_{12}$-Alkanoyl; durch eine Di($C_1$-$C_6$-alkyl)phosphonatgruppe substituiertes $C_2$-$C_{12}$-Alkanoyl; $C_6$-$C_9$-Cycloalkylcarbonyl, Benzoyl,

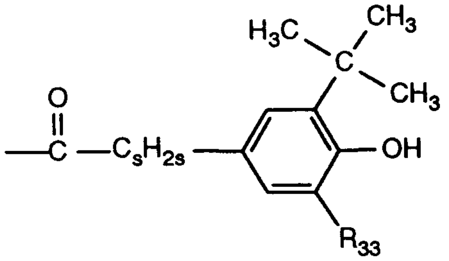 , 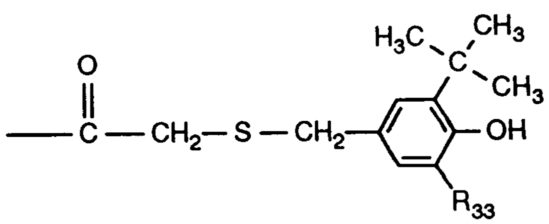 ,

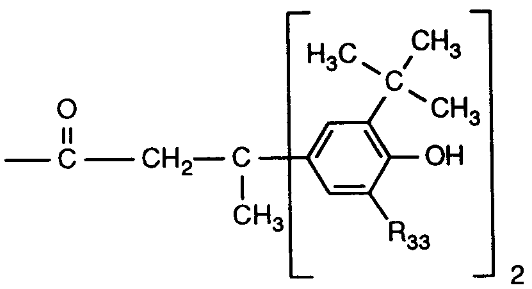 , 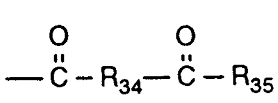 oder 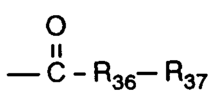

bedeutet,
$R_{33}$ Wasserstoff oder $C_1$-$C_4$-Alkyl darstellt,
$R_{34}$ $C_1$-$C_{12}$-Alkylen, $C_2$-$C_8$-Alkenylen, $C_2$-$C_8$-Alkyliden, $C_7$-$C_{12}$-Phenylalkyliden, $C_5$-$C_8$-Cycloalkylen oder Phenylen darstellt,

$R_{35}$ Hydroxy, $[-O^{\theta} \frac{1}{r} M^{r+}]$, oder $C_1$-$C_{18}$-Alkoxy bedeutet,
$R_{36}$ Sauerstoff oder -NH- darstellt,
$R_{37}$ $C_1$-$C_8$-Alkyl oder Phenyl ist, und
s 1 oder 2 bedeutet.

Ebenfalls bevorzugt ist ein Verfahren zur Herstellung von Verbindungen der Formel I, worin

$R_1$ Phenanthryl, Thienyl, Dibenzofuryl, unsubstituiertes oder mit $C_1$-$C_4$-Alkyl substituiertes Carbazolyl; oder Fluorenyl darstellt, oder $R_1$ einen Rest der Formel V

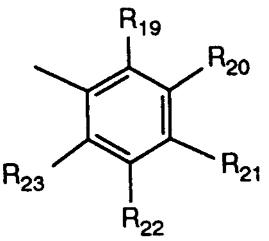

(V)

bedeutet, worin
$R_{19}$, $R_{20}$, $R_{21}$, $R_{22}$ und $R_{23}$ unabhängig voneinander Wasserstoff, Chlor, Hydroxy, $C_1$-$C_{18}$-Alkyl, $C_1$-$C_{18}$-Alkoxy, $C_1$-$C_{18}$-Alkylthio, $C_3$-$C_4$-Alkenyloxy, $C_3$-$C_4$-Alkinyloxy, Phenyl, Benzoyl, Benzoyloxy oder

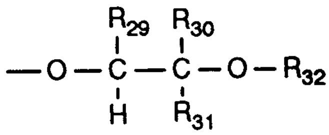

darstellen,
$R_{29}$ Wasserstoff ist,
$R_{30}$ Wasserstoff, Phenyl oder $C_1$-$C_{18}$-Alkyl bedeutet, oder ferner die Reste $R_{29}$ und $R_{30}$ zusammen mit den Kohlenstoffatomen, an die sie gebunden sind, einen unsubstituierten oder durch 1 bis 3 $C_1$-$C_4$-Alkyl substituierten Cyclohexylenring bilden;
$R_{31}$ Wasserstoff oder $C_1$-$C_4$-Alkyl darstellt, und
$R_{32}$ Wasserstoff, $C_1$-$C_{12}$-Alkanoyl oder Benzoyl bedeutet.

Besonders bevorzugt ist ein Verfahren zur Herstellung von Verbindungen der Formel I, worin

$R_{19}$ Wasserstoff oder $C_1$-$C_4$-Alkyl darstellt,
$R_{20}$ Wasserstoff oder $C_1$-$C_4$-Alkyl bedeutet,
$R_{21}$ Wasserstoff, Chlor, Hydroxy, $C_1$-$C_{12}$-Alkyl, $C_1$-$C_4$-Alkoxy, $C_1$-$C_4$-Alkylthio, Phenyl oder $-O-CH_2-CH_2-O-R_{32}$ darstellt,
$R_{22}$ Wasserstoff oder $C_1$-$C_4$-Alkyl ist,
$R_{23}$ Wasserstoff oder $C_1$-$C_4$-Alkyl bedeutet, und
$R_{32}$ $C_1$-$C_4$-Alkanoyl darstellt.

Ganz besonders bevorzugt ist ein Verfahren zur Herstellung von Verbindungen der Formel I, worin

$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{12}$-Alkoxy, $C_1$-$C_{12}$-Alkylthio, $C_1$-$C_4$-Alkylamino, Di-($C_1$-$C_4$-alkyl)amino, $C_1$-$C_{18}$-Alkanoyloxy, $C_1$-$C_{18}$-Alkanoylamino, $C_3$-$C_{18}$-Alkenoyloxy, durch Sauerstoff, Schwefel oder $>N-R_8$ unterbrochenes $C_3$-$C_{18}$-Alkanoyloxy; $C_6$-$C_9$-Cycloalkylcarbonyloxy, Benzoyloxy oder durch $C_1$-$C_8$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich $-(CH_2)_p-COR_9$ oder $-(CH_2)_qOH$ dar-

stellt, oder wenn $R_3$ und $R_5$ Wasserstoff sind, $R_4$ zusätzlich einen Rest der Formel II

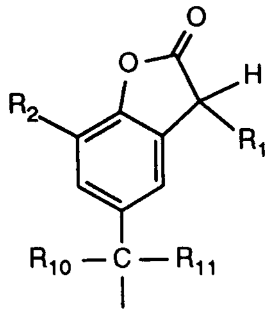

(II)

bedeutet,

$R_8$ Wasserstoff oder $C_1$-$C_6$-Alkyl ist,

$R_9$ Hydroxy, $C_1$-$C_{18}$-Alkoxy oder

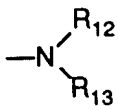

bedeutet,

$R_{10}$ und $R_{11}$ Methylgruppen sind oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;

$R_{12}$ und $R_{13}$ unabhängig voneinander Wasserstoff oder $C_1$-$C_8$-Alkyl darstellen, und q 2, 3, 4, 5 oder 6 bedeutet.

Speziell bevorzugt ist ein Verfahren zur Herstellung von Verbindungen der Formel I, worin mindestens zwei der Reste $R_2$, $R_3$, $R_4$ und $R_5$ Wasserstoff sind.

Ebenfalls ganz besonders bevorzugt ist ein Verfahren zur Herstellung von Verbindungen der Formel I, worin $R_3$ und $R_5$ Wasserstoff sind.

Ganz speziell bevorzugt ist ein Verfahren zur Herstellung von Verbindungen der Formel I, worin

$R_2$, $R_3$, $R_4$ und $R_5$ unabhängig voneinander Wasserstoff, Chlor, Hydroxy, $C_1$-$C_{18}$-Alkyl, $C_7$-$C_9$-Phenylalkyl, Phenyl, $C_5$-$C_8$-Cycloalkyl, $C_1$-$C_6$-Alkoxy, Cyclohexylcarbonyloxy oder Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich $-(CH_2)_p-COR_9$ darstellt, oder wenn $R_3$ und $R_5$ Wasserstoff sind, $R_4$ zusätzlich einen Rest der Formel II bedeutet,

$R_9$ Hydroxy oder $C_1$-$C_{18}$-Alkoxy bedeutet, und

$R_{10}$ und $R_{11}$ Methylgruppen sind oder zusammen mit dem C-Atom, an das sie gebunden sind, einen $C_5$-$C_8$-Cycloalkylidenring bilden.

Von bevorzugtem Interesse ist ein Verfahren zur Herstellung von Verbindungen der Formel I, worin

$R_2$ $C_1$-$C_{18}$-Alkyl oder Cyclohexyl bedeutet,

$R_3$ Wasserstoff ist,

$R_4$ $C_1$-$C_4$-Alkyl, Cyclohexyl oder einen Rest der Formel II darstellt,

$R_5$ Wasserstoff ist, und

$R_{10}$ und $R_{11}$ zusammen mit dem C-Atom, an das sie gebunden sind, einen Cyclohexylidenring bilden.

Von ebenfalls bevorzugtem Interesse ist ein Verfahren zur Herstellung von Verbindungen der Formel I, worin

$R'_{15}$ Wasserstoff, $C_1$-$C_{18}$-Alkanoyl, $C_3$-$C_{18}$-Alkenoyl, durch Sauerstoff, Schwefel oder $>N-R_8$ unterbrochenes $C_3$-$C_{18}$-Alkanoyl; $C_6$-$C_9$-Cycloalkylcarbonyl, Thenoyl, Furoyl, Benzoyl oder durch $C_1$-$C_8$-Alkyl substituiertes Benzoyl; Naphthoyl oder durch $C_1$-$C_8$-Alkyl substituiertes Naphthoyl; $C_1$-$C_{18}$-Alkansulfonyl, durch Fluor substituiertes $C_1$-

$C_{18}$-Alkansulfonyl; Phenylsulfonyl oder durch $C_1$-$C_8$-Alkyl substituiertes Phenylsulfonyl;

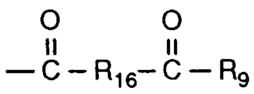 oder 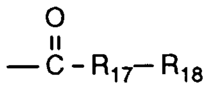

bedeutet,

$R_{16}$ eine direkte Bindung, $C_1$-$C_{12}$-Alkylen, durch Sauerstoff, Schwefel oder $>N—R_8$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen darstellt,

$R_{17}$ Sauerstoff oder -NH- bedeutet, und

$R_{18}$ $C_1$-$C_{12}$-Alkyl oder Phenyl ist.

Von ganz speziell bevorzugtem Interesse ist ein Verfahren zur Herstellung von Verbindungen der Formel I, worin

$R_{15}$ Chlor, Brom oder -OR'$_{15}$ darstellt,

R'$_{15}$ Wasserstoff, $C_1$-$C_{12}$-Alkanoyl, durch Sauerstoff unterbrochenes $C_3$-$C_{12}$-Alkanoyl; Cyclohexylcarbonyl, Benzoyl, Naphthoyl, $C_1$-$C_{12}$-Alkansulfonyl, durch Fluor substituiertes $C_1$-$C_{12}$-Alkansulfonyl; Phenylsulfonyl oder durch $C_1$-$C_4$-Alkyl substituiertes Phenylsulfonyl; oder

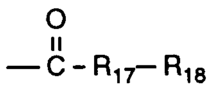

bedeutet,

$R_{17}$ -NH- darstellt, und

$R_{18}$ $C_1$-$C_8$-Alkyl oder Phenyl ist.

Ebenfalls von ganz speziell bevorzugtem Interesse ist ein Verfahren zur Herstellung von Verbindungen der Formel I, worin

$R_{15}$ -OR'$_{15}$ darstellt,

R'$_{15}$ Wasserstoff, $C_1$-$C_4$-Alkanoyl oder

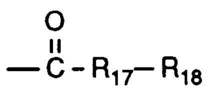

bedeutet,

$R_{17}$ -NH- darstellt, und

$R_{18}$ $C_1$-$C_4$-Alkyl ist.

Bevorzugte Reaktionsbedingungen des erfindungsgemässen Verfahrens sind die folgenden:

Die Reaktion kann bei erhöhter Temperatur, insbesondere Temperaturen von 70 bis 200°C in der Schmelze oder in einem Lösungsmittel gegebenenfalls unter leichtem Vakuum durchgeführt werden.

Besonders bevorzugt wird die Reaktion im Siedebereich der eingesetzten Verbindung der Formel IV durchgeführt.

Bevorzugt wird als Lösungsmittel die Verbindung der Formel IV, die gleichzeitig den Reaktionspartner darstellt, verwendet.

Als Lösungsmittel können aber auch solche verwendet werden, die sich an der Reaktion nicht beteiligen, wie beispielsweise halogenierte Kohlenwasserstoffe, Kohlenwasserstoffe, Ether oder desaktivierte Aromaten.

Bevorzugte halogenierte Kohlenwasserstoffe sind beispielsweise Dichlormethan, 1,2-Dichlorethan, Chloroform oder Tetrachlorkohlenstoff.

Bevorzugte Kohlenwasserstoffe sind beispielsweise Oktan und die kommerziell erhältlichen Isomeren-Fraktionen wie beispielsweise Hexan-Fraktion, Siedebenzin oder Ligroin.

Bevorzugte Ether sind beispielsweise Dibutylether, Methyl-tert-butylether oder Diethylenglykoldimethylether.

Beispiele für desaktivierte Aromaten sind Nitrobenzol oder Pyridin.

Bedeutet in der Verbindung der Formel III $R'_{15}$ Wasserstoff (3-Hydroxy-3H-benzofuran-2-one), wird das Wasser, das bei der Reaktion entsteht, zweckmässig kontinuierlich entfernt. Bevorzugt geschieht dies durch Zugabe eines Mittels, das Wasser absorbiert, wie beispielsweise Molekularsiebe. Ganz besonders bevorzugt wird das Wasser durch Destillation über einen Wasserabscheider kontinuierlich azeotrop entfernt.

Von Interesse ist auch ein Verfahren zur Herstellung von Verbindungen der Formel I, worin die Umsetzung in Gegenwart eines Katalysators durchgeführt wird.

Als Katalysatoren eignen sich Protonensäuren, Lewis-Säuren, Aluminiumsilikate, Ionenaustauscherharze, Zeolithe, natürlich vorkommende Schichtsilikate oder modifizierte Schichtsilikate.

Geeignete Protonensäuren sind beispielsweise Säuren von anorganischen oder organischen Salzen, wie z.B. Salzsäure, Schwefelsäure, Phosphorsäure, Methansulfonsäure, p-Toluolsulfonsäure oder Carbonsäuren wie z.B. Essigsäure. Besonders bevorzugt ist die p-Toluolsulfonsäure.

Geeignete Lewis-Säuren sind beispielsweise Zinntetrachlorid, Aluminiumchlorid, Zinkchlorid oder Bortrifluorid-etherat. Zinntetrachlorid und Aluminiumchlorid sind speziell bevorzugt.

Geeignete Aluminiumsilikate sind beispielsweise solche, die in der Petrochemie weit verbreitet sind und auch als amorphe Aluminiumsilikate bezeichnet werden. Diese Verbindungen enthalten ca. 10-30 % Siliciumoxid und 70-90 % Aluminiumoxid. Ein besonders bevorzugtes Aluminiumsilikat ist HA-HPV® von Ketjen (Akzo).

Geeignete Ionenaustauscherharze sind beispielsweise Styrol-Divinylbenzol-Harze, die noch Sulfosäuregruppen tragen, wie z.B. Amberlite 200® und Amberlyst® von Rohm und Haas oder Dowex 50® von Dow Chemicals; perfluorierte Ionenaustauscherharze, wie z.B. Nafion H® von DuPont; oder andere supersaure Ionenaustauscherharze wie von T. Yamaguchi, Applied Catalysis, 61, 1-25 (1990) oder M. Hino et al., J. Chem. Soc. Chem. Commun. 1980, 851-852 beschrieben.

Geeignete Zeolithe sind beispielsweise solche, die in der Petrochemie als Cracking-Katalysatoren weit verbreitet sind und als kristalline Silicium-Aluminiumoxide mit unterschiedlicher Kristallstruktur bekannt sind. Besonders bevorzugt sind die Faujasite der Firma Union Carbide wie beispielsweise Zeolith X® Zeolith Y® und ultrastabiler Zeolith Y®; Zeolith Beta® und Zeolith ZSM-12® von der Firma Mobil Oil Co.; und Zeolith Mordenit® von der Firma Norton.

Geeignete natürlich vorkommende Schichtsilikate werden auch als "Saure Erden" bezeichnet und sind beispielsweise Bentonite oder Montmorillonite, die grosstechnisch abgebaut, gemahlen, mit Mineralsäuren behandelt und kalziniert werden. Besonders geeignete natürlich vorkommende Schichtsilikate sind die Fulcat®-Typen der Firma Laporte Adsorbents Co., wie z.B. Fulcat 22A®, Fulcat 22B®, Fulcat 20®, Fulcat 30® oder Fulcat 40®; oder die Fulmont®-Typen der Firma Laporte Adsobents Co., wie z.B. Fulmont XMP-3® oder Fulmont XMP-4®. Ein speziell bevorzugter Katalysator für das erfindungsgemässe Verfahren ist Fulcat 22B®. Die anderen Fulcat®-Typen und Fulmont®-Typen sind aber ebenfalls in diese bevorzugte Klasse einzuordnen, weil es nur geringfügige Unterschiede zwischen den einzelnen Typen gibt, wie beispielsweise in der Anzahl der sauren Zentren.

Modifizierte Schichtsilikate werden auch als "Pillared Clays" bezeichnet und leiten sich von den oben beschriebenen natürlich vorkommenden Schichtsilikaten ab, indem sie zwischen den Silicatschichten noch Oxide von beispielsweise Zirkon, Eisen, Zink, Nickel, Chrom, Cobalt oder Magnesium enthalten. Dieser Katalysator-Typ ist in der Literatur, wie z.B. von J. Clark et al., J. Chem. Soc. Chem. Commun. 1989, 1353-1354 beschrieben, weit verbreitet, wird aber nur von sehr wenigen Firmen hergestellt. Besonders bevorzugte modifizierte Schichtsilikate sind beispielsweise Envirocat EPZ-10®, Envirocat EPZG® oder Envirocat EPIC® von der Firma Contract Chemicals.

Speziell bevorzugt ist auch ein Verfahren zur Herstellung von Verbindungen der Formel I, worin die Umsetzung in Gegenwart eines Katalysators, der ein natürlich vorkommendes Schichtsilikat oder ein modifiziertes Schichtsilikat bedeutet, durchgeführt wird.

Von ganz speziellem Interesse ist auch ein Verfahren zur Herstellung von Verbindungen der Formel I, worin die Umsetzung in Gegenwart eines Katalysators, der ein Fulcat®-Typ bedeutet, durchgeführt wird.

Der Katalysator wird zweckmässig in einer Menge von 1 bis 60 Gew.-%, im Falle eines besonders bevorzugten Fulcat®-Typ-Katalysators in einer Menge von 1 bis 30 Gew.-%, bezüglich eingesetzter Verbindung der Formel III, verwendet.

Von besonderem Interesse ist auch ein Verfahren zur Herstellung von Verbindungen der Formel I, worin, wenn n 1 ist, das molare Mengenverhältnis der Verbindung der Formel III zur Verbindung der Formel IV 1:1 bis 1:20 beträgt, und wenn n 2 ist, das molare Mengenverhältnis der Verbindung der Formel III zur Verbindung der Formel IV 3:1 bis 2:1 beträgt.

Bei dem erfindungsgemässen Verfahren können mit Verbindungen der Formel IV, die auch bei anderen bekannten elektrophilen Substitutionsreaktionen Isomerengemische liefern, ebenfalls Isomerengemische von Verbindungen der Formel I entstehen. Die relative Isomerenverteilung richtet sich nach den allgemein bekannten Grundregeln der organischen Chemie für elektrophile aromatische Substitutionsreaktionen.

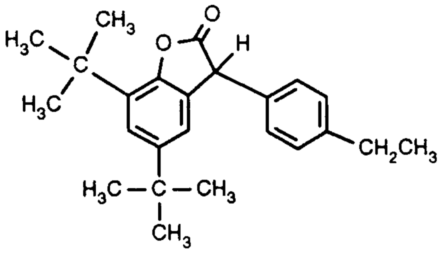

(105)

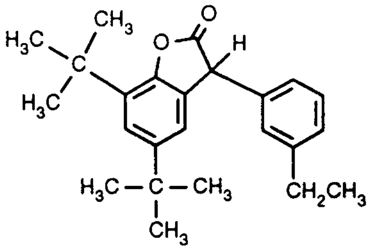

(105A)

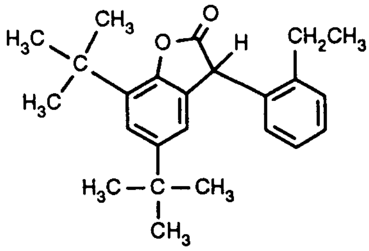

(105B)

Wie in Beispiel 4 beschrieben, wird beispielsweise ausgehend von 5,7-Di-tert-butyl-3-hydroxy-3H-benzofuran-2-on (Verbindung (201), Tabelle 2) mit Ethylbenzol, sowie Fulcat 22B als Katalysator, 59,2 % des para-Isomeren (Verbindung (105). Tabelle 1), 10,8 % des meta-Isomeren (Verbindung (105A) und 21,1 % des ortho-Isomeren (Verbindung (105B) erhalten.

Die Isomeren können durch fraktionierte Kristallisation oder Chromatographie an beispielsweise Kieselgel gereinigt und getrennt werden. Als Stabilisatoren für organische Materialien werden bevorzugt die Isomerengemische eingesetzt.

Die Verbindungen der Formel IV sind bekannt und zum Teil käuflich oder können nach an sich bekannten Verfahren erhalten werden.

Die Verbindungen der Formel III, worin $R'_{15}$ Wasserstoff bedeutet, können, wie von H. Sterk et al., Monatshefte für Chemie 99, 2223 (1968) beschrieben, zum Teil in ihren tautomeren Formen der Formel IIIa oder Formel IIIb

(IIIa)

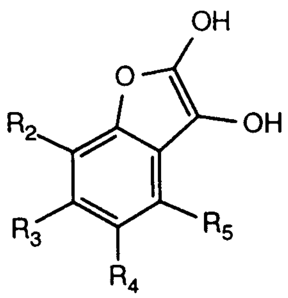

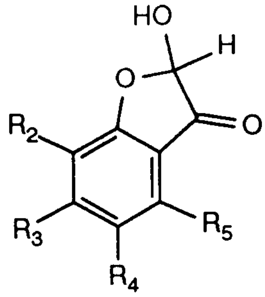

(IIIb)

vorliegen. Im Rahmen dieser Anmeldung ist die Formel III in allen Fällen so zu verstehen, dass sie auch die beiden obigen tautomeren Formeln IIIa und IIIb mitumfassen.

Die Verbindungen der Formel III können in Analogie zu den eingangs erwähnten Literaturstellen hergestellt werden. Bevorzugt wird jedoch ein neues Verfahren, das Gegenstand einer Parallel-Patentanmeldung ist, dadurch gekennzeichnet, dass

a) ein Aequivalent Phenol der Formel VII

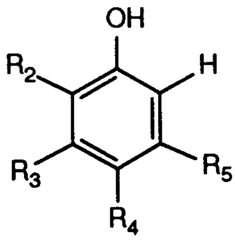

(VII)

worin die allgemeinen Symbole wie in Formel I definiert sind, mit 0,8 bis 2,0 Aequivalent Glyoxylsäure, insbesondere 0,8 bis 1,2 Aequivalent Glyoxylsäure, zu einer Verbindung der Formel VIII

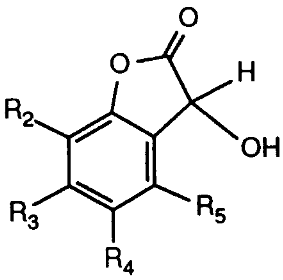

(VIII)

worin die allgemeinen Symbole wie in Formel I definiert sind, umgesetzt wird, und

b) zur Herstellung von Verbindungen der Formel I, worin $R'_{15}$ nicht Wasserstoff bedeutet, die erhaltene Verbindung der Formel VIII mit einer Halogenwasserstoffsäure, einem Halogenid einer Schwefel-Sauerstoffsäure, einem Halogenid der Phosphorsäure, einem Halogenid der phosphorigen Säure, einer Säure der Formel IX

$$R'_{15}\text{-OH} \qquad (IX)$$

einem Säurehalogenid der Formel X

$$R'_{15}\text{-Y} \qquad (X)$$

einem Ester der Formel XI

$$R'_{15}\text{-O-}R_{38} \qquad (XI)$$

einem symmetrischen oder unsymmetrischen Anhydrid der Formel XII

$$R'_{15}\text{-O-}R'_{15} \qquad (XII)$$

oder einem Isocyanat der Formel XIII

$$R_{39}\text{-N=C=O} \qquad (XIII)$$

worin $R'_{15}$ die obige Bedeutung hat, mit der Bedingung, dass $R'_{15}$ in den Verbindungen der Formeln IX, X, XI und XII nicht Wasserstoff bedeutet;
$R_{38}$ $C_1$-$C_8$-Alkyl darstellt,
$R_{39}$ $C_1$-$C_{18}$-Alkyl oder Phenyl bedeutet, und
Y Fluor, Chlor, Brom oder Iod bedeutet, umgesetzt wird.

Die Glyoxylsäure kann entweder kristallin oder vorteilhaft in Form der handelsüblichen wässrigen Lösung, in der Regel 40 bis 60 % wässrigen Lösung, eingesetzt werden.

Von besonderem Interesse ist auch ein Verfahren zur Herstellung von Verbindungen der Formel VIII, worin die Glyoxylsäure als 40 bis 60 % wässrige Glyoxylsäure, insbesondere 50 % wässrige Glyoxylsäure, verwendet wird.

Das in der Glyoxylsäure vorhandene Wasser sowie das Reaktionswasser wird während der Umsetzung abdestilliert. Dabei wird vorteilhaft ein mit Wasser azeotrop siedendes Lösungsmittel verwendet.

Geeignete mit Wasser azeotrop siedende Lösungsmittel sind beispielsweise Kohlenwasserstoffe wie z.B. Cyclohexan oder Methylcyclohexan; Aromaten wie z.B. Benzol oder Toluol; halogenierte Kohlenwasserstoffe wie z.B. 1,2-Dichlorethan; oder Aether wie z.B. Methyl-tert-butylether.

Wird die Umsetzung des Phenols der Formel VII mit Glyoxylsäure ohne Lösungsmittel zu den Verbindungen der Formel VIII in der Schmelze durchgeführt, wird das Reaktionswasser zweckmässig bei Normaldruck, insbesondere vorteilhaft unter leichtem Vakuum, abdestilliert.

Die Reaktion wird bevorzugt bei erhöhter Temperatur, insbesondere in einem Temperaturbereich von 60 bis 120°C, durchgeführt. Ein speziell bevorzugter Temperaturbereich ist 60 bis 90°C.

Die Reaktion kann durch Zusatz einer geringen Menge einer Protonensäure wie beispielsweise p-Toluolsulfonsäure, Methansulfonsäure, Schwefelsäure oder Salzsäure; oder einer Lewis-Säure wie beispielsweise Bortrifluorid-etherat oder Aluminiumchlorid katalysiert werden.

Die Menge an Katalysator beträgt 0,01 bis 5 Mol-%, bevorzugt 0,1 bis 1,0 Mol-%, bezüglich eingesetztem Phenol der Formel VII.

Die Reaktionsbedingungen für den Verfahrensschritt b) zur Herstellung von Verbindungen der Formel III, worin $R'_{15}$ nicht Wasserstoff bedeutet, ausgehend von Verbindungen der Formel VIII, sind allgemein bekannt und können beispielsweise in Analogie zu Veresterungsvorschriften gemäss Organikum 1986, Seiten 186-191, Seite 388 und Seiten 402-408, ausgewählt werden.

Geeignete Halogenwasserstoffsäuren sind beispielsweise Salzsäure, Bromwasserstoffsäure oder Jodwasserstoffsäure. Salzsäure ist bevorzugt.

Geeignete Halogenide einer Schwefel-Sauerstoffsäure sind beispielsweise Thionylchlorid, Sulfurylchlorid oder Thionylbromid. Thionylchlorid ist bevorzugt.

Geeignete Halogenide der Phosphorsäure und der phosphorigen Säure sind beispielsweise Phosphortrichlorid, Phosphortribromid, Phosphortrijodid, Phosphorpentachlorid, Phosphoroxychlorid oder Phosphorpentafluorid. Besonders bevorzugt ist Phosphoroxychlorid.

Im Verfahrensschritt b) wird bevorzugt ein Halogenid einer Schwefel-Wasserstoffsäure, wie beispielsweise Thionylchlorid; ein Säurehalogenid der Formel X; ein Ester der Formel XI; oder ein symmetrisches Anhydrid der Formel XII verwendet.

Wird im Verfahrensschritt b) ein Halogenid einer Schwefel-Wasserstoffsäure wie beispielsweise Thionylchlorid verwendet, wird die Umsetzung mit einer Verbindung der Formel VIII bevorzugt ohne Lösungsmittel und bei einer Temperatur von 0 bis 40°C, insbesondere Raumtemperatur durchgeführt. Das Thionylchlorid wird zweckmässig in einem 2 bis 10 fachen, insbesondere 2 bis 6 fachen Ueberschuss bezüglich eingesetzter Verbindung der Formel VIII verwendet. Die Reaktion kann auch in Gegenwart eines Katalysators wie beispielsweise Dimethylformamid durchgeführt werden.

Wird im Verfahrensschritt b) eine Säure der Formel IX ($R'_{15}$-OH) verwendet, wird die Umsetzung mit einer Verbindung der Formel VIII bevorzugt in Gegenwart eines inerten organischen Lösungsmittels wie beispielsweise Dichlormethan, Dioxan, Diethylether oder Tetrahydrofuran, und in Gegenwart eines Reagens, das Wasser physikalisch oder chemisch bindet, wie beispielsweise Molekularsieb oder Dicyclohexylcarbodiimid, durchgeführt.

Wird im Verfahrensschritt b) ein Säurehalogenid der Formel X ($R'_{15}$-Y) verwendet, worin Y bevorzugt Chlor oder Brom, insbesondere Chlor, bedeutet, wird die Umsetzung mit einer Verbindung der Formel VIII bevorzugt in Gegenwart eines Lösungsmittels und einer Base durchgeführt.

Die Base kann in unterschiedlichen Mengen eingesetzt werden, von katalytischen über stöchiometrische Mengen bis hin zu mehrfachem molarem Überschuß bezüglich der Verbindung der Formel VIII. Der beispielsweise bei der Reaktion gebildete Chlorwasserstoff wird gegebenenfalls durch die Base in Chlorid überführt, das durch Filtration und/oder Waschen mit einer geeigneten wässrigen oder festen Phase entfernt werden kann; dabei kann auch ein zweites, nicht mit Wasser mischbares Lösungsmittel eingesetzt werden. Die Reinigung des Produktes erfolgt zweckmäßig durch Umkristallisation des Rückstandes der eingeengten oder zur Trockne eingedampften organischen Phase.

Geeignete Lösungsmittel zur Durchführung der Reaktion sind u.a. Kohlenwasserstoffe (beispielsweise Toluol, Xylol, Hexan, Pentan oder weitere Petroletherfraktionen), halogenierte Kohlenwasserstoffe (beispielsweise Di- oder Trichlormethan, 1,2-Dichlorethan, 1,1,1-Trichlorethan), Ether (z.B. Diethylether, Dibutylether oder Tetrahydrofuran), ferner Acetonitril, Dimethylformamid, Dimethylsulfoxid, N-Methylpyrrolidon.

Geeignete Basen sind u.a. tertiäre Amine, z.B. Trimethylamin, Triethylamin, Tributylamin, N,N-Dimethylanilin, N,N-Diethylanilin; Pyridine; Hydride (z.B. Lithium-, Natrium-, Kaliumhydrid) oder Alkoholate (z.B. Natriummethylat).

Wird im Verfahrensschritt b) ein Ester der Formel XI ($R'_{15}$-O-$R_{38}$) verwendet, worin $R_{38}$ bevorzugt $C_1$-$C_4$-Alkyl, insbesondere Methyl oder Ethyl bedeutet, wird die Umsetzung mit einer Verbindung der Formel VIII bevorzugt in

Gegenwart eines Lösungsmittels, das mit Alkoholen azeotrope Gemische bildet, durchgeführt. Der bei der Reaktion entstehende Alkohol ($R_{38}$-OH) kann zweckmässig kontinuierlich abdestilliert werden.

Geeignete mit Alkoholen azeotrop siedende Lösungsmittel beteiligen sich nicht an der Umsetzung und sind beispielsweise Kohlenwasserstoffe wie z.B. Cyclohexan; Aromaten wie z.B. Benzol oder Toluol; halogenierte Kohlenwasserstoffe wie z.B. 1,2-Dichlorethan; oder Aether wie z.B. Methyl-tert-butylether.

Die Reaktion kann durch Zusatz einer geringen Menge einer Protonensäure wie beispielsweise p-Toluolsulfonsäure, Methansulfonsäure, Schwefelsäure oder Salzsäure; sowie einer Lewis-Säure wie beispielsweise Bortrifluorid-etherat oder Aluminiumchlorid katalysiert werden.

Wird im Verfahrensschritt b) ein symmetrisches Anhydrid der Formel XII ($R'_{15}$-O-$R'_{15}$) verwendet, worin $R'_{15}$ bevorzugt $C_2$-$C_6$-Alkanoyl, insbesondere Acetyl, bedeutet, wird die Umsetzung mit einer Verbindung der Formel VIII bevorzugt ohne Zugabe eines weiteren Lösungsmittels und bei einer Temperatur von 20 bis 200°C, z. B. Siedetemperatur des Anhydrids der Formel XII, insbesondere 60 bis 180°C, durchgeführt.

Wird im Verfahrensschritt b) ein Isocyanit der Formel XIII ($R_{39}$-N=C=O) verwendet, wird die Umsetzung der Formel VIII bevorzugt ohne Zugabe eines weiteren Lösungsmittels und bei einer Temperatur von 20 bis 200°C, z.B. Siedetemperatur des Isocyanats der Formel XIII, insbesondere 60 bis 180°C, durchgeführt.

Die Umsetzung mit einem Isocyanat wird ebenfalls bevorzugt in Gegenwart eines Katalysators durchgeführt. Bevorzugte Katalysatoren entsprechen denjenigen, wie sie bereits für die Umsetzung der Verbindung der Formel III mit einer Verbindung der Formel IV oben erwähnt werden.

Die Phenole der Formel VII sind bekannt oder können nach an sich bekannten Verfahren erhalten werden.

Bisphenolverbindungen der Formel XIV

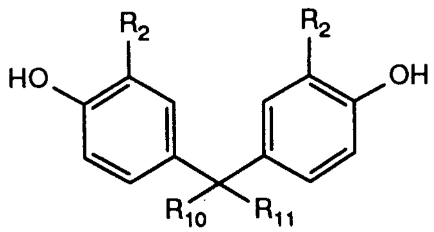

(XIV)

können gemäss Houben-Weyl, Methoden der organischen Chemie, Band 6/1c, 1030, hergestellt werden.

Die Verbindungen der Formel I können auch in einem sogenannten Ein-Topf-Verfahren ausgehend von den Phenolen der Formel VII hergestellt werden.

Die vorliegende Erfindung betrifft daher auch ein Verfahren zur Herstellung von Verbindungen der Formel I

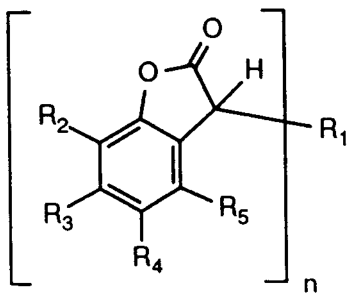

(I)

dadurch gekennzeichnet, dass ein Aequivalent Phenol der Formel VII

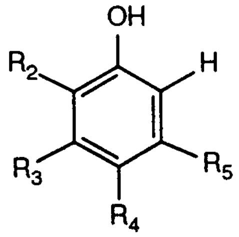

(VII)

mit 0,8 bis 2,0 Aequivalent Glyoxylsäure zu einer Verbindung der Formel VIII

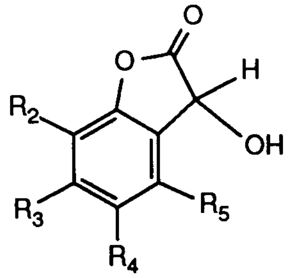

(VIII)

umgesetzt wird, und diese anschliessend ohne Isolierung mit einer Verbindung der Formel IV

$$[H]_n\text{-}R_1 \quad (IV)$$

zur Reaktion gebracht wird.

Die Definitionen der allgemeinen Symbole für das erfindungsgemässe Ein-Topf-Verfahren sind die gleichen wie für das andere vorgängig diskutierte erfindungsgemässe Verfahren.

Die bevorzugten Reaktionsparameter des Ein-Topf-Verfahrens entsprechen den Bevorzugungen für die zwei Einzelschritte, die bereits ausführlich diskutiert wurden.

Die beim Ein-Topf-Verfahren zuerst gebildeten 3-Hydroxy-3H-benzofuran-2-one der Formel VIII können vor der weiteren Umsetzung mit einer Verbindung der Formel IV einem zusätzlichen Reaktionsschritt unterworfen werden, indem die Hydroxygruppe durch Halogen substituiert oder mit einer Abgangsgruppe aktiviert wird.

Die vorliegende Erfindung betrifft deshalb auch ein Ein-Topf-Verfahren zur Herstellung von Verbindungen der Formel I, dadurch gekennzeichnet, dass ein Aequivalent Phenol der Formel VII mit 0,8 bis 2,0 Aequivalent Glyoxylsäure zu den 3-Hydroxy-3H-benzofuran-2-onen der Formel VIII umgesetzt wird und ohne Isolierung vor der weiteren Umsetzung mit einer Verbindung der Formel IV in einem zusätzlichen Reaktionsschritt mit einer Halogenwasserstoffsäure, einem Halogenid einer Schwefel-Sauerstoffsäure, einem Halogenid der Phosphorsäure, einem Halogenid einer phosphorigen Säure, einer Säure der Formel IX,

$$R'_{15}\text{-OH} \quad (IX)$$

einem Säurehalogenid der Formel X,

$$R'_{15}\text{-Y} \quad (X)$$

einem Ester der Formel XI

$$R'_{15}\text{-O-}R_{38} \quad (XI)$$

einem symmetrischen oder unsymmetrischen Anhydrid der Formel XII

$$R'_{15}\text{-O-}R'_{15} \quad (XII)$$

oder einem Isocyanat der Formel XIII

$$R_{39}\text{-N=C=O} \qquad (XIII)$$

worin $R'_{15}$ in den Formeln IX, X, XI und XII nicht Wasserstoff bedeutet;

$R_{38}$ $C_1$-$C_8$-Alkyl darstellt,
$R_{39}$ $C_1$-$C_{18}$-Alkyl oder Phenyl ist, und
Y Fluor, Chlor, Brom oder Iod bedeutet, zu Verbindungen der Formel III

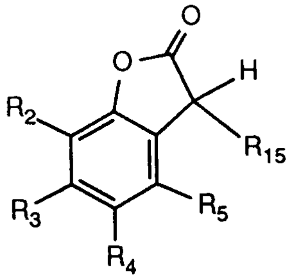

(III)

umgesetzt wird, worin, wenn $R_{15}$ = -OR'$_{15}$, $R'_{15}$ nicht Wasserstoff ist.

Die bevorzugten Reaktionsparameter in diesem zusätzlichen Reaktionsschritt entsprechen denjenigen wie sie bei der Herstellung der Verbindungen der Formel III ausgehend von Verbindungen der Formel VIII ausführlich beschrieben sind.

Ein speziell bevorzugtes Ein-Topf-Verfahren zur Herstellung von Verbindungen der Formel I ist dadurch gekennzeichnet, dass die eingesetzte Verbindung der Formel VII von der eingesetzten Verbindung der Formel IV verschieden ist.

Die folgenden Beispiele erläutern die Erfindung weiter. Angaben in Teilen oder Prozenten beziehen sich auf das Gewicht.

<u>Beispiel 1</u>:

Verfahren zur Herstellung von 5,7-Di-tert-butyl-3-(2,5-dimethylphenyl)-3H-benzofuran-2-on (Verbindung (101), Tabelle 1) ausgehend von 5,7-Di-tert-butyl-3-hydroxy-3H-benzofuran-2-on (Verbindung (201), Tabelle 2) mit p-Xylol, sowie Fulcat 22B als Katalysator.

a) Herstellung von 5,7-Di-tert-butyl-3-hydroxy-3H-benzofuran-2-on (Verbindung (201), Tabelle 2).

Eine Mischung von 212,5 g (1,00 Mol) 2,4-Di-tert-butyl-phenol (97 %ig), 163,0 g (1,10 Mol) 50 % wässrige Glyoxylsäure und 0,5 g (2,6 mMol) p-Toluolsulfonsäure Monohydrat in 300 ml 1,2-Dichlorethan wird unter Stickstoffatmosphäre während 3,5 Stunden am Wasserabscheider unter Rückfluss gekocht. Anschliessend wird das Reaktionsgemisch am Vakuumrotationsverdampfer eingeengt. Der Rückstand wird in 800 ml Hexan aufgenommen und dreimal mit Wasser gewaschen. Die wässrigen Phasen werden im Scheidetrichter abgetrennt und mit 300 ml Hexan nachextrahiert. Die organischen Phasen werden vereinigt, über Magnesiumsulfat getrocknet und am Vakuumrotationsverdampfer eingeengt. Der Rückstand liefert 262,3 g (~100 %) des analytisch reinen 5,7-Di-tert-butyl-3-hydroxy-3H-benzofuran-2-ons in Form eines dicken, gelblichen Harzes (Verbindung (201), Tabelle 2).

In Analogie zu Beispiel 1a werden aus den entsprechenden Phenolen wie beispielsweise 2-tert-Butyl-4-methyl-phenol; 4-tert-Butyl-2-methyl-phenol; 2,4-Dicyclohexyl-phenol; 2-(Hexadec-2-yl)-4-methyl-phenol, 3-[3-tert-Butyl-4-hydroxy-phenyl]-propionsäure, 2,4-Di($\alpha$,$\alpha$-dimethylbenzyl)-phenol und 4-Methyl-2-(1,1,3,3-tetramethyl-but-1-yl)-phenol mit Glyoxylsäure die Verbindungen (202), (203), (204), (205), (209), (210) und (211) hergestellt. Zur Herstellung der Verbindung (207) werden ausgehend vom 1,1-Bis-(3-tert-Butyl-4-hydroxy-phenyl)-cyclohexan zwei Aequivalente Glyoxylsäure eingesetzt.

b) Herstellung von 5,7-Di-tert-butyl-3-(2,5-dimethylphenyl)-3H-benzofuran-2-on (Verbindung (101), Tabelle 1).

Eine Lösung von 262,3 g (1,00 Mol) 5,7-Di-tert-butyl-3-hydroxy-3H-benzofuran-2-on (Verbindung (201), Tabelle 2, Beispiel 1a) in 500 ml (4,05 Mol) p-Xylol wird mit 40 g Fulcat 22B versetzt und während 1,5 Stunden am Wassserabscheider unter Rückfluss gekocht. Der Fulcat 22B-Katalysator wird anschliessend abfiltriert und das überschüssige p-Xylol am Vakuumrotationsverdampfer abdestilliert. Kristallisation des Rückstandes aus 400 ml

Methanol liefert 280,6 g (80 %) 5,7-Di-tert-butyl-3-(2,5-dimethylphenyl)-3H-benzofuran-2-on, Smp. 93-97°C (Verbindung (101), Tabelle 1).

Beispiel 2:

Verfahren zur Herstellung von 5,7-Di-tert-butyl-3-(2,5-dimethylphenyl)-3H-benzofuran-2-on (Verbindung (101), Tabelle 1) ausgehend von 3-Acetoxy-5,7-di-tert-butyl-3H-benzofuran-2-on (Verbindung (206), Tabelle 2) mit p-Xylol, sowie Fulcat 22B als Katalysator.

a) Herstellung von 3-Acetoxy-5,7-di-tert-butyl-3H-benzofuran-2-on (Verbindung (206), Tabelle 2).

Eine Mischung von 21,2 g (0,10 Mol) 2,4-Di-tert-butyl-phenol (97 %ig), 16,3 g (0,11 Mol) 50 % wässrige Glyoxylsäure und 0,05 g (0,26 mMol) p-Toluolsulfonsäure Monohydrat in 30 ml 1,2-Dichlorethan wird unter Stickstoffatmosphäre während 3,5 Stunden am Wasserabscheider unter Rückfluss gekocht. Anschliessend wird das Reaktionsgemisch am Vakuumrotationsverdampfer eingeengt. Der Rückstand wird mit 9,9 ml (0,105 Mol) Essigsäureanhydrid versetzt und während 90 Minuten unter Rückluss gekocht. Anschliessend wird das Reaktionsgemisch auf Raumtemperatur abgekühlt, mit 100 ml tert-Butyl-methylether verdünnt und nacheinander mit Wasser und verdünnter Natriumbicarbonat-Lösung gewaschen. Die wässrigen Phasen werden abgetrennt und mit 50 ml tert-Butyl-methylether nachextrahiert. Die organischen Phasen werden vereinigt, über Magnesiumsulfat getrocknet und am Vakuumrotationsverdampfer eingeengt. Chromatographie des Rückstandes an Kieselgel mit dem Laufmittelsystem Dichlormethan/Hexan = 2:1 liefert 28,0 g (92 %) 3-Acetoxy-5,7-di-tert-butyl-3H-benzofuran-2-on als dikkes, rötliches Harz (Verbindung (206), Tabelle 2).

b) Herstellung von 5,7-Di-tert-butyl-3-(2,5-dimethylphenyl)-3H-benzofuran-2-on (Verbindung (101), Tabelle 1).

Eine Lösung von 15,3 (50,0 mMol) 3-Acetoxy-5,7-di-tert-butyl-3H-benzofuran-2-on (Verbindung (206), Tabelle 2, Beispiel 2a) in 25 ml (0,20 Mol) p-Xylol wird mit 1,0 g Fulcat 22B versetzt und während 17 Stunden am Wasserabscheider unter Rückfluss gekocht. Der Fulcat 22B-Katalysator wird anschliessend abfiltriert und das überschüssige p-Xylol am Vakuumrotationsverdampfer abdestilliert. Kristallisation des Rückstandes aus 20 ml Methanol liefert 10,5 g (60 %) 5,7-Di-tert-butyl-3-(2,5-dimethylphenyl)-3H-benzofuran-2-on, Smp. 93-97°C (Verbindung (101), Tabelle 1).

Beispiel 3:

Verfahren zur Herstellung von 3-(3,4-Dimethylphenyl)-5,7-di-tert-butyl-3H-benzofuran-2-on (Verbindung (103), Tabelle 1) ausgehend von 5,7-Di-tert-butyl-3-hydroxy-3H-benzofuran-2-on (Verbindung (201), Tabelle 2) mit o-Xylol, sowie Fulcat 22B als Katalysator.

Eine Lösung von 262,3 g (1,00 Mol) 5,7-Di-tert-butyl-3-hydroxy-3H-benzofuran-2-on (Verbindung (201), Tabelle 2, Beispiel 1a) in 500 ml (4,05 Mol) o-Xylol wird mit 40 g Fulcat 22B versetzt und während 1,5 Stunden am Wassserabscheider unter Rückfluss gekocht. Der Fulcat 22B-Katalysator wird anschliessend abfiltriert und das überschüssige o-Xylol am Vakuumrotationsverdampfer abdestilliert. Kristallisation des Rückstandes aus 500 ml Methanol liefert 244 g (69 %) 3-(3,4-Dimethylphenyl)-5,7-di-tert-butyl-3H-benzofuran-2-on, Smp. 130-132°C (Verbindung (103), Tabelle 1), welche noch ca. 1,3 % des Strukturisomeren [3-(2,3-Dimethylphenyl)-5,7-di-tert-butyl-3H-benzofuran-2-on, Verbindung (103A)] enthält. Aus der Mutterlauge werden weitere 42,4 g Produkt gewonnen, welches aufgrund einer GC-MS-Analyse aus 12,3 % der Verbindung (103) und 87,7 % der isomeren Verbindung (103A) besteht.

Beispiel 4:

Verfahren zur Herstellung von 5,7-Di-tert-butyl-3-(4-ethylphenyl)-3H-benzofuran-2-on (Verbindung (105), Tabelle 1) ausgehend von 5,7-Di-tert-butyl-3-hydroxy-3H-benzofuran-2-on (Verbindung (201), Tabelle 2) mit Ethylbenzol, sowie Fulcat 22B als Katalysator.

Eine Lösung von 262,3 g (1,00 Mol) 5,7-Di-tert-butyl-3-hydroxy-3H-benzofuran-2-on (Verbindung (201), Tabelle 2, Beispiel 1a) in 500 ml (4,08 Mol) Ethylbenzol wird mit 40 g Fulcat 22B versetzt und während 1,5 Stunden am Wassserabscheider unter Rückfluss gekocht. Der Fulcat 22B-Katalysator wird anschliessend abfiltriert und das überschüssige Ethylbenzol am Vakuumrotationsverdampfer abdestilliert. Aufgrund einer GC-MS-Analyse liefert der Rückstand ein Gemisch von 59,2 % des para-Isomeren (Verbindung (105). Tabelle 1), 10,8 % des meta-Isomeren (Verbindung (105A) und 21,1 % des ortho-Isomeren (Verbindung (105B). Kristallisation des Rückstandes aus 400 ml Methanol liefert 163,8 g (47 %) 5,7-Di-tert-butyl-3-(4-ethylphenyl)-3H-benzofuran-2-on (Verbindung (105), Tabelle 1)(para-Isomeres), welches noch 5,6 % meta-Isomeres (5,7-Di-tert-butyl-3-(3-ethylphenyl)-3H-benzofuran-2-on (Verbindung (105A)) und 1,3 % ortho-Isomeres 5,7-Di-tert-butyl-3-(2-ethylphenyl)-3H-benzofuran-2-on (Verbindung (105B) enthält. Durch nochmalige Kristallisation aus Methanol kann das praktisch reine para-Isomere (Verbindung (105),

Tabelle 1), Smp. 127-132°C, erhalten werden.

In Analogie zu Beispiel 4 werden aus 5,7-Di-tert-butyl-3-hydroxy-3H-benzofuran-2-on (Verbindung (201), Tabelle 2, Beispiel 1a) und den entsprechenden Aromaten wie beispielsweise m-Xylol, Isopropylbenzol (Cumol), tert-Butylbenzol, 2,6-Dimethylanisol, Anisol, Acetoxyethoxybenzol, Chlorbenzol, Biphenyl, Thiophen, p-Xylol, Dibenzofuran, Phenanthren und Diphenylether die Verbindungen (102), (106), (107), (116), (117), (118), (120), (122), (123), (124), (125), (126) und (127) hergestellt. Zur Herstellung der Verbindung (127) werden ausgehend vom Diphenylether zwei Aequivalente 5,7-Di-tert-butyl-3-hydroxy-3H-benzofuran-2-on eingesetzt.

<u>Beispiel 5</u>:

Verfahren zur Herstellung von 5,7-Di-tert-butyl-3-(2,3,4,5,6-pentamethylphenyl)-3H-benzofuran-2-on (Verbindung (111), Tabelle 1) ausgehend von 5,7-Di-tert-butyl-3-hydroxy-3H-benzofuran-2-on (Verbindung (201), Tabelle 2) mit Pentamethylbenzol, sowie Zinntetrachlorid als Katalysator.

Eine Lösung von 19,7 g (75,0 mMol) 5,7-Di-tert-butyl-3-hydroxy-3H-benzofuran-2-on (Verbindung (201), Tabelle 2, Beispiel 1a) in 50 ml 1,2-Dichlorethan wird mit 11,5 g (77,5 mMol) Pentamethylbenzol und 10 ml (85,0 mMol) Zinntetrachlorid versetzt und anschliessend während einer Stunde am Rückfluss gekocht. Das Reaktionsgemisch wird mit Wasser versetzt und dreimal mit Toluol extrahiert. Die organischen Phasen werden abgetrennt, mit Wasser gewaschen, vereinigt, über Natriumsulfat getrocknet und am Vakuumrotationsverdampfer eingeengt. Kristallisation des Rückstandes aus Ethanol liefert 26,3 g (89 %) 5,7-Di-tert-butyl-3-(2,3,4,5,6-pentamethylphenyl)-3H-benzofuran-2-on, Smp. 185-190°C (Verbindung (111), Tabelle 1).

In Analogie zu Beispiel 5 werden aus 5,7-Di-tert-butyl-3-hydroxy-3H-benzofuran-2-on (Verbindung (201), Tabelle 2, Beispiel 1a) und den entsprechenden Aromaten wie beispielsweise n-Dodecylbenzol und 1,2,3-Trimethylbenzol die Verbindungen (109) und (110) hergestellt.

<u>Beispiel 6</u>:

Verfahren zur Herstellung von 5,7-Di-tert-butyl-3H-phenyl-3H-benzofuran-2-on (Verbindung (108), Tabelle 1) ausgehend von 5,7-Di-tert-butyl-3-hydroxy-3H-benzofuran-2-on (Verbindung (201), Tabelle 2) mit Benzol, sowie Aluminiumtrichlorid als Katalysator.

Eine Lösung von 131,2 g (0,50 Mol) 5,7-Di-tert-butyl-3-hydroxy-3H-benzofuran-2-on (Verbindung (201), Tabelle 2, Beispiel 1a) in 250 ml (2,82 Mol) Benzol wird während 25 Minuten mit 73,3 g (0,55 Mol) gemahlenem Aluminiumtrichlorid versetzt und während 1,5 Stunden auf Rückflusstemperatur aufgeheizt. Anschliessend wird noch während 1,5 Stunden unter Rückfluss gekocht. Das Reaktionsgemisch wird auf Raumtemperatur abgekühlt und vorsichtig unter Kühlen mit 200 ml Wasser und dann soviel konzentrierter Salzsäure versetzt bis ein homogenes zweiphasiges Gemisch entsteht. Die organische Phase wird abgetrennt, mit Wasser gewaschen, über Natriumsulfat getrocknet und am Vakuumrotationsverdampfer eingeengt. Kristallisation des Rückstandes aus Ethanol liefert 97,8 g (64 %) 5,7-Di-tert-butyl-3-phenyl-3H-benzofuran-2-on, Smp. 116-119°C (Verbindung (108), Tabelle 1).

In Analogie zu Beispiel 6 werden aus den entsprechenden 3-Hydroxy-3H-benzofuran-2-onen wie beispielsweise 7-[2-(Hexadec-2-yl)]-3-hydroxy-5-methyl-3H-benzofuran-2-on (Verbindung (205), Tabelle 2), 5,7-Dicyclohexyl-3-hydroxy-3H-benzofuran-2-on (Verbindung (204), Tabelle 2) und 5,7-Di-tert-butyl-3-hydroxy-3H-benzofuran-2-on (Verbindung (201), Tabelle 2) und den entsprechenden Aromaten wie beispielsweise Benzol und Thioanisol die Verbindungen (113), (114) und (119) hergestellt.

<u>Beispiel 7</u>:

Verfahren zur Herstellung von 5,7-Di-tert-butyl-3-(4-methylphenyl)-3H-benzofuran-2-on (Verbindung (104), Tabelle 1) ausgehend von 2,4-Di-tert-butylphenol ohne Isolierung von 5,7-Di-tert-butyl-3-hydroxy-3H-benzofuran-2-on (Verbindung (201), Tabelle 2) mit Glyoxylsäure und Toluol, sowie Fulcat 22B als Katalysator.

Ein Gemisch von 21,2 g (0,10 Mol) 2,4-Di-tert-butylphenol (97 %ig), 16,3 g( 0,11 Mol) 50 % wässrige Glyoxylsäure, 2,0 g Fulcat 22B und 50 ml Toluol wird unter einer Stickstoffatmosphäre während 8 Stunden am Wasserabscheider unter Rückfluss gekocht. Der Fulcat 22B-Katalysator wird anschliessend abfiltriert und das überschüssige Toluol am Vakuumrotationsverdampfer abdestilliert. Kristallisation des Rückstandes aus 40 ml Ethanol liefert 14,2 g (42 %) 5,7-Di-tert-butyl-3-(4-methylphenyl)-3H-benzofuran-2-on, Smp. 130-133°C (Verbindung (104), Tabelle 1).

In Analogie zu Beispiel 7 wird ausgehend von 2-tert-Butyl-4-methyl-phenol anstelle von 2,4-Di-tert-butylphenol die Verbindung (112) hergestellt.

Beispiel 8:

Verfahren zur Herstellung von 4,4'-Di-(5,7-di-tert-butyl-3H-benzofuran-2-on-3-yl)-N-methyl-diphenylamin (Verbindung (121), Tabelle 1) ausgehend von 5,7-Di-tert-butyl-3-hydroxy-3H-benzofuran-2-on (Verbindung (201), Tabelle 2) mit N-Methyl-diphenylamin, sowie p-Toluolsulfonsäure als Katalysator.

Zu einer siedenden Lösung von 9,20 g (50,0 mMol) N-Methyl-diphenylamin und 0,20 g p-Toluolsulfonsäure Monohydrat in 50 ml Ligroin (Alkangemisch mit Siedebereich 140-160°C) wird während 2 Stunden 30,2 g (115,0 mMol) 5,7-Di-tert-butyl-3-hydroxy-3H-benzofuran-2-on (Verbindung (201), Tabelle 2, Beispiel 1a) gegeben. Das Reaktionsgemisch wird anschliessend während 4 Stunden am Wasserabscheider unter Rückfluss gekocht. Dann wird das Reaktionsgemisch abgekühlt und am Vakuumrotationsverdampfer eingeengt. Zweimalige Kristallisation des Rückstandes aus Isopropanol/Wasser = 9:1 liefert 18,9 g (56 %) 4,4'-Di-(5,7-di-tert-butyl-3H-benzofuran-2-on-3-yl)-N-methyl-diphenylamin, Smp. 135-145°C (Verbindung (121), Tabelle 1).

Beispiel 9:

Verfahren zur Herstellung von 5,7-Di-tert-butyl-3-(3,5-dimethyl-4-hydroxyphenyl)-3H-benzofuran-2-on (Verbindung (115), Tabelle 1) ausgehend von 5,7-Di-tert-butyl-3-hydroxy-3H-benzofuran-2-on (Verbindung (201), Tabelle 2) mit 2,6-Dimethylphenol, sowie p-Toluolsulfonsäure als Katalysator.

Zu einer siedenden Lösung von 12,2 g (100,0 mMol) 2,6-Dimethylphenol und 0,20 g p-Toluolsulfonsäure Monohydrat in 50 ml Essigsäure wird während 2 Stunden 30,2 g (115,0 mMol) 5,7-Di-tert-butyl-3-hydroxy-3H-benzofuran-2-on (Verbindung (201), Tabelle 2, Beispiel 1a) gegeben. Das Reaktionsgemisch wird anschliessend während 4 Stunden unter Rückfluss gekocht. Dann wird das Reaktionsgemisch abgekühlt und am Vakuumrotationsverdampfer eingeengt. Zweimalige Kristallisation des Rückstandes aus Isopropanol/Wasser = 9:1 liefert 28,5 g (78 %) 5,7-Di-tert-butyl-3-(3,5-dimethyl-4-hydroxy-phenyl)-3H-benzofuran-2-on, Smp. 225-228°C (Verbindung (115), Tabelle 1).

Beispiel 10:

Verfahren zur Herstellung von 7-tert-Butyl-5-methyl-3-(9-methyl-9H-carbazol-3-yl)-3H-benzofuran-2-on (Verbindung (128), Tabelle 1) ausgehend von 7-tert-Butyl-3-hydroxy-5-methyl-3H-benzofuran-2-on (Verbindung (202), Tabelle 2) mit N-Methylcarbazol und n-Octan, sowie Fulcat 22B als Katalysator.

Ein Gemisch von 2,2 g (10,0 mMol) 7-tert-Butyl-3-hydroxy-5-methyl-3H-benzofuran-2-on (Verbindung (202), Beispiel 1a, Tabelle 2), 1,8 g( 10,0 mMol) N-Methylcarbazol und 0,2 g Fulcat 22B und 20 ml n-Octan wird unter einer Stickstoffatmosphäre während 5 Stunden unter Rückfluss gekocht. Der Fulcat 22B-Katalysator wird anschliessend abfiltriert und das überschüssige n-Octan am Vakuumrotationsverdampfer abdestilliert. Chromatographie des Rückstandes an Kieselgel mit dem Laufmittelsystem Dichlormethan/Hexan = 1:2 bis 1:1 und anschliessende Kristallisation der reinen Fraktionen aus Methanol liefert 0,70 g (10 %) 7-tert-Butyl-5-methyl-3-(9-methyl-9H-carbazol-3-yl)-3H-benzofuran-2-on, Smp. 84-90°C (Verbindung (128), Tabelle 1). Das Produkt kann in geringer Menge noch andere Strukturisomere bezüglich Substitution am Carbazolring enthalten.

Beispiel 11:

Verfahren zur Herstellung von 5,7-Di-tert-butyl-3-(9H-fluoren-3-yl)-3H-benzofuran-2-on (Verbindung (129), Tabelle 1) ausgehend von 2,4-Di-tert-butyl-phenol ohne Isolierung von 5,7-Di-tert-butyl-3-hydroxy-3H-benzofuran-2-on (Verbindung (201), Tabelle 2) mit Glyoxylsäure und Fluoren, sowie p-Toluolsulfonsäure und Fulcat 22B als Katalysator.

Ein Gemisch von 15,9 g (75 mMol) 2,4-Di-tert-butylphenol (97 %ig), 12,2 g( 82 mMol) 50 % wässrige Glyoxylsäure, 40 mg (0,20 mMol) p-Toluolsulfonsäure Monohydrat und 25 ml 1,2-Dichlorethan wird unter einer Stickstoffatmosphäre während 3,5 Stunden am Wasserabscheider unter Rückfluss gekocht. Anschliessend wird das Reaktionsgemisch am Vakuumrotationsverdampfer eingeengt. Der Rückstand wird in 30 ml n-Octan gelöst und mit 12,5 g (75 mMol) Fluoren und 3 g Fulcat 22B versetzt. Diese Mischung wird unter einer Stickstoffatmosphäre während 3,5 Stunden am Wasserabscheider unter Rückfluss gekocht Das Reaktionsgemisch wird abgekühlt, filtriert und das Filtrat am Vakuumrotationsverdampfer eingeengt. Chromatographie des Rückstandes an Kieselgel mit dem Laufmittelsystem Dichlormethan/Hexan = 2:1 und anschliessende Kristallisation der reinen Fraktionen aus Methanol liefert 5,28 g (17 %) 5,7-Di-tert-butyl-3-(9H-fluoren-3-yl)-3H-benzofuran-2-on, Smp. 140-153°C (Verbindung (129), Tabelle 1). Das Produkt kann in geringer Menge noch andere Strukturisomere bezüglich Substitution am Fluorenring enthalten.

Beispiel 12:

Verfahren zur Herstellung eines ca. 5,7:1 Isomerengemisches von 3-(3,4-Dimethylphenyl)-5,7-di-tert-butyl-3H-benzofuran-2-on (Verbindung (103), Tabelle 1) und 3-(2,3-Dimethylphenyl)-5,7-di-tert-butyl-3H-benzofuran-2-on (Verbindung (103A)) ausgehend von 2,4-Di-tert-butylphenol mit Glyoxylsäure und o-Xylol, sowie Fulcat oder Fulmont als Katalysator.

In einem 1,5 l Doppelmantelreaktor mit Wasserabscheider werden 206,3 g (1,0 Mol) 2,4-Di-tert-butylphenol, 485 g (5,5 Mol) o-Xylol, 0,5 g (2,6 mMol) p-Toluolsulfonsäure Monohydrat und 163 g (1,1 Mol) 50 % wässrige Glyoxylsäure vorgelegt. Das Gemisch wird unter Rühren auf 85-90°C erwärmt und die Apparatur gleichzeitig bis auf ca. 450 mbar evakuiert. Sobald die Innentemperatur 85-90°C erreicht, beginnt ein Gemisch aus o-Xylol/Wasser abzudestillieren, wobei das o-Xylol rückflussiert und das Wasser ausgekreist wird. Das Vakuum wird nun kontinuierlich erhöht, damit die Innentemperatur bei 85-90°C gehalten werden kann. Gesamthaft destillieren in 3 bis 4 Stunden ca. 98-100 ml Wasser ab. Das Vakuum wird nun mit Stickstoff entspannt und zu der gelben, klaren Lösung werden 40 g Katalysator (Fulcat 30 oder 40, Fulmont XMP-3 oder XMP-4) gegeben. Die Apparatur wird bis 700 mbar evakuiert und die Suspension wird bei einer Heizbadtemperatur von 165°C gerührt. Das gebildete Reaktionswasser beginnt ab einer Innentemperatur von ca. 128°C azeotrop abzudestillieren. Die Innentemperatur steigt gegen Ende auf maximal 140°C an. Insgesamt scheiden sich in 1 bis 2 Stunden ca. 20 ml Wasser ab. Das Vakuum wird nun mit Stickstoff entspannt. Das Reaktionsgemisch wird auf 90-100°C abgekühlt und filtriert. Die Apparatur und der Filterrückstand werden mit 100 g o-Xylol gespühlt. Das Filtrat wird in einen 1500 ml Doppelmantelreaktor überführt und unter Vakuum eingeengt. Dabei werden 360 g o-Xylol zurück gewonnen. Der rötlich gelbe Rückstand wird auf 70°C gekühlt und via Tropftrichter vorsichtig mit 636 g Methanol versetzt. Die Innentemperatur wird dabei bei 60-65°C gehalten. Die Lösung wird angeimpft und während ca. 30 Minuten unter Rühren bei 60-65°C auskristallisiert. Der Kristallbrei wird dann innerhalb 2 Stunden auf eine Innentemperatur von -5°C abgekühlt und bei dieser Temperatur eine weitere Stunde ausgerührt. Die Kristalle werden abgenutscht und der Rückstand mit 400 g kaltem (-5°C) Methanol in 5 Portionen gewaschen. Das gut trocken gezogene Produkt wird im Vakuumtrockenschrank bei 50-60°C getrocknet. Es werden 266 g eines weissen Feststoffs erhalten. Dieses Material besteht aufgrund einer gaschromatographischen Analyse aus ca. 85 % 3-(3,4-Dimethylphenyl)-5,7-di-tert-butyl-3H-benzofuran-2-on (Verbindung (103), Tabelle 1) sowie ca. 15 % des isomeren 3-(2,3-Dimethylphenyl)-5,7-di-tert-butyl-3H-benzofuran-2-on (Verbindung (103A)).

Beispiel 13:

Herstellung von 3-(N-Methylcarbamoyloxy)-5-methyl-7-tert-butyl-3H-benzofuran-2-on (Verbindung (212), Tabelle 2).

Eine Mischung von 5,5 g (25,0 mMol) 7-tert-Butyl-3-hydroxy-5-methyl-3H-benzofuran-2-on (Verbindung (202), Beispiel 1a), 3 ml (50,0 mMol) Methylisocyanat und 2 Tropfen Methansulfonsäure werden 3 1/4 Stunden unter Rückfluss gekocht. Anschliessend werden nochmals 3 ml (50,0 mMol) Methylisocyanat und 2 Tropfen Methansulfonsäure zugegeben. Nach weiteren 16 Stunden Rückfluss wird das Reaktionsgemisch abgekühlt, mit Dichlormethan verdünnt und mit Wasser und 5 % wässriger Natriumhydrogencarbonat-Lösung gewaschen. Die organischen Phasen werden vereiningt, über Magnesiumsulfat getrocknet und Vakuumrotationsverdampfer eingeengt. Kristallisations des Rückstandes aus Toluol liefert 4,45 g (65 %) 3-(N-Methylcarbamoyloxy)-5-methyl-7-tert-butyl-3H-benzofuran-2-on, Smp. 138-143°C (Verbindung (212), Tabelle 2).

Beispiel 14:

Herstellung von 7-tert-Butyl-3-chlor-5-methyl-3H-benzofuran-2-on (Verbindung (208), Tabelle 2).

Eine Suspension von 2,2 g (10,0 mMol) 7-tert-Butyl-3-hydroxy-5-methyl-3H-benzofuran-2-on (Verbindung (202), Beispiel 1a, Tabelle 2) in 2,4 ml (55,0 mMol) Thionylchlorid wird mit einem Tropfen Dimethylformamid versetzt und 2 Stunden bei Raumtemperatur gerührt. Das überschüssige Thionylchlorid wird anschliessend am Vakuumrotationsverdampfer abdestilliert. Chromatographie des Rückstandes an Kieselgel mit dem Laufmittelsystem Dichlormethan/Hexan = 1:1 und Kristallisation der reinen Fraktionen aus Methanol liefert 0,30 g (13 %) 7-tert-Butyl-3-chlor-5-methyl-3H-benzofuran-2-on, Smp. 81-86°C (Verbindung (208), Tabelle 2).

Tabelle 1:

| Nr. | Verbindung | Smp. (°C) | C (%), H (%) (berechnet/gefunden) | | Ausbeute (%) |
|---|---|---|---|---|---|
| 101 | 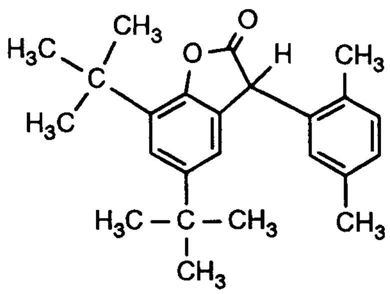 | 93-97 | 82,24<br>82,10 | 8,63<br>8,66 | 80 |
| 102 | 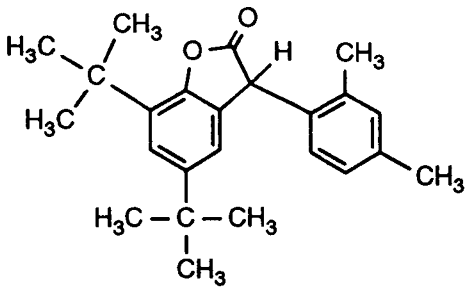 | 92-96 | 82,24<br>82,19 | 8,63<br>8,78 | 52[a)] |
| 103 | 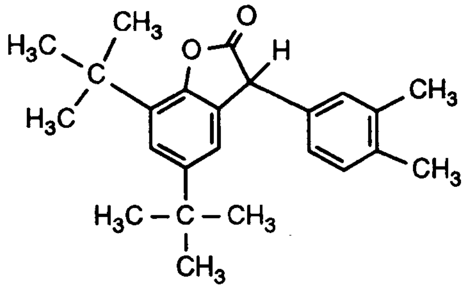 | 130-132 | 82,24<br>82,36 | 8,63<br>8,62 | 69[a)] |
| 104 | 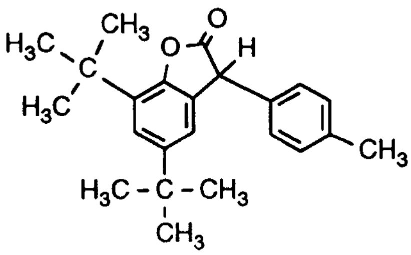 | 130-133 | 82,10<br>82,13 | 8,39<br>8,31 | 42[a)] |

a) Das Produkt kann in geringer Menge noch andere Strukturisomere bezüglich Substitution am Phenylring in 3-Stellung des Benzofuran-2-ons enthalten.

Tabelle 1: (Fortsetzung)

| Nr. | Verbindung | Smp. (°C) | C (%), H (%) (berechnet/gefunden) | Ausbeute (%) |
|---|---|---|---|---|
| 105 | 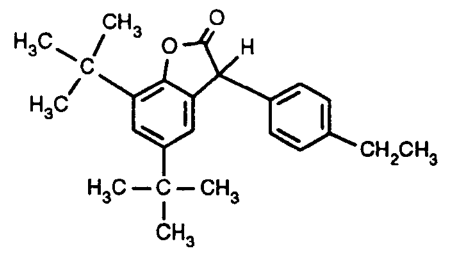 | 127-132 | 82,24 8,63<br>82,39 8,65 | 47[a)] |
| 106 | 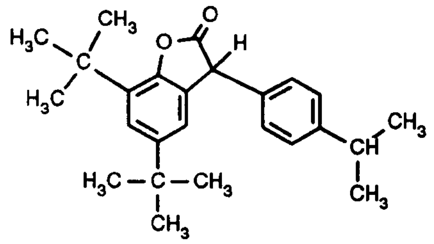 | 109-115 | 82,37 8,85<br>82,24 8,91 | 41[a)] |
| 107 | 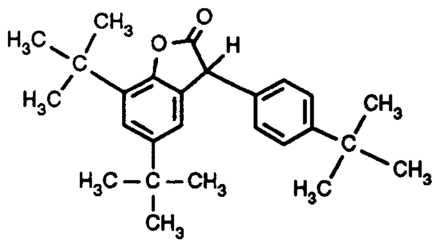 | 110-115 | 82,49 9,05<br>82,49 9,03 | 68[a)] |
| 108 | 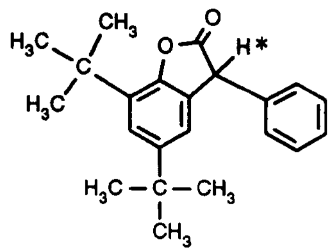 | 116-119 | Charakterisiert durch $^1$H-NMR ($CDCl_3$) δ(H*) = 4,84 ppm | 64 |

a) Das Produkt kann in geringer Menge noch andere Strukturisomere bezüglich Substitution am Phenylring in 3-Stellung des Benzofuran-2-ons enthalten.

Tabelle 1: (Fortsetzung)

| Nr. | Verbindung | Smp. (°C) | C (%), H (%) (berechnet/gefunden) | Ausbeute (%) |
|---|---|---|---|---|
| 109 | 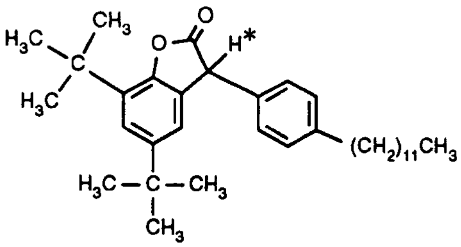 | Oel | Charakterisiert durch $^1$H-NMR ($CDCl_3$) $\delta(H^*)$ = 4,84 ppm | 66[a)] |
| 110 | 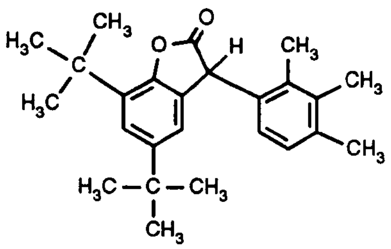 | 118-122 | 82,37 8,85<br>82,31 8,84 | 74[a)] |
| 111 | 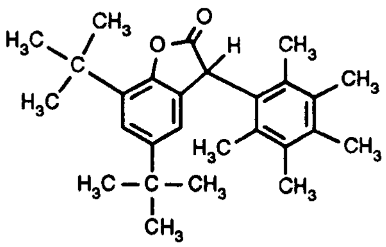 | 185-190 | 82,61 9,24<br>82,41 9,43 | 89 |
| 112 | 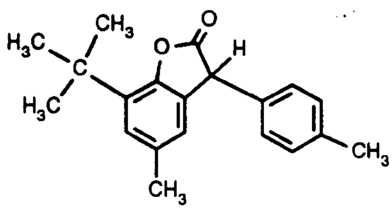 | 69-80 | 81,60 7,53<br>81,42 7,57 | 70[a)] |

a) Das Produkt kann in geringer Menge noch andere Strukturisomere bezüglich Substitution am Phenylring in 3-Stellung des Benzofuran-2-ons enthalten.

Tabelle 1: (Fortsetzung)

| Nr. | Verbindung | Smp. (°C) | C (%), H (%) (berechnet/gefunden) | Ausbeute (%) |
|---|---|---|---|---|
| 113 | 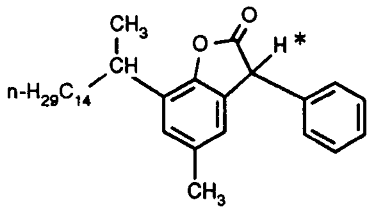 | Oel | Charakterisiert durch $^1$H-NMR ($CDCl_3$) δ(H*) = 4,85 ppm | 56 |
| 114 | 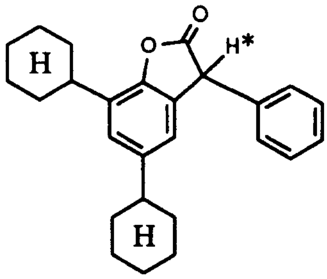 | Harz | Charakterisiert durch $^1$H-NMR ($CDCl_3$) δ(H*) = 4,86 ppm | 57 |
| 115 | 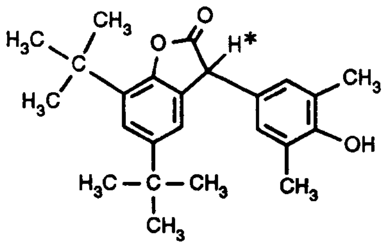 | 225-228 | Charakterisiert durch $^1$H-NMR ($CDCl_3$) δ(H*) = 4,70 ppm | 78[a)] |
| 116 | 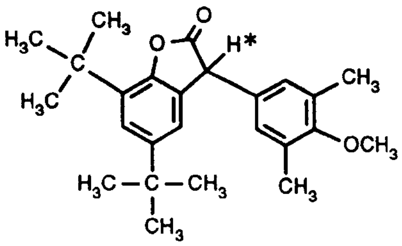 | 133-135 | Charakterisiert durch $^1$H-NMR ($CDCl_3$) δ(H*) = 4,72 ppm | 52[a)] |

a) Das Produkt kann in geringer Menge noch andere Strukturisomere bezüglich Substitution am Phenylring in 3-Stellung des Benzofuran-2-ons enthalten.

Tabelle 1: (Fortsetzung)

| Nr. | Verbindung | Smp. (°C) | C (%), H (%) (berechnet/gefunden) | Ausbeute (%) |
|---|---|---|---|---|
| 117 | 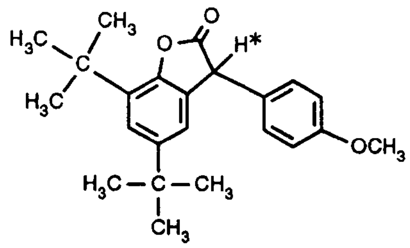 | 102-104 | Charakterisiert durch $^1$H-NMR ($CDCl_3$) $\delta(H^*) = 4,78$ ppm | 65[a)] |
| 118 | 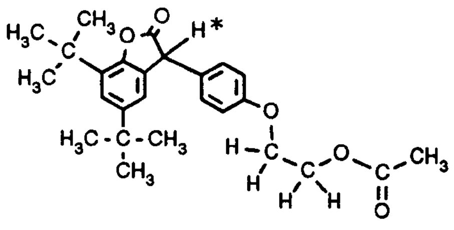 | 91-94 | Charakterisiert durch $^1$H-NMR ($CDCl_3$) $\delta(H^*) = 4,78$ ppm | 23[a)] |
| 119 | 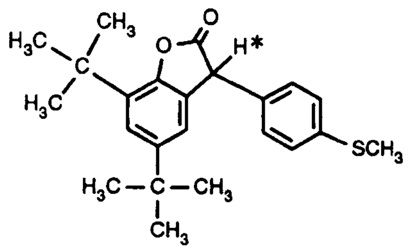 | 125-131 | Charakterisiert durch $^1$H-NMR ($CDCl_3$) $\delta(H^*) = 4,79$ ppm | 18[a)] |
| 120 | 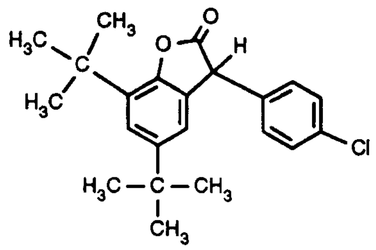 | 121-126 | 74,04 7,06<br>74,02 7,11 | 37[a)] |

a) Das Produkt kann in geringer Menge noch andere Strukturisomere bezüglich Substitution am Phenylring in 3-Stellung des Benzofuran-2-ons enthalten.

Tabelle 1: (Fortsetzung)

| Nr. | Verbindung | Smp. (°C) | C (%), H (%), N (%) (berechnet/gefunden) | Ausbeute (%) |
|---|---|---|---|---|
| 121 | 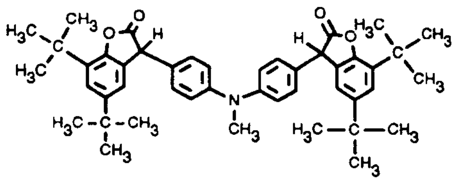 | 135-145 | 80,44 7,95 2,08<br>80,20 8,06 1,96 | 56[a)] |
| 122 | 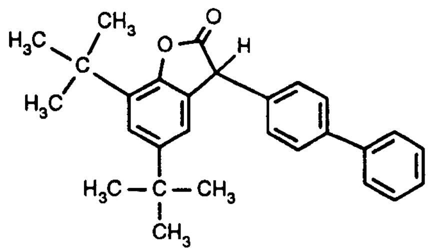 | 168-170 | 84,38 7,57<br>84,23 7,66 | 25[a)] |
| 123 | 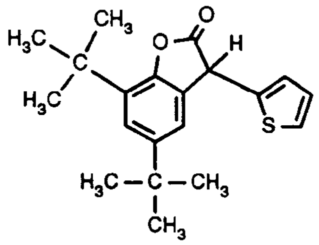 | 86-93 | C (%), H (%), S (%)<br>73,13 7,37 9,76<br>73,10 7,38 9,69 | 11[a)] |
| 124 | 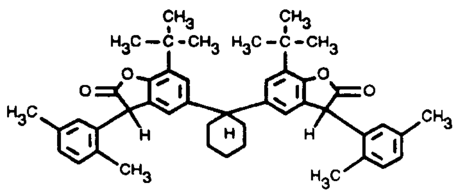 | 220-228 | 82,60 7,84<br>82,58 7,85 | 40 |

a) Das Produkt kann in geringer Menge noch andere Strukturisomere bezüglich Substitution am Phenylring in 3-Stellung des Benzofuran-2-ons enthalten.

Tabelle 1: (Fortsetzung)

| Nr. | Verbindung | Smp. (°C) | C (%), H (%) (berechnet/gefunden) | Ausbeute (%) |
|---|---|---|---|---|
| 125 | 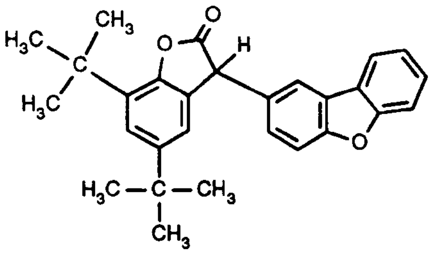 | 142-154 | 81,52 6,84<br>80,97 6,85 | 33[a)] |
| 126 | 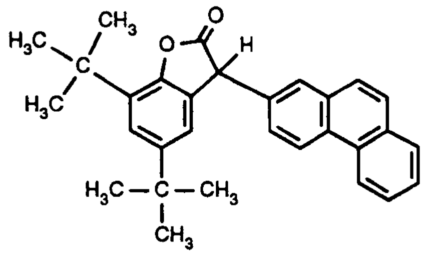 | 186-189 | 85,27 7,16<br>85,15 7,20 | 17[a)] |
| 127 | 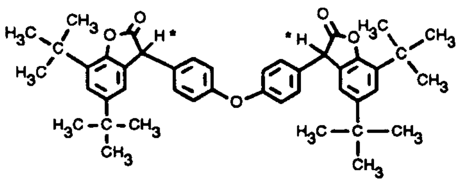 | Harz | Charakterisiert durch $^{1}$H-NMR ($CDCl_3$) δ(H*) = 4,82 ppm | 31[a)] |
| 128 | 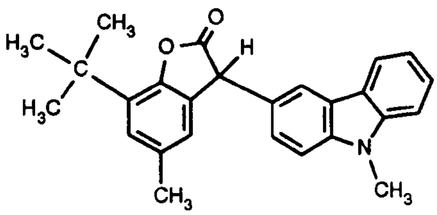 | 84-90 | 81,43 6,57<br>81,37 6,72 | 10[a)] |

a) Das Produkt kann in geringer Menge noch andere Strukturisomere bezüglich Substitution am Arylring in 3-Stellung des Benzofuran-2-ons enthalten.

Tabelle 1: (Fortsetzung)

| Nr. | Verbindung | Smp. (°C) | C (%), H (%) (berechnet/gefunden) | Ausbeute (%) |
|---|---|---|---|---|
| 129 | 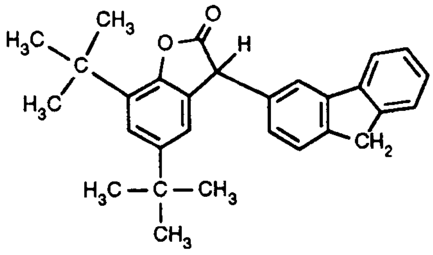 | 140-153 | 84,84 7,37<br>84,66 7,52 | 17[a)] |

a) Das Produkt kann in geringer Menge noch andere Strukturisomere bezüglich Substitution am Fluoren in 3-Stellung des Benzofuran-2-ons enthalten.

Tabelle 2:

| Nr. | Verbindung | Smp. (°C) | C (%), H (%) (berechnet/gefunden) | Ausbeute (%) |
|---|---|---|---|---|
| 201 | 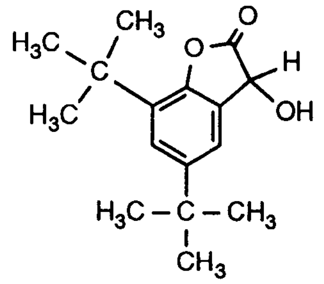 | Harz | 73,25 8,45<br>73,33 8,50 | 100 |
| 202 | 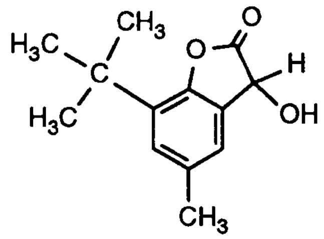 | 152-160 | 70,89 7,32<br>70,40 7,40 | 82 |
| 203 | 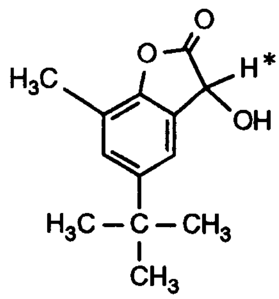 | Harz | Charakterisiert durch $^1$H-NMR ($CDCl_3$) δ(H*) = 5,33 ppm<br>a) Chromatographiert an Kieselgel ($CH_2Cl_2$/Hexan = 4 : 1) | 45[a)] |
| 204 | 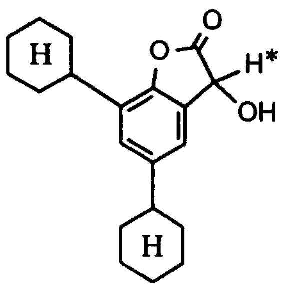 | Harz | Charakterisiert durch $^1$H-NMR ($CDCl_3$) δ(H*) = 5,30 ppm | ~100 |

Tabelle 2: (Fortsetzung)

| Nr. | Verbindung | Smp. (°C) | C (%), H (%) (berechnet/gefunden) | Ausbeute (%) |
|---|---|---|---|---|
| 205 | 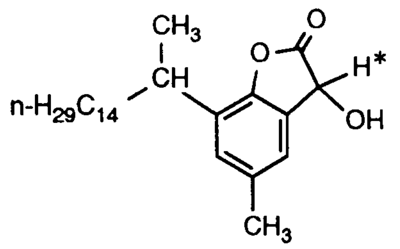 | Harz | Charakterisiert durch $^1$H-NMR ($CDCl_3$) δ(H*) = 5,31 ppm | 98 |
| 206 | 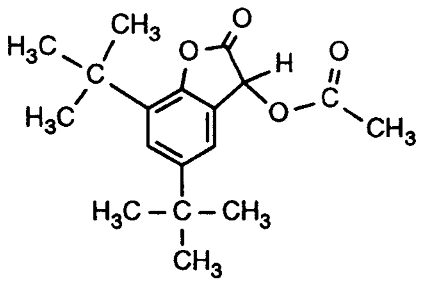 | Harz | 71,03 7,95<br>71,10 7,98 | 92 |
| 207 | 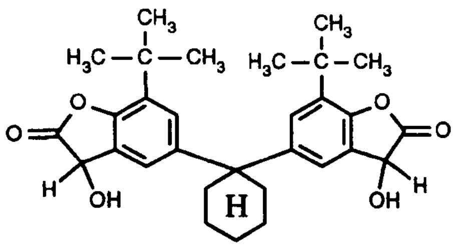 | Harz | Charakterisiert durch $^1$H-NMR ($CDCl_3$) δ(tert-Butyl) = 1,34 ppm | ~100 |
| 208 | 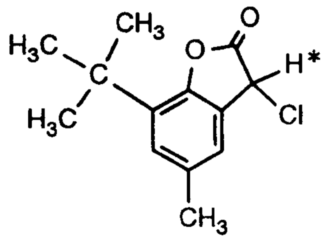 | 81-86 | Charakterisiert durch $^1$H-NMR ($CDCl_3$) δ(H*) = 5,34 ppm | 13 |

Tabelle 2: (Fortsetzung)

| Nr. | Verbindung | Smp. (°C) | C (%), H (%) (berechnet/gefunden) | Ausbeute (%) |
|---|---|---|---|---|
| 209 | 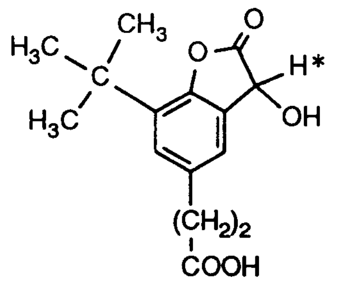 | Harz | Charakterisiert durch $^1$H-NMR ($CDCl_3$) δ(H*) = 5,29 ppm | ~100 |
| 210 | 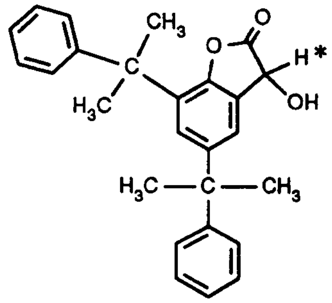 | Harz | Charakterisiert durch $^1$H-NMR ($CDCl_3$) δ(H*) = 5,08 ppm | 38 |
| 211 | 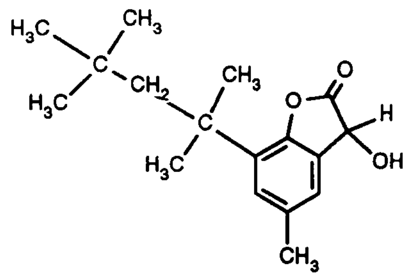 | 100-103 | 73,88 8,75<br>73,73 8,75 | 61 |
| 212 | 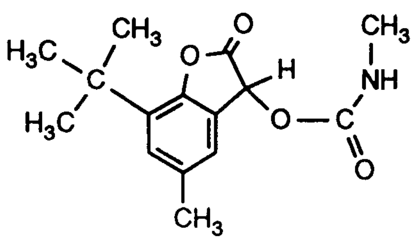 | 138-143 | 64,97 6,91<br>65,02 6,89 | 65 |

**Patentansprüche**

1. Verfahren zur Herstellung von Verbindungen der Formel I

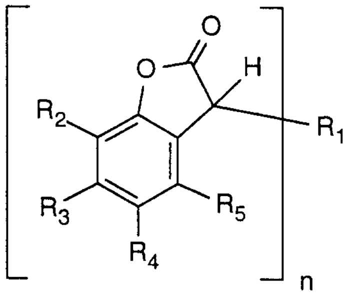

(I)

worin, wenn n 1 ist,

$R_1$ unsubstituiertes oder durch Chlor, Amino, Hydroxy, $C_1$-$C_{18}$-Alkyl, $C_1$-$C_{18}$-Alkoxy, $C_1$-$C_{18}$-Alkylthio, $C_3$-$C_4$-Alkenyloxy, $C_3$-$C_4$-Alkinyloxy, $C_1$-$C_4$-Alkylamino, Di-($C_1$-$C_4$-Alkyl)amino, Phenyl, Benzyl, Benzoyl oder Benzoyloxy substituiertes Phenyl, Naphthyl, Phenanthryl, Anthryl, 5,6,7,8-Tetrahydro-2-naphthyl, Thienyl, Benzo[b]thienyl, Naphtho [2,3-b]thienyl, Thiathrenyl, Furyl, Benzofuryl, Isobenzofuryl, Dibenzofuryl, Chromenyl, Xanthenyl, Phenoxathiinyl, Pyrrolyl, Imidazolyl, Pyrazolyl, Pyrazinyl, Pyrimidinyl, Pyridazinyl, Indolizinyl, Isoindolyl, Indolyl, Indazolyl, Purinyl, Chinolizinyl, Isochinolyl, Chinolyl, Phtalazinyl, Naphthyridinyl, Chinoxalinyl, Chinazolinyl,Cinnolinyl, Pteridinyl, Carbazolyl, β-Carbolinyl, Phenanthridinyl, Acridinyl, Perimidinyl, Phenanthrolinyl, Phenazinyl, Isothiazolyl, Phenothiazinyl, Isoxazolyl, Furazanyl, Biphenyl, Tetralinyl, Fluorenyl oder Phenoxazinyl darstellt, oder $R_1$ einen Rest der Formel V oder VI

(V) 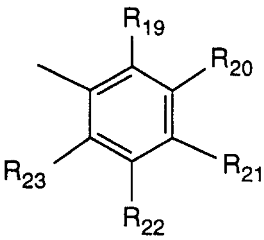

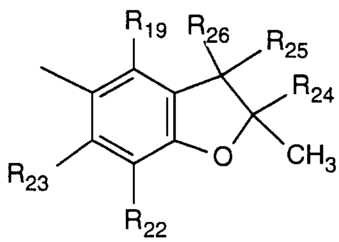 (VI)

bedeutet,
wenn n 2 ist,
$R_1$ unsubstituiertes oder mit $C_1$-$C_4$-Alkyl oder Hydroxy substituiertes Phenylen oder Naphthylen; oder $-R_6$-X-$R_7$- darstellt,
$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{18}$-Alkoxy, $C_1$-$C_{15}$-Alkylthio, $C_1$-$C_4$-Alkylamino, Di-($C_1$-$C_4$-alkyl)amino, $C_1$-$C_{25}$-Alkanoyloxy, $C_1$-$C_{25}$-Alkanoylamino, $C_3$-$C_{25}$-Alkenoyloxy, durch Sauerstoff, Schwefel oder $>N-R_8$ unterbrochenes $C_3$-$C_{25}$-Alkanoyloxy; $C_6$-$C_9$-Cycloalkylcarbonyloxy, Benzoyloxy oder durch $C_1$-$C_{12}$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich $-(CH_2)_p-COR_9$ oder $-(CH_2)_qOH$ darstellt, oder wenn $R_3$ und $R_5$ Wasserstoff sind, $R_4$ zusätzlich einen Rest der Formel II

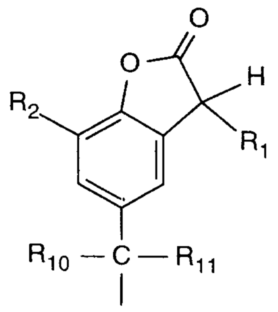

(II)

bedeutet, worin $R_1$ wie oben für n = 1 angegeben, definiert ist,

$R_6$ und $R_7$ unabhängig voneinander unsubstituiertes oder durch $C_1$-$C_4$-Alkyl substituiertes Phenylen oder Naphthylen darstellen,

$R_8$ Wasserstoff oder $C_1$-$C_5$-Alkyl ist,

$R_9$ Hydroxy, $[-O^{\theta}\ \frac{1}{r}\ M^{r+}]$, $C_1$-$C_{18}$-Alkoxy oder

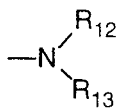

bedeutet,

$R_{10}$ und $R_{11}$ unabhängig voneinander Wasserstoff, $CF_3$, $C_1$-$C_{12}$-Alkyl oder Phenyl darstellen, oder $R_{10}$ und $R_{11}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;

$R_{12}$ und $R_{13}$ unabhängig voneinander Wasserstoff oder $C_1$-$C_{18}$-Alkyl darstellen,

$R_{14}$ Wasserstoff oder $C_1$-$C_{18}$-Alkyl bedeutet,

$R_{19}$, $R_{20}$, $R_{21}$, $R_{22}$ und $R_{23}$ unabhängig voneinander Wasserstoff, Halogen, Hydroxy, $C_1$-$C_{25}$-Alkyl, durch Sauerstoff, Schwefel oder $>N-R_8$ unterbrochenes $C_2$-$C_{25}$-Alkyl;

$C_1$-$C_{25}$-Alkoxy, durch Sauerstoff, Schwefel oder $>N-R_8$ unterbrochenes $C_2$-$C_{25}$-Alkoxy; $C_1$-$C_{25}$-Alkylthio, $C_3$-$C_{25}$-Alkenyl, $C_3$-$C_{25}$-Alkenyloxy, $C_3$-$C_{25}$-Alkinyl, $C_3$-$C_{25}$-Alkinyloxy, $C_7$-$C_9$-Phenylalkyl, $C_7$-$C_9$-Phenylalkoxy, unsubstituiertes oder durch $C_1$-$C_4$-Alkyl substituiertes Phenyl; unsubstituiertes oder durch $C_1$-$C_4$-Alkyl substituiertes Phenoxy; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes $C_5$-$C_8$-Cycloalkoxy; $C_1$-$C_4$-Alkylamino, Di($C_1$-$C_4$-alkyl)amino, $C_1$-$C_{25}$-Alkanoyl, durch Sauerstoff, Schwefel oder $>N-R_8$ unterbrochenes $C_3$-$C_{25}$-Alkanoyl; $C_1$-$C_{25}$-Alkanoyloxy, durch Sauerstoff, Schwefel oder $>N-R_8$ unterbrochenes $C_3$-$C_{25}$-Alkanoyloxy; $C_1$-$C_{25}$-Alkanoylamino, $C_3$-$C_{25}$-Alkenoyl, durch Sauerstoff, Schwefel oder $>N-R_8$ unterbrochenes $C_3$-$C_{25}$-Alkenoyl;

$C_3$-$C_{25}$-Alkenoyloxy, durch Sauerstoff, Schwefel oder $>N-R_8$ unterbrochenes $C_3$-$C_{25}$-Alkenoyloxy; $C_6$-$C_9$-Cycloalkylcarbonyl, $C_6$-$C_9$-Cycloalkylcarbonyloxy, Benzoyl oder durch $C_1$-$C_{12}$-Alkyl substituiertes Benzoyl; Benzoyloxy oder durch $C_1$-$C_{12}$-Alkyl substituiertes Benzoyloxy;

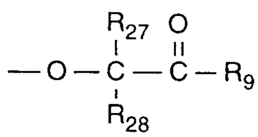 , 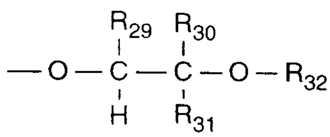

darstellen, oder ferner in Formel V die Reste $R_{19}$ und $R_{20}$ oder die Reste $R_{20}$ und $R_{21}$ zusammen mit den Kohlenstoffatomen, an die sie gebunden sind, einen Benzoring bilden,

$R_{24}$ Wasserstoff, $C_1$-$C_4$-Alkyl, unsubstituiertes oder durch $C_1$-$C_4$-Alkyl substituiertes Phenyl bedeutet,

$R_{25}$ und $R_{26}$ Wasserstoff, $C_1$-$C_4$-Alkyl oder Phenyl bedeuten, mit der Bedingung, dass mindestens einer der

Reste $R_{25}$ und $R_{26}$ Wasserstoff ist,

$R_{27}$ und $R_{28}$ unabhängig voneinander Wasserstoff, $C_1$-$C_4$-Alkyl oder Phenyl darstellen,

$R_{29}$ Wasserstoff oder $C_1$-$C_4$-Alkyl ist,

$R_{30}$ Wasserstoff, unsubstituiertes oder durch $C_1$-$C_4$-Alkyl substituiertes Phenyl; $C_1$-$C_{25}$-Alkyl, durch Sauerstoff, Schwefel oder $>N—R_8$ unterbrochenes $C_2$-$C_{25}$-Alkyl; unsubstituiertes oder am Phenylrest durch 1 bis 3 $C_1$-$C_4$-Alkyl substituiertes $C_7$-$C_9$-Phenylalkyl; durch Sauerstoff, Schwefel oder $>N—R_8$ unterbrochenes unsubstituiertes oder am Phenylrest durch 1 bis 3 $C_1$-$C_4$-Alkyl substituiertes $C_7$-$C_{25}$-Phenylalkyl bedeutet, oder ferner die Reste $R_{29}$ und $R_{30}$ zusammen mit den Kohlenstoffatomen, an die sie gebunden sind, einen unsubstituierten oder durch 1 bis 3 $C_1$-$C_4$-Alkyl substituierten $C_5$-$C_{12}$-Cycloalkylenring bilden;

$R_{31}$ Wasserstoff oder $C_1$-$C_4$-Alkyl darstellt,

$R_{32}$ Wasserstoff, $C_1$-$C_{25}$-Alkanoyl, $C_3$-$C_{25}$-Alkenoyl, durch Sauerstoff, Schwefel oder $>N—R_8$ unterbrochenes $C_3$-$C_{25}$-Alkanoyl; durch eine Di($C_1$-$C_6$-alkyl)phosphonatgruppe substituiertes $C_2$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl;

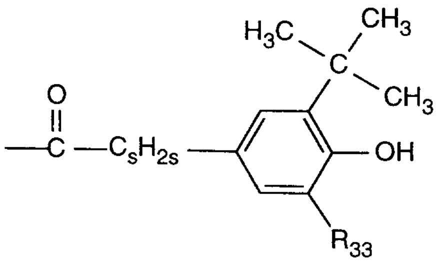 ,

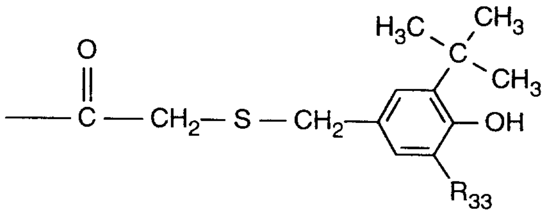 , 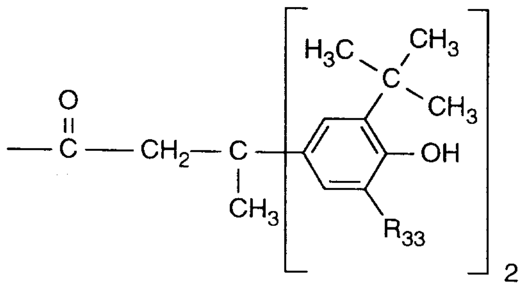 ,

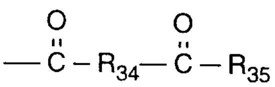 oder 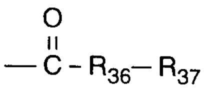

bedeutet,

$R_{33}$ Wasserstoff oder $C_1$-$C_8$-Alkyl darstellt,

$R_{34}$ eine direkte Bindung, $C_1$-$C_{18}$-Alkylen, durch Sauerstoff, Schwefel oder $>N—R_8$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

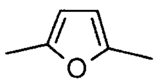 oder 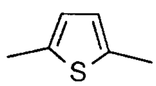

darstellt,

$R_{35}$ Hydroxy, $[-O^{\theta} \frac{1}{r} M^{r+}]$, $C_1$-$C_{18}$-Alkoxy oder

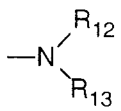

bedeutet,

$R_{36}$ Sauerstoff, -NH- oder

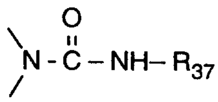

darstellt,

$R_{37}$ $C_1$-$C_{18}$-Alkyl oder Phenyl ist,

M ein r-wertiges Metallkation ist,

X eine direkte Bindung, Sauerstoff, Schwefel oder -$NR_{14}$- darstellt,

n 1 oder 2 ist,

p 0, 1 oder 2 bedeutet,

q 1, 2, 3, 4, 5 oder 6 darstellt,

r 1, 2 oder 3 ist, und

s 0, 1 oder 2 bedeutet,

dadurch gekennzeichnet, dass eine Verbindung der Formel III

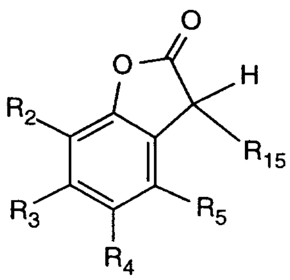

(III)

worin

$R_{15}$ Halogen oder -OR'$_{15}$ darstellt,

R'$_{15}$ Wasserstoff, $C_1$-$C_{25}$-Alkanoyl, $C_3$-$C_{25}$-Alkenoyl, durch Sauerstoff, Schwefel oder $>N—R_8$ unterbrochenes $C_3$-$C_{25}$-Alkanoyl; $C_6$-$C_9$-Cycloalkylcarbonyl, Thenoyl, Furoyl, Benzoyl oder durch $C_1$-$C_{12}$-Alkyl substituiertes Benzoyl; Naphthoyl oder durch $C_1$-$C_{12}$-Alkyl substituiertes Naphthoyl; $C_1$-$C_{25}$-Alkansulfonyl, durch Fluor substituiertes $C_1$-$C_{25}$-Alkansulfonyl; Phenylsulfonyl oder durch $C_1$-$C_{12}$-Alkyl substituiertes Phenylsulfonyl;

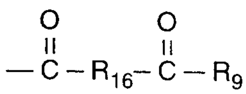 oder 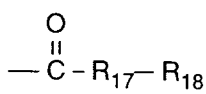

bedeutet,

$R_{16}$ eine direkte Bindung, $C_1$-$C_{18}$-Alkylen, durch Sauerstoff, Schwefel oder $>N—R_8$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

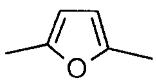 oder 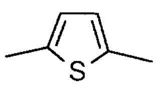

darstellt,

$R_{17}$ Sauerstoff, -NH- oder

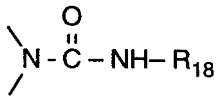

bedeutet, und

$R_{18}$ $C_1$-$C_{18}$-Alkyl oder Phenyl ist,

mit einer Verbindung der Formel IV

$$[H]_n\text{-}R_1 \qquad \text{(IV)}$$

umgesetzt wird.

**2.** Verfahren gemäss Anspruch 1, worin, wenn n 2 ist,

$R_1$ -$R_6$-X-$R_7$- darstellt,

$R_6$ und $R_7$ Phenylen bedeuten,

X Sauerstoff oder -$NR_{14}$- darstellt, und

$R_{14}$ $C_1$-$C_4$-Alkyl bedeutet.

**3.** Verfahren gemäss Anspruch 1, worin

$R_{19}$, $R_{20}$, $R_{21}$, $R_{22}$ und $R_{23}$ unabhängig voneinander Wasserstoff, Chlor, Brom, Hydroxy, $C_1$-$C_{18}$-Alkyl, durch Sauerstoff oder Schwefel unterbrochenes $C_2$-$C_{18}$-Alkyl; $C_1$-$C_{18}$-Al-koxy, durch Sauerstoff oder Schwefel unterbrochenes $C_2$-$C_{18}$-Alkoxy; $C_1$-$C_{18}$-Alkylthio, $C_3$-$C_{12}$-Alkenyloxy, $C_3$-$C_{12}$-Alkinyloxy, $C_7$-$C_9$-Phenylalkyl, $C_7$-$C_9$-Phenylalkoxy, unsubstituiertes oder durch $C_1$-$C_4$-Alkyl substituiertes Phenyl; Phenoxy, Cyclohexyl, $C_5$-$C_8$-Cycloalkoxy; $C_1$-$C_4$-Alkylamino, Di-($C_1$-$C_4$-alkyl)amino, $C_1$-$C_{12}$-Alkanoyl, durch Sauerstoff oder Schwefel unterbrochenes $C_3$-$C_{12}$-Alkanoyl; $C_1$-$C_{12}$-Alkanoyloxy, durch Sauerstoff oder Schwefel unterbrochenes $C_3$-$C_{12}$-Alkanoyloxy; $C_1$-$C_{12}$-Alkanoylamino, $C_3$-$C_{12}$-Alkenoyl, $C_3$-$C_{12}$-Alkenoyloxy, Cyclohexylcarbonyl, Cyclohexylcarbonyloxy, Benzoyl oder durch $C_1$-$C_4$-Alkyl substituiertes Benzoyl; Benzoyloxy oder durch $C_1$-$C_4$-Alkyl substituiertes Benzoyloxy;

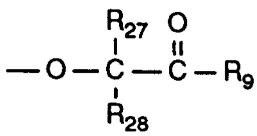 , 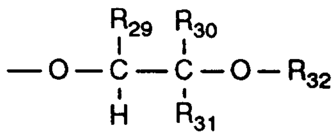

darstellen, oder ferner in Formel V die Reste $R_{19}$ und $R_{20}$ oder die Reste $R_{20}$ und $R_{21}$ zusammen mit den Kohlenstoffatomen, an die sie gebunden sind, einen Benzoring bilden,

$R_{24}$ Wasserstoff oder $C_1$-$C_4$-Alkyl bedeutet,

$R_{25}$ und $R_{26}$ Wasserstoff oder $C_1$-$C_4$-Alkyl bedeuten, mit der Bedingung, dass mindestens einer der Reste $R_{25}$ und $R_{26}$ Wasserstoff ist,

$R_{27}$ und $R_{28}$ unabhängig voneinander Wasserstoff oder $C_1$-$C_4$-Alkyl darstellen,

$R_{29}$ Wasserstoff ist,

$R_{30}$ Wasserstoff, Phenyl, $C_1$-$C_{18}$-Alkyl, durch Sauerstoff oder Schwefel unterbrochenes $C_2$-$C_{18}$-Alkyl; $C_7$-$C_9$-Phenylalkyl, durch Sauerstoff oder Schwefel unterbrochenes unsubstituiertes oder am Phenylrest durch 1 bis 3 $C_1$-$C_4$-Alkyl substituiertes $C_7$-$C_{18}$-Phenylalkyl bedeutet, oder ferner die Reste $R_{29}$ und $R_{30}$ zusammen mit den Kohlenstoffatomen, an die sie gebunden sind, einen unsubstituierten oder durch 1 bis 3 $C_1$-$C_4$-Alkyl sub-

stituierten Cyclohexylenring bilden;

$R_{31}$ Wasserstoff oder $C_1$-$C_4$-Alkyl darstellt,
$R_{32}$ Wasserstoff, $C_1$-$C_{18}$-Alkanoyl, $C_3$-$C_{12}$-Alkenoyl, durch Sauerstoff oder Schwefel unterbrochenes $C_3$-$C_{12}$-Alkanoyl; durch eine Di($C_1$-$C_6$-alkyl)phosphonatgruppe substituiertes $C_2$-$C_{12}$-Alkanoyl; $C_6$-$C_9$-Cycloalkylcarbonyl, Benzoyl,

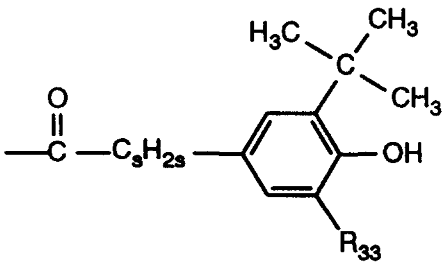 , 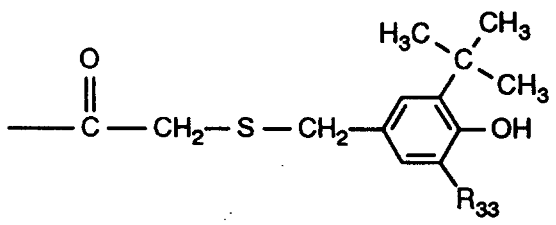 ,

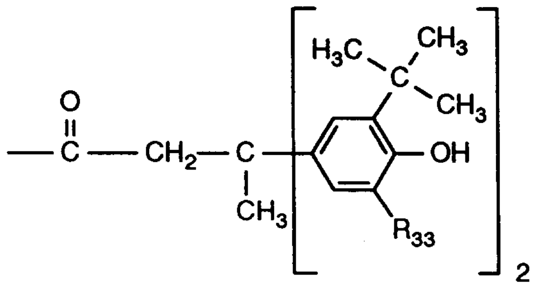 , 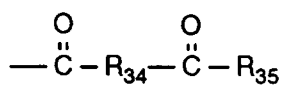 oder 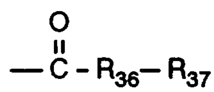

bedeutet,
$R_{33}$ Wasserstoff oder $C_1$-$C_4$-Alkyl darstellt,
$R_{34}$ $C_1$-$C_{12}$-Alkylen, $C_2$-$C_8$-Alkenylen, $C_2$-$C_8$-Alkyliden, $C_7$-$C_{12}$-Phenylalkyliden, $C_5$-$C_8$-Cycloalkylen oder Phenylen darstellt,
$R_{35}$ Hydroxy, [$-O^{\theta} \frac{1}{r} M^{r+}$] oder $C_1$-$C_{18}$-Alkoxy bedeutet,
$R_{36}$ Sauerstoff oder -NH- darstellt,
$R_{37}$ $C_1$-$C_8$-Alkyl oder Phenyl ist und
s 1 oder 2 bedeutet.

**4.** Verfahren gemäss Anspruch 1 worin

$R_1$ Phenanthryl, Thienyl, Dibenzofuryl, unsubstituiertes oder mit $C_1$-$C_4$-Alkyl substituiertes Carbazolyl; oder Fluorenyl darstellt, oder $R_1$ einen Rest der Formel V

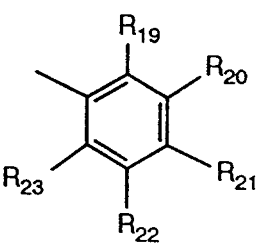 (V)

bedeutet, worin
$R_{19}$, $R_{20}$, $R_{21}$, $R_{22}$ und $R_{23}$ unabhängig voneinander Wasserstoff, Chlor, Hydroxy, $C_1$-$C_{18}$-Alkyl, $C_1$-$C_{18}$-Alkoxy, $C_1$-$C_{18}$-Alkylthio, $C_3$-$C_4$-Alkenyloxy, $C_3$-$C_4$-Alkinyloxy, Phenyl, Benzoyl, Benzoyloxy oder

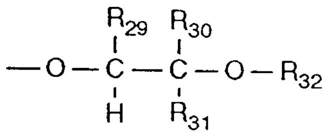

darstellen,

$R_{29}$ Wasserstoff ist,
$R_{30}$ Wasserstoff, Phenyl oder $C_1$-$C_{18}$-Alkyl bedeutet, oder ferner die Reste $R_{29}$ und $R_{30}$ zusammen mit den Kohlenstoffatomen, an die sie gebunden sind, einen unsubstituierten oder durch 1 bis 3 $C_1$-$C_4$-Alkyl substituierten Cyclohexylenring bilden;
$R_{31}$ Wasserstoff oder $C_1$-$C_4$-Alkyl darstellt, und
$R_{32}$ Wasserstoff, $C_1$-$C_{12}$-Alkanoyl oder Benzoyl bedeutet.

**5.** Verfahren gemäss Anspruch 4, worin

$R_{19}$ Wasserstoff oder $C_1$-$C_4$-Alkyl darstellt,
$R_{20}$ Wasserstoff oder $C_1$-$C_4$-Alkyl bedeutet,
$R_{21}$ Wasserstoff, Chlor, Hydroxy, $C_1$-$C_{12}$-Alkyl, $C_1$-$C_4$-Alkoxy, $C_1$-$C_4$-Alkylthio, Phenyl oder $-O-CH_2-CH_2-O-R_{32}$ darstellt,
$R_{22}$ Wasserstoff oder $C_1$-$C_4$-Alkyl ist,
$R_{23}$ Wasserstoff oder $C_1$-$C_4$-Alkyl bedeutet, und
$R_{32}$ $C_1$-$C_4$-Alkanoyl darstellt.

**6.** Verfahren gemäss Anspruch 1, worin

$R_2$, $R_3$, $R_4$ und $R_5$ unabhängig voneinander Wasserstoff, Chlor, Hydroxy, $C_1$-$C_{25}$-Alkyl, $C_7$-$C_9$-Phenylalkyl, unsubstituiertes oder durch $C_1$-$C_4$-Alkyl substituiertes Phenyl, unsubstituiertes oder durch $C_1$-$C_4$-Alkyl substituiertes $C_5$-$C_8$-Cycloalkyl; $C_1$-$C_{12}$-Alkoxy, $C_1$-$C_{12}$-Alkylthio, $C_1$-$C_4$-Alkylamino, Di-($C_1$-$C_4$-alkyl)amino, $C_1$-$C_{18}$-Alkanoyloxy, $C_1$-$C_{18}$-Alkanoylamino, $C_3$-$C_{18}$-Alkenoyloxy, durch Sauerstoff, Schwefel oder $>N-R_8$ unterbrochenes $C_3$-$C_{18}$-Alkanoyloxy; $C_6$-$C_9$-Cycloalkylcarbonyloxy, Benzoyloxy oder durch $C_1$-$C_8$-Alkyl substituiertes Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich -$(CH_2)_p$-$COR_9$, oder -$(CH_2)_q$OH darstellt, oder wenn $R_3$ und $R_5$ Wasserstoff sind, $R_4$ zusätzlich einen Rest der Formel II

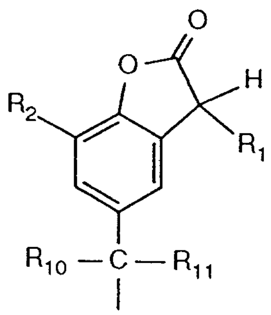
(II)

bedeutet,
$R_8$ Wasserstoff oder $C_1$-$C_6$-Alkyl ist,
$R_9$ Hydroxy, $C_1$-$C_{18}$-Alkoxy oder

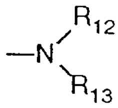

bedeutet,

$R_{10}$ und $R_{11}$ Methylgruppen sind oder zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden;
$R_{12}$ und $R_{13}$ unabhärgig voneinander Wasserstoff oder $C_1$-$C_8$-Alkyl darstellen, und q 2, 3, 4, 5 oder 6 bedeutet.

**7.** Verfahren gemäss Anspruch 1, worin mindestens zwei der Reste $R_2$, $R_3$, $R_4$ und $R_5$ Wasserstoff sind.

**8.** Verfahren gemäss Anspruch 1, worin $R_3$ und $R_5$ Wasserstoff sind.

**9.** Verfahren gemäss Anspruch 1, worin

$R_2$, $R_3$, $R_4$ und $R_5$ unabhängig voneinander Wasserstoff, Chlor, Hydroxy, $C_1$-$C_{18}$-Alkyl, $C_7$-$C_9$-Phenylalkyl, Phenyl, $C_5$-$C_8$-Cycloalkyl, $C_1$-$C_6$-Alkoxy, Cyclohexylcarbonyloxy oder Benzoyloxy darstellen, oder ferner die Reste $R_2$ und $R_3$ oder die Reste $R_3$ und $R_4$ oder die Reste $R_4$ und $R_5$ zusammen mit den Kohlenstoffatomen, an die sie gebunden sind, einen Benzoring bilden, $R_4$ zusätzlich $-(CH_2)_p-COR_9$ darstellt, oder wenn $R_3$ und $R_5$ Wasserstoff sind, $R_4$ zusätzlich einen Rest der Formel II bedeutet,
$R_9$ Hydroxy oder $C_1$-$C_{18}$-Alkoxy bedeutet, und
$R_{10}$ und $R_{11}$ Methylgruppen sind oder zusammen mit dem C-Atom, an das sie gebunden sind, einen $C_5$-$C_8$-Cycloalkylidenring bilden.

**10.** Verfahren gemäss Anspruch 1, worin

$R_2$ $C_1$-$C_{18}$-Alkyl oder Cyclohexyl bedeutet,
$R_3$ Wasserstoff ist,
$R_4$ $C_1$-$C_4$-Alkyl, Cyclohexyl oder einen Rest der Formel II darstellt,
$R_5$ Wasserstoff ist, und
$R_{10}$ und $R_{11}$ zusammen mit dem C-Atom, an das sie gebunden sind, einen Cyclohexylidenring bilden.

**11.** Verfahren gemäss Anspruch 1, worin

$R'_{15}$ Wasserstoff, $C_1$-$C_{18}$-Alkanoyl, $C_3$-$C_{18}$-Alkenoyl, durch Sauerstoff, Schwefel oder $>N-R_8$ unterbrochenes $C_3$-$C_{18}$-Alkanoyl; $C_6$-$C_9$-Cycloalkylcarbonyl, Thenoyl, Furoyl, Benzoyl oder durch $C_1$-$C_8$-Alkyl substituiertes Benzoyl; Naphthoyl oder durch $C_1$-$C_8$-Alkyl substituiertes Naphthoyl; $C_1$-$C_{18}$-Alkansulfonyl, durch Fluor substituiertes $C_1$-$C_{18}$-Alkansulfonyl; Phenylsulfonyl oder durch $C_1$-$C_8$-Alkyl substituiertes Phenylsulfonyl;

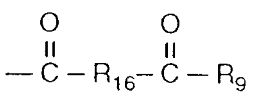 oder 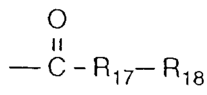

bedeutet,

$R_{16}$ eine direkte Bindung $C_1$-$C_{12}$-Alkylen, durch Sauerstoff, Schwefel oder $>N-R_8$ unterbrochenes $C_2$-$C_{12}$-Alkylen; $C_2$-$C_{12}$-Alkenylen, $C_2$-$C_{12}$-Alkyliden, $C_7$-$C_{12}$-Phenylalkyliden, $C_5$-$C_8$-Cycloalkylen, $C_7$-$C_8$-Bicycloalkylen oder Phenylen darstellt,
$R_{17}$ Sauerstoff oder -NH- bedeutet, und
$R_{18}$ $C_1$-$C_{12}$-Alkyl oder Phenyl ist.

**12.** Verfahren gemäss Anspruch 1, worin

$R_{15}$ Chlor, Brom oder $-OR'_{15}$ darstellt,
$R'_{15}$ Wasserstoff, $C_1$-$C_{12}$-Alkanoyl, durch Sauerstoff unterbrochenes $C_3$-$C_{12}$-Alkanoyl; Cyclohexylcarbonyl, Benzoyl, Naphthoyl, $C_1$-$C_{12}$-Alkansulfonyl, durch Fluor substituiertes $C_1$-$C_{12}$-Alkansulfonyl; Phenylsulfonyl

oder durch $C_1$-$C_4$-Alkyl substituiertes Phenylsulfonyl; oder

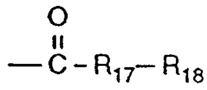

bedeutet,
$R_{17}$ -NH- darstellt, und
$R_{18}$ $C_1$-$C_8$-Alkyl oder Phenyl ist.

**13.** Verfahren gemäss Anspruch 1, worin

$R_{15}$ -OR'$_{15}$ darstellt,
R'$_{15}$ Wasserstoff, $C_1$-$C_4$-Alkanoyl oder

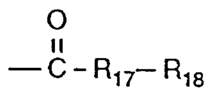

bedeutet,
$R_{17}$ -NH- darstellt, und
$R_{18}$ $C_1$-$C_4$-Alkyl ist.

**14.** Verfahren gemäss Anspruch 1, wohn die Umsetzung in Gegenwart eines Katalysators durchgeführt wird.

**15.** Verfahren gemäss Anspruch 14, worin der Katalysator eine Protonensäure, eine Lewis-Säure, ein Aluminiumsilikat, ein Ionenaustauscherharz, ein Zeolith, ein natürlich vorkommendes Schichrsilikat oder ein modifiziertes Schichtsilikat bedeutet.

**16.** Verfahren gemäss Anspruch 14, worin der Katalysator ein natürlich vorkommendes Schichtsilikat oder ein modifiziertes Schichtsilikat bedeutet.

**17.** Verfahren gemäss Anspruch 16, worin das natürlich vorkommende Schichtsilikat ein Fulcat®- oder Fulmont®-Typ ist.

**18.** Verfahren gemäss Anspruch 1, worin, wenn n 1 ist, das molare Mengenverhältnis der Verbindung der Formel III zur Verbindung der Formel IV 1:1 bis 1:20 beträgt, und wenn n 2 ist, das molare Mengenverhältnis der Verbindung der Formel III zur Verbindung der Formel IV 3:1 bis 2:1 beträgt.

**19.** Verfahren zur Herstellung von Verbindungen der Formel I

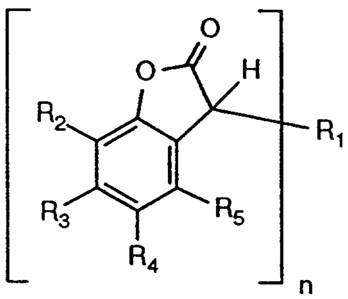

(I)

worin die allgemeinen Symbole wie in Anspruch 1 definiert sind, dadurch gekennzeichnet, dass ein Aequivalent Phenol der Formel VII

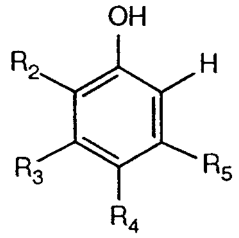

(VII)

worin die allgemeinen Symbole wie in Anspruch 1 definiert sind, mit 0,8 bis 2,0 Aequivalent Glyoxylsäure zu einer Verbindung der Formel VIII

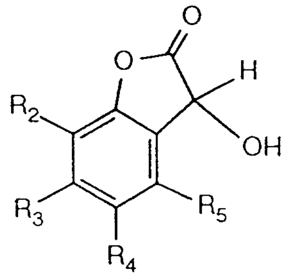

(VIII)

worin die allgemeinen Symbole wie in Anspruch 1 definiert sind, umgesetzt wird, und diese anschliessend ohne Isolierung mit einer Verbindung der Formel IV worin $R_1$ und n wie in Anspruch 1 definiert sind, zur Reaktion gebracht wird.

**20.** Verfahren gemäss Anspruch 19, worin die Verbindung der Formel VIII ohne Isolierung vor der weiteren Umsetzung mit einer Verbindung der Formel IV in einem zusätzlichen Reaktionsschritt mit einer Halogenwasserstoffsäure, einem Halogenid einer Schwefel-Sauerstoffsäure, einem Halogenid der Phosphorsäure, einem Halogenid der phosphorigen Säure, einer Säure der Formel IX,

$$R'_{15}\text{-OH} \qquad \text{(IX)}$$

einem Säurehalogenid der Formel X,

$$R'_{15}\text{-Y} \qquad \text{(X)}$$

einem Ester der Formel XI

$$R'_{15}\text{-O-}R_{38} \qquad \text{(XI)}$$

einem symmetrischen oder unsymmetrischen Anhydrid der Formel XII

$$R'_{15}\text{-O-}R'_{15} \qquad \text{(XII)}$$

oder einem Isocyanat der Formel XIII

$$R_{39}\text{-N=C=O} \qquad \text{(XIII)}$$

worin $R'_{15}$ in den Verbindungen der Formeln IX, X, XI und XII wie in Anspruch 1 definiert ist, mit der Bedingung, dass $R'_{15}$ nicht Wasserstoff bedeutet;

$R_{38}$ $C_1$-$C_8$-Alkyl darstellt,

$R_{39}$ $C_1$-$C_{18}$-Alkyl oder Phenyl bedeutet, und
Y Fluor, Chlor, Brom oder Iod darstellt, zu einer Verbindung der Formel III

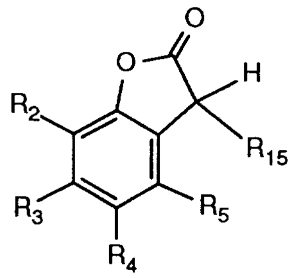
(III)

umgesetzt wird, worin die Reste $R_2$, $R_3$, $R_4$, $R_5$ und $R_{15}$ wie in Anspruch 1 definiert sind, wobei im Fall $R_{15}$ = -OR'$_{15}$, R'$_{15}$ nicht Wasserstoff ist.

**Claims**

**1.** A process for the preparation of a compound of formula I

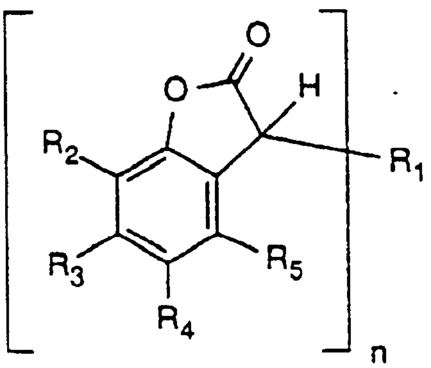
(I)

wherein, when n is 1,

$R_1$ is unsubstituted or chloro-, amino-, hydroxy-, $C_1$-$C_{18}$alkyl-, $C_1$-$C_{18}$alkoxy-, $C_1$-$C_{18}$alkylthio-, $C_3$-$C_4$-alkenyloxy-, $C_3$-$C_4$alkynyloxy-, $C_1$-$C_4$alkylamino-, di($C_1$-$C_4$alkyl)amino-, phenyl-, benzyl-, benzoyl- or benzoyloxysubstituted phenyl, naphthyl, phenanthryl, anthryl, 5,6,7,8-tetrahydro-2-naphthyl, thienyl, benzo[b]thienyl, naphtho[2,3-b]thienyl, thianthrenyl, furyl, benzofuryl, isobenzofuryl, dibenzofuryl, chromenyl, xanthenyl, phenoxathiinyl, pyrrolyl, imidazolyl, pyrazolyl, pyrazinyl, pyrimidinyl, pyridazinyl, indolizinyl, isoindolyl, indolyl, indazolyl, purinyl, quinozinyl, isoquinolyl, quinolyl, phthalazinyl, naphthyridinyl, quinoxalinyl, quinazolinyl, cinnolinyl, pteridinyl, carbazolyl, β-carbolinyl, phenanthridinyl, acridinyl, perimidinyl, phenanthrolinyl, phenazinyl, isothiazolyl, phenothiazinyl, isoxazolyl, furazanyl, biphenyl, tetralinyl, fluorenyl or phenoxazinyl, or $R_1$ is a radical of formula V or VI

(V) 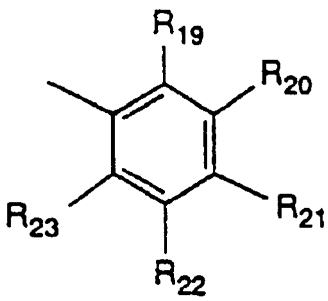 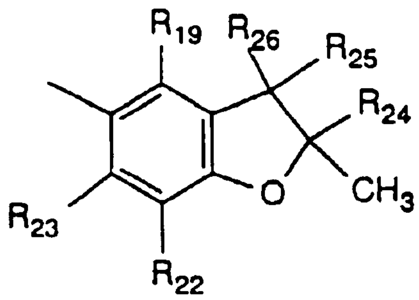 (VI)

when n is 2,

$R_1$ is unsubstituted or $C_1$-$C_4$alkyl- or hydroxysubstituted phenylene or naphthylene; or is -$R_6$-X-$R_7$-,

$R_2$, $R_3$, $R_4$ and $R_5$ are each independently of one another hydrogen, chloro, hydroxy, $C_1$-$C_{25}$alkyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$cycloalkyl; $C_1$-$C_{18}$alkoxy, $C_1$-$C_{18}$alkylthio, $C_1$-$C_4$alkylamino, di($C_1$-$C_4$alkyl)amino, $C_1$-$C_{25}$alkanoyloxy, $C_1$-$C_{25}$alkanoylamino, $C_3$-$C_{25}$alkenoyloxy, $C_3$-$C_{25}$alkanoyloxy which is interrupted by oxygen, sulfur, or $>N-R_8$; $C_6$-$C_9$cycloalkylcarbonyloxy, benzoyloxy or $C_1$-$C_{12}$alkyl-substituted benzoyloxy; or else the radicals $R_2$ and $R_3$ or $R_3$ and $R_4$ or $R_4$ and $R_5$, together with the linking carbon atoms, form a benzo ring; $R_4$ is additionally -$(CH_2)_p$-$COR_9$ or -$(CH_2)_q$OH, or, if $R_3$ and $R_5$ are hydrogen, $R_4$ is additionally a radical of formula II

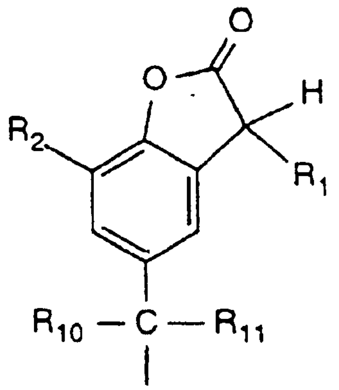 (II)

wherein $R_1$ is as defined above for n = 1,

$R_6$ and $R_7$ are each independently of the other unsubstituted or $C_1$-$C_4$alkyl-substituted phenylene or naphthylene,

$R_8$ is hydrogen or $C_1$-$C_8$alkyl,

$R_9$ is hydroxy, $[-O^{\theta} \frac{1}{r} M^{r+}]$, $C_1$-$C_{18}$alkoxy or

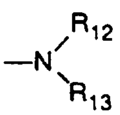 ,

$R_{10}$ and $R_{11}$ are each independantly of the other hydrogen, $CF_3$, $C_1$-$C_{12}$alkyl or phenyl, or $R_{10}$ and $R_{11}$, together with the linking carbon atom, form a $C_5$-$C_8$cycloalkylidene ring which is unsubstituted or substituted by 1 to 3 $C_1$-$C_4$alkyl groups,

$R_{12}$ and $R_{13}$ are each independently of the other hydrogen or $C_1$-$C_{18}$alkyl,

$R_{14}$ is hydrogen or $C_1$-$C_{18}$alkyl,

$R_{19}$, $R_{20}$, $R_{21}$, $R_{22}$ and $R_{23}$ are each independently of one another hydrogen, halogen, hydroxy, $C_1$-$C_{25}$alkyl, $C_2$-$C_{25}$alkyl which is interrupted by oxygen, sulfur or $>N-R_8$;

$C_1$-$C_{25}$alkoxy, $C_2$-$C_{25}$alkoxy which is interrupted by oxygen, sulfur or $>N-R_8$ ; $C_1$-$C_{25}$alkylthio, $C_3$-$C_{25}$alkenyl, $C_3$-$C_{25}$alkenyloxy, $C_3$-$C_{25}$alkynyl, $C_3$-$C_{25}$alkynyloxy, $C_7$-$C_9$-phenylalkyl, $C_7$-$C_9$phenylalkoxy, unsubstituted or $C_1$-$C_4$-alkyl-substituted phenyl; unsubstituted or $C_1$-$C_4$alkyl-substituted phenoxy; unsubstituted or $C_1$-$C_4$alkyl-substituted $C_5$-$C_8$cycloalkyl; unsubstituted or $C_1$-$C_4$alkyl-substituted $C_5$-$C_8$cycloalkoxy; $C_1$-$C_4$alkylamino, di($C_1$-$C_4$-alkyl)amino, $C_1$-$C_{25}$alkanoyl, $C_3$-$C_{25}$alkanoyl which is interrupted by oxygen, sulfur or $>N-R_8$; $C_1$-$C_{25}$-alkanoyloxy, $C_3$-$C_{25}$alkanoyioxy which is interrupted by oxygen, sulfur or $>N-R_8$; $C_1$-$C_{25}$alkanoylamino, $C_3$-$C_{25}$-alkenoyl, $C_3$-$C_{25}$alkenoyl which is interrupted by oxygen, sulfur, or $>N-R_8$; $C_3$-$C_{25}$alkenoyloxy, $C_3$-$C_{25}$alkenoyloxy which is interrupted by oxygen, sulfur or $>N-R_8$; $C_6$-$C_9$-cycloalkylcarbonyl, $C_6$-$C_9$cycloalkylcarbonyloxy, benzoyl or $C_1$-$C_{12}$alkyl-substituted benzoyl; benzoyloxy or $C_1$-$C_{12}$alkyl -substituted benzoyloxy;

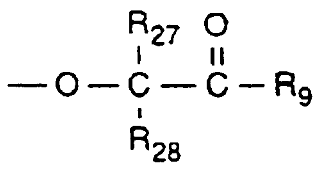 , 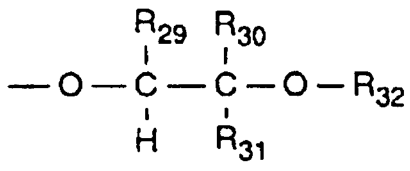 ,

or in formula V the radicals $R_{19}$ and $R_{20}$ or $R_{20}$ and $R_{21}$, together with the linking carbon atoms, form a benzo ring,

$R_{24}$ is hydrogen, $C_1$-$C_4$alkyl, unsubstituted or $C_1$-$C_4$alkyl-substituted phenyl,

$R_{25}$ and $R_{26}$ are hydrogen, $C_1$-$C_4$alkyl or phenyl, with the proviso that at least one of $R_{25}$ and $R_{26}$ is hydrogen,

$R_{27}$ and $R_{28}$ are each independently of the other hydrogen, $C_1$-$C_4$alkyl or phenyl,

$R_{29}$ is hydrogen or $C_1$-$C_4$alkyl,

$R_{30}$ is hydrogen, unsubstituted or $C_1$-$C_4$alkyl-substituted phenyl; $C_1$-$C_{25}$alkyl, $C_2$-$C_{25}$alkyl which is interrupted by oxygen, sulfur or $>N-R_8$; $C_7$-$C_9$phenylalkyl which is unsubstituted or substituted on the phenyl moiety by 1 to 3 $C_1$-$C_4$alkyl groups; $C_7$-$C_{25}$phenylalkyl which is interrupted by oxygen, sulfur or $>N-R_8$ and is unsubstituted or substituted on the phenyl moiety by 1 to 3 $C_1$-$C_4$alkyl groups; or the radicals $R_{29}$ and $R_{30}$, together with the linking carbon atoms, form a $C_5$-$C_{12}$cycloalkylene ring which is unsubstituted or substituted by 1 to 3 $C_1$-$C_4$alkyl groups,

$R_{31}$ is hydrogen or $C_1$-$C_4$alkyl,

$R_{32}$ is hydrogen, $C_1$-$C_{25}$alkanoyl, $C_3$-$C_{25}$alkenoyl, $C_3$-$C_{25}$-alkanoyl which is interrupted by oxygen, sulfur or $>N-R_8$; $C_2$-$C_{25}$alkanoyl which is substituted by a di($C_1$-$C_6$-alkyl)phosphonate group; $C_6$-$C_9$cycloalkylcarbonyl, thenoyl, furoyl, benzoyl or $C_1$-$C_{12}$alkyl-substituted benzoyl;

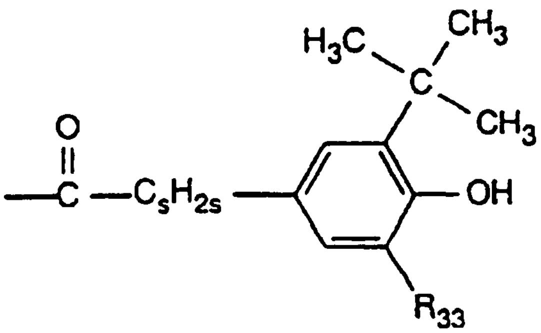 ,

,

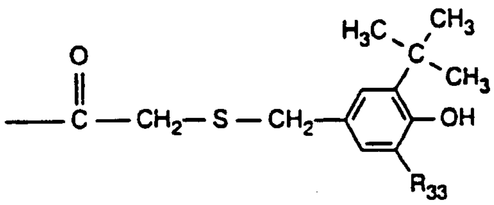 , 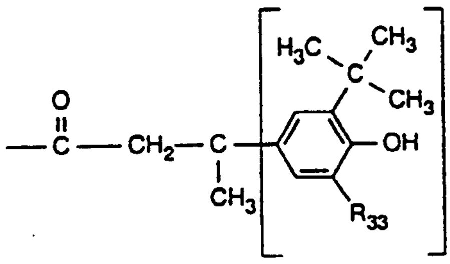 ,

,

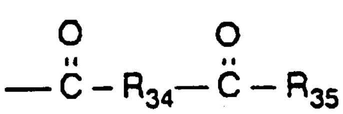 or 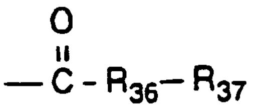 ,

$R_{33}$ is hydrogen or $C_1$-$C_8$alkyl,

$R_{34}$ is a direct bond, $c_1$-$C_{18}$alkylene, $C_2$-$C_{18}$alkylene which is interrupted by oxygen, sulfur or $>N—R_8$; $C_2$-$C_{18}$-alkenylene, $C_2$-$C_{20}$alkylidene, $C_7$-$C_{20}$phenylalkylidene, $C_5$-$C_8$cycloalkylene, $C_7$-$C_8$bicycloalkylene, unsubstituted or $C_1$-$C_4$alkyl-substituted phenylene,

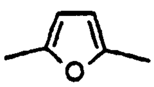 or 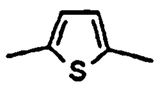 ,

$R_{35}$ is hydroxy, $[-O^{\theta} \frac{1}{r} M^{r+}]$, $C_1$-$C_{18}$alkoxy or

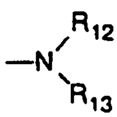 ,

$R_{36}$ is oxygen, -NH- or

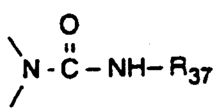 ,

$R_{37}$ is $C_1$-$C_{18}$alkyl or phenyl,

M is a metal cation of valency r,

X is a direct bond, oxygen, sulfur or $-NR_{14}-$,
n is 1 or 2,
p is 0, 1 or 2,
q is 1, 2, 3, 4, 5 or 6,
r is 1, 2 or 3,
s is 0, 1 or 2,
which process comprises reacting a compound of formula III

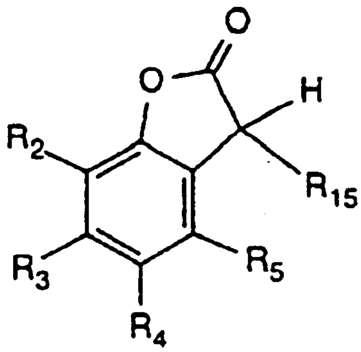

(III)

wherein
$R_{15}$ is halogen or $-OR'_{15}$,
$R'_{15}$ is hydrogen, $C_1$-$C_{25}$alkanoyl, $C_3$-$C_{25}$alkenoyl, $C_3$-$C_{25}$ alkanoyl which is interrupted by oxygen, sulfur or $>N-R_8$; $C_6$-$C_9$cycloalkylcarbonyl, thenoyl, furoyl, benzoyl or $C_1$-$C_{12}$alkyl-substituted benzoyl; naphthoyl or $C_1$-$C_{12}$alkyl-substituted naphthoyl; $C_1$-$C_{25}$alkanesulfonyl, fluoro-substituted $C_1$-$C_{25}$alkanesulfonyl; phenylsulfonyl or $C_1$-$C_{12}$alkyl-substituted phenylsulfonyl;

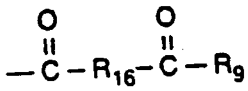 or 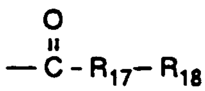,

$R_{16}$ is a direct bond, $C_1$-$C_{18}$alkylene, $C_2$-$C_{18}$alkylene which is interrupted by oxygen, sulfur or $>N-R_8$; $C_2$-$C_{18}$alkenylene, $C_2$-$C_{20}$alkylidene, $C_7$-$C_{20}$phenylalkylidene, $C_5$-$C_8$cycloalkylene, $C_7$-$C_8$bicycloalkylene, unsubstituted or $C_1$-$C_4$alkyl-substituted phenylene,

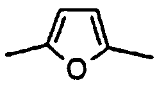 or 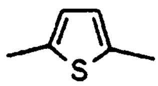,

$R_{17}$ is oxygen, -NH- or

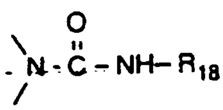,

and
$R_{18}$ is $C_1$-$C_{18}$alkyl or phenyl,
with a compound of formula IV

$$[H]_n\text{-}R_1 \qquad (IV).$$

**2.** A process according to claim 1, wherein, when n is 2,

$R_1$ is $-R_6\text{-}X\text{-}R_7-$,
$R_6$ and $R_7$ are phenylene,
X is oxygen or $-NR_{14}-$, and
$R_{14}$ is $C_1$-$C_4$alkyl.

**3.** A process according to claim 1, wherein

$R_{19}$, $R_{20}$, $R_{21}$, $R_{22}$ and $R_{23}$ are each independently of one another hydrogen, chloro, bromo, hydroxy, $C_1$-$C_{18}$alkyl, $C_2$-$C_{18}$alkyl which is interrupted by oxygen or sulfur; $C_1$-$C_{18}$alkoxy, $C_2$-$C_{18}$alkoxy which is interrupted by oxygen or sulfur; $C_1$-$C_{18}$alkylthio, $C_3$-$C_{12}$alkenyloxy, $C_3$-$C_{12}$-alkynyloxy, $C_7$-$C_9$phenylalkyl, $C_7$-$C_9$phenylalkoxy, unsubstituted or $C_1$-$C_4$alkyl-substituted phenyl; phenoxy, cyclohexyl, $C_5$-$C_8$cycloalkoxy; $C_1$-$C_4$alkylamino, di($C_1$-$C_4$alkyl) amino, $C_1$-$C_{12}$alkanoyl, $C_3$-$C_{12}$alkanoyl which is interrupted by oxygen or sulfur; $C_1$-$C_{12}$alkanoyloxy, $C_3$-$C_{12}$alkanoyloxy which is interrupted by oxygen or sulfur; $C_1$-$C_{12}$alkanoylamino, $C_3$-$C_{12}$alkenoyl, $C_3$-$C_{12}$-alkenoyloxy, cyclohexylcarbonyl, cyclohexylcarbonyloxy, benzoyl or $C_1$-$C_4$alkyl-substituted benzoyl; benzoyloxy or $C_1$-$C_4$alkyl-substituted benzoyloxy;

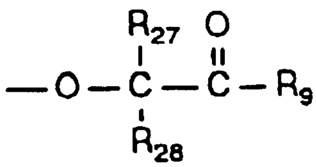 , 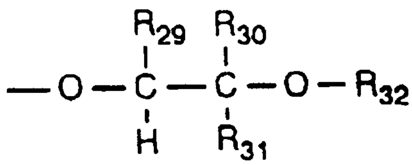 ,

or in formula V the radicals $R_{19}$ and $R_{20}$ or $R_{20}$ and $R_{21}$, together with the linking carbon atoms, form a benzo ring,
$R_{24}$ is hydrogen or $C_1$-$C_4$alkyl,
$R_{25}$ and $R_{26}$ are hydrogen or $C_1$-$C_4$alkyl, with the proviso that at least one of $R_{25}$ and $R_{26}$ is hydrogen,
$R_{27}$ and $R_{28}$ are each independently of the other hydrogen or $C_1$-$C_4$alkyl,
$R_{29}$ is hydrogen,
$R_{30}$ is hydrogen, phenyl, $C_1$-$C_{18}$alkyl, $C_2$-$C_{18}$alkyl which is interrupted by oxygen or sulfur; $C_7$-$C_9$phenylalkyl, $C_7$-$C_{18}$phenylalkyl which is interrupted by oxygen or sulfur and unsubstituted or substituted on the phenyl moiety by 1 to 3 $C_1$-$C_4$alkyl groups, or the radicals $R_{29}$ and $R_{30}$, together with the linking carbon atoms, form a cyclohexylene ring which is unsubstituted or substituted by 1 to 3 $C_1$-$C_4$alkyl groups,
$R_{31}$ is hydrogen or $C_1$-$C_4$alkyl,
$R_{32}$ is hydrogen, $C_1$-$C_{18}$alkanoyl, $C_3$-$C_{12}$alkenoyl, $C_3$-$C_{12}$ alkanoyl which is interrupted by oxygen or sulfur; $C_2$-$C_{12}$alkanoyl which is substituted by a di($C_1$-$C_6$-alkyl)phosphonate group; $C_6$-$C_9$cycloalkylcarbonyl, benzyl,

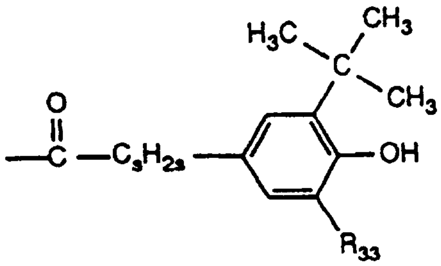 , 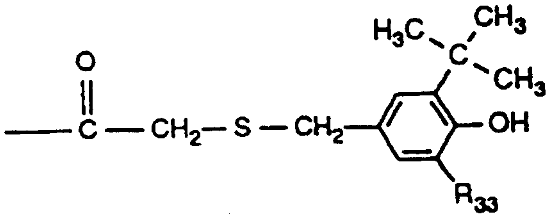 ,

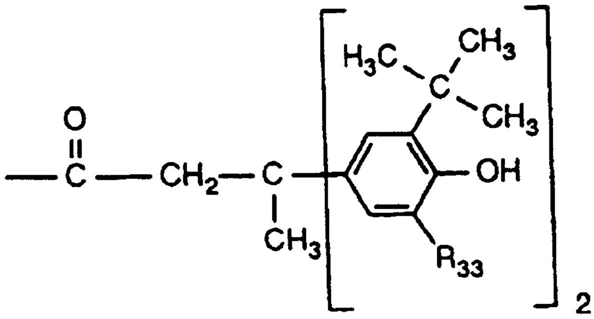 , 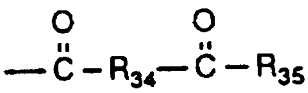 or

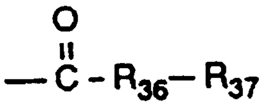 ,

$R_{33}$ is hydrogen or $C_1$-$C_4$alkyl,
$R_{34}$ is $C_1$-$C_{12}$alkylene, $C_2$-$C_8$alkenylene, $C_2$-$C_8$alkylidene, $C_7$-$C_{12}$phenylalkylidene, $C_5$-$C_8$cycloalkylene or phenylene,
$R_{35}$ is hydroxy, $[-O^{\theta} \frac{1}{r} M^{r+}]$ or $C_1$-$C_{18}$alkoxy,
$R_{36}$ is oxygen or -NH-,
$R_{37}$ is $C_1$-$C_8$alkyl or phenyl, and
s is 1 or 2.

**4.** A process according to claim 1, wherein

$R_1$ is phenanthryl, thienyl, dibenzofuryl, unsubstituted or $C_1$-$C_4$alkyl-substituted carbazolyl; or fluorenyl, or $R_1$ is a radical of formula V

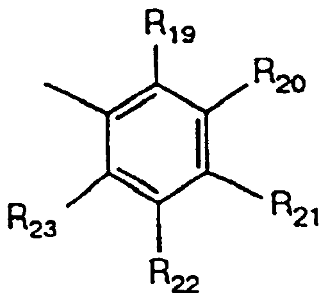 (V)

wherein $R_{19}$, $R_{20}$, $R_{21}$, $R_{22}$ and $R_{23}$ are each independently of one another hydrogen, chloro, hydroxy, $C_1$-$C_{18}$alkyl, $C_1$-$C_{18}$alkoxy, $C_1$-$C_{18}$alkylthio, $C_3$-$C_4$alkenyloxy, $C_3$-$C_4$-alkynyloxy, phenyl, benzoyl, benzoyloxy or

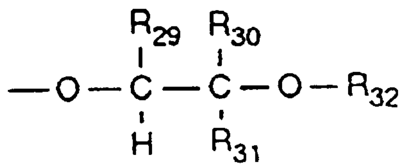

,

$R_{29}$ is hydrogen,
$R_{30}$ is hydrogen, phenyl or $C_1$-$C_{18}$alkyl or the radicals $R_{29}$ and $R_{30}$, together with the linking carbon atoms, form a cyclohexylene ring which is unsubstituted or substituted by 1 to 3 $C_1$-$C_4$alkyl groups,
$R_{31}$ is hydrogen or $C_1$-$C_4$alkyl, and
$R_{32}$ is hydrogen, $C_1$-$C_{12}$alkanoyl or benzoyl.

**5.** A process according to claim 4, wherein

$R_{19}$ is hydrogen or $C_1$-$C_4$alkyl,
$R_{20}$ is hydrogen or $C_1$-$C_4$alkyl,
$R_{21}$ is hydrogen, chloro, hydroxy, $C_1$-$C_{12}$alkyl, $C_1$-$C_4$-alkoxy, $C_1$-$C_4$alkylthio, phenyl or -O-$CH_2$-$CH_2$-O-$R_{32}$,
$R_{22}$ is hydrogen or $C_1$-$C_4$alkyl,
$R_{23}$ is hydrogen or $C_1$-$C_4$alkyl, and
$R_{32}$ is $C_1$-$C_4$alkanoyl.

**6.** A process according to claim 1, wherein

$R_2$, $R_3$, $R_4$ and $R_5$ are each independently of one another hydrogen, chloro, hydroxy, $C_1$-$C_{25}$alkyl, $C_7$-$C_9$phenylalkyl, unsubstituted or $C_1$-$C_4$alkyl-substituted phenyl, unsubstituted or $C_1$-$C_4$alkyl-substituted $C_5$-$C_8$cycloalkyl; $C_1$-$C_{12}$alkoxy, $C_1$-$C_{12}$alkylthio, $C_1$-$C_4$alkylamino, di($C_1$-$C_4$-alkyl)amino, $C_1$-$C_{18}$alkanoyloxy, $C_1$-$C_{18}$alkanoylamino, $C_3$-$C_{18}$alkenoyloxy, $C_3$-$C_{18}$alkanoyloxy which is interrupted by oxygen, sulfur or $>N—R_8$; $C_6$-$C_9$-cycloalkylcarbonyloxy, benzoyloxy or $C_1$-$C_8$alkyl-substituted benzoyloxy, or the radicals $R_2$ and $R_3$ or $R_3$ and $R_4$ or $R_4$ and $R_5$, together with the linking carbon atoms, form a benzo ring, $R_4$ is additionaly -$(CH_2)_p$-$COR_9$ or -$(CH_2)_q$OH, or, if $R_3$ and $R_5$ are hydrogen, $R_4$ is additionally a radical of formula II

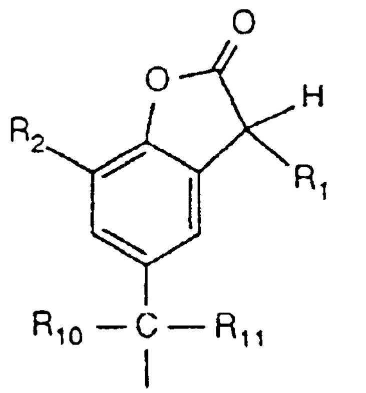

(II)

$R_8$ is hydrogen or $C_1$-$C_6$alkyl,
$R_9$ is hydroxy, $C_1$-$C_{18}$alkoxy or

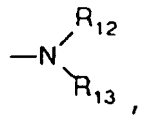

$R_{10}$ and $R_{11}$ are methyl groups or, together with the linking carbon atom, form a $C_5$-$C_8$cycloalkylidene ring which is unsubstituted or substituted by 1 to 3 $C_1$-$C_4$alkyl groups;
$R_{12}$ and $R_{13}$ are each independently of the other hydrogen or $C_1$-$C_8$alkyl, and
q is 2,3,4,5 or 6.

**7.** A process according to claim 1, wherein at least two of the radicals $R_2$, $R_3$, $R_4$ and $R_5$ are hydrogen.

**8.** A process according to claim 1, wherein $R_3$ and $R_5$ are hydrogen.

**9.** A process according to claim 1, wherein

$R_2$, $R_3$, $R_4$ and $R_5$ are each independently of one another hydrogen, chloro, hydroxy, $C_1$-$C_{18}$alkyl, $C_7$-$C_9$phenylalkyl, phenyl, $C_5$-$C_8$cycloalkyl, $C_1$-$C_6$alkoxy, cyclohexylcarbonyloxy or benzoyloxy, or the radicals $R_2$ and $R_3$ or $R_3$ and $R_4$ or $R_4$ and $R_5$, together with the linking carbon atoms, form a benzo ring, $R_4$ is additionally $-(CH_2)_p$-$COR_9$, or if $R_3$ and $R_5$ are hydrogen, $R_4$ is additionally a radical of formula II,
$R_9$ is hydroxy or $C_1$-$C_{18}$alkoxy, and
$R_{10}$ and $R_{11}$ are methyl groups or, together with the linking carbon atom, form a $c_5$-$C_8$cycloalkylidene ring.

**10.** A process according to claim 1, wherein

$R_2$ is $C_1$-$C_{19}$alkyl or cyclohexyl,
$R_3$ is hydrogen,
$R_4$ is $C_1$-$C_4$alkyl, cyclohexyl or a radical of formula II,
$R_5$ is hydrogen, and
$R_{10}$ and $R_{11}$, together with the linking carbon atom, form a cyclohexylidene ring.

**11.** A process according to claim 1, wherein

$R'_{15}$ is hydrogen, $C_1$-$C_{18}$alkanoyl, $C_3$-$C_{18}$alkenoyl, $C_3$-$C_{18}$-alkanoyl which is interrupted by oxygen, sulfur or $>$N—$R_8$; $C_6$-$C_9$cycloalkylcarbonyl, thenoyl, furoyl, benzoyl or $C_1$-$C_8$alkyl-substituted benzoyl; naphthoyl or $C_1$-$C_8$alkyl-substituted naphthoyl; $C_1$-$C_{18}$alkanesulfonyl, fluoro-substituted $C_1$-$C_{18}$alkanesulfonyl; phenylsulfonyl or $C_1$-$C_8$alkyl-substituted phenylsulfonyl;

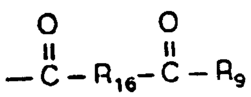 or 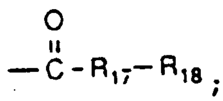

$R_{16}$ is a direct bond, $C_1$-$C_{12}$alkylene, $C_2$-$C_{12}$alkylene which is interrupted by oxygen, sulfur or $>$N—$R_8$; $C_2$-$C_{12}$alkenylene, $C_2$-$C_{12}$alkylidene, $C_7$-$C_{12}$phenylalkylidene, $C_5$-$C_8$cycloalkylene, $C_7$-$C_8$bicycloalkylene or phenylene,
$R_{17}$ is oxygen or -NH-, and
$R_{18}$ is $C_1$-$C_{12}$alkyl or phenyl.

**12.** A process according to claim 1, wherein

$R_{15}$ is chloro, bromo or $-OR'_{15}$,
$R'_{15}$ is hydrogen, $C_1$-$C_{12}$alkanoyl, $C_{3-12}$alkanoyl which is interrupted by oxygen; cyclohexylcarbonyl, benzoyl, naphthoyl, $C_1$-$C_{12}$alkanesulfonyl, fluoro-substituted $C_1$-$C_{12}$-alkanesulfonyl; phenylsulfonyl or $C_1$-$C_4$alkyl-sub-

stituted phenylsulfonyl; or

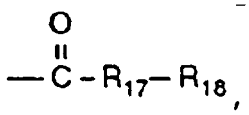

$R_{17}$ is -NH-, and
$R_{18}$ is $C_1$-$C_8$alkyl or phenyl.

**13.** A process according to claim 1, wherein

$R_{15}$ is -OR'$_{15}$,
R'$_{15}$ is hydrogen, $C_1$-$C_4$alkanoyl or

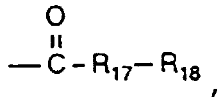

$R_{17}$ is -NH-, and
$R_{18}$ is $C_1$-$C_4$alkyl.

**14.** A process according to claim 1, wherein the reaction is carried out in the presence of a catalyst.

**15.** A process according to claim 14, wherein the catalyst is a protonic acid, a Lewis acid, an aluminium silicate, an ion exchange resin, a zeolite, a naturally occurring sheet silicate or a modified sheet silicate.

**16.** A process according to claim 14, wherein the catalyst is a naturally occurring sheet silicate or a modified sheet silicate.

**17.** A process according to claim 16, wherein the naturally occurring sheet silicate is a Fulcat® or Fulmont® type.

**18.** A process according to claim 1, wherein, when n is 1, the molar ratio of the compound of formula III to the compound of formula IV is 1:1 to 1:20 and, when n is 2, the molar ratio of the compound of the formula III to the compound of formula IV is 3:1 to 2:1.

**19.** A process for the preparation of a compound of formula I

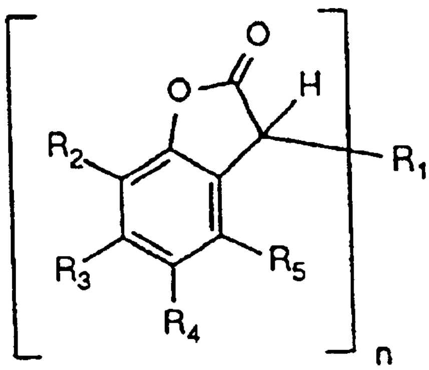

(I)

wherein the general symbols are as defined in claim 1, which comprises reacting one equivalent of a phenol of for-

mula VII

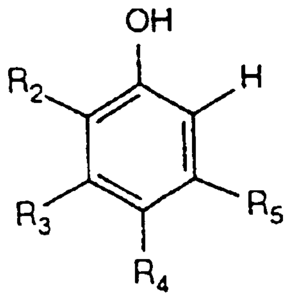

(VII)

wherein the general symbols are as defined in claim 1, with 0.8 to 2.0 equivalents of glyoxylic acid to give a compound of formula VIII

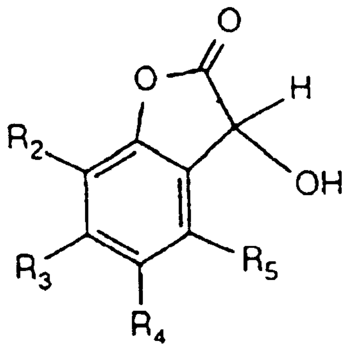

(VIII)

wherein the general symbols are as defined in claim 1, and subsequently reacting said compound of formula VIII, without isolation, with a compound of formula IV

$$[H]_n\text{-}R_1 \qquad (IV)$$

wherein $R_1$ and n as as defined in claim 1.

**20.** A process according to claim 19, which comprises reacting the compound of formula VIII without isolation before the further reaction with a compound of formula IV in an additional reaction step with a hydrohalic acid, a halide of a sulfur oxyacid, a halide of phosphoric acid, a halide of phosphorous acid, an acid of formula IX

$$R'_{15}\text{-OH} \qquad (IX),$$

an acid halide of formula X

$$R'_{15}\text{-Y} \qquad (X),$$

an ester of formula XI

$$R'_{15}\text{-O-}R_{38} \qquad (XI),$$

a symmetrical or unsymmetrical anhydride of formula XII

$$R'_{15}\text{-O-}R'_{15} \qquad (XII)$$

or an isocyanate of formula XIII

$R_{39}$-N=C=O (XIII)

wherein $R'_{15}$ in the compounds of formulae IX, X, XI and XII is as defined in claim 1, with the proviso that

$R'_{15}$ is not hydrogen;
$R_{38}$ is $C_1$-$C_8$alkyl,
$R_{39}$ is $C_1$-$C_{18}$alkyl or phenyl, and
Y is fluoro, chloro, bromo or iodo, to give a compound of formula III

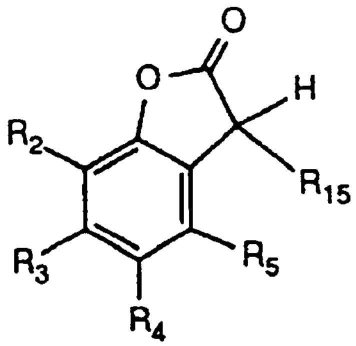 (III)

wherein the radicals $R_2$, $R_3$, $R_4$, $R_5$ and $R_{15}$ are as defined in claim 1, and, if $R_{15}$ = -OR'$_{15}$, $R'_{15}$ is not hydrogen.

**Revendications**

**1.** Procédé de préparation de composés de formule I

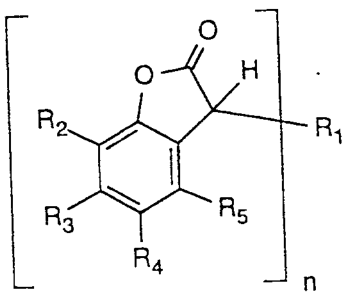 (I)

où, lorsque n vaut 1,

$R_1$ représente des groupes phényle, naphtyle, phénanthryle, anthryle, 5,6,7,8-tétrahydro-2-naphtyle, thiényle, benzo[b]thiényle, naphto[2,3-b]thiényle, thiathrényle, furyle, benzofuryle, isobenzofuryle, dibenzofuryle, chroményle, xanthényle, phénoxanthiinyle, pyrrolyle, imidazolyle, pyrazolyle, pyrazinyle, pyrimidinyle, pyridazinyle, indolizinyle, isoindolyle, indolyle, indazolyle, purinyle, quinolizinyle, isoquinolyle, quinolyle, phtalazinyle, naphthyridinyle, quinoxalinyle, quinazolinyle, cinnolinyle, ptéridinyle, carbazolyle, β-carbolinyle, phénanthridinyle, acridinyle, périmidinyle, phénanthrolinyle, phénazinyle, isothiazolyle, phénothiazinyle, isoxazolyle, furazanyle, biphényle, tétralinyle, fluorényle ou phénoxazinyle non substitué ou substitué par des substituants chloro, amino, hydroxy, alkyle en $C_1$-$C_{18}$, alkoxy en $C_1$-$C_{18}$, (alkyl en $C_1$-$C_{18}$)thio, alcényloxy en $C_3$-$C_4$, alcynyloxy en $C_3$-$C_4$, (alkyl en $C_1$-$C_4$)amino, di-(alkyl en $C_1$-$C_4$)amino, phényle, benzyle, benzoyle ou benzoyloxy, ou $R_1$ représente un reste de formule V ou VI

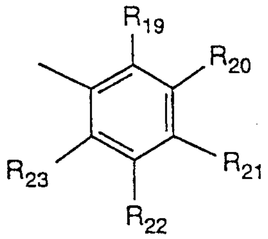 (V) 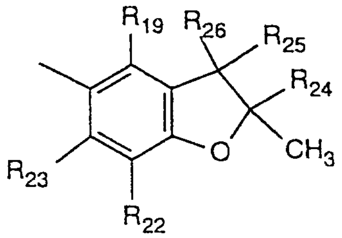 (VI),

lorsque n vaut 2,

$R_1$ représente un groupe phénylène ou naphtylène non substitué ou substitué par des substituants alkyle en $C_1$-$C_4$ ou hydroxy ; ou représente un groupe

$R_2$, $R_3$, $R_4$ et $R_5$ représentent, indépendamment les uns des autres, des atomes d'hydrogène, des atomes de chlore, des groupes hydroxy, alkyle en $C_1$-$C_{25}$, phénylalkyle en $C_7$-$C_9$, phényle non substitué ou substitué par des substituants alkyle en $C_1$-$C_4$, cycloalkyle en $C_5$-$C_8$ non substitué ou substitué par des substituants alkyle en $C_1$-$C_4$ ; alkoxy en $C_1$-$C_{18}$, (alkyl en $C_1$-$C_{18}$)thio, (alkyl en $C_1$-$C_4$)-amino, di-(alkyl en $C_1$-$C_4$)-amino, alcanoyloxy en $C_1$-$C_{25}$, (alcanoyl en $C_1$-$C_{25}$)amino, alcénoyloxy en $C_3$-$C_{25}$, alcanoyloxy en $C_3$-$C_{25}$ interrompu par des atomes d'oxygène, de soufre ou des groupes $>N-R_8$; (cycloalkyl en $C_6$-$C_9$)carbonyloxy, benzoyloxy ou benzoyloxy substitué par des substituants alkyle en $C_1$-$C_{12}$, ou de plus les restes $R_2$ et $R_3$ ou les restes $R_3$ et $R_4$ ou les restes $R_4$ et $R_5$ forment, conjointement avec les atomes de carbone auxquels ils sont liés, un cycle benzo, $R_4$ représente de plus un groupe $-(CH_2)_p-COR_9$ ou $-(CH_2)_qOH$, ou lorsque $R_4$ et $R_5$ représentent des atomes d'hydrogène, $R_4$ représente de plus un reste de formule II

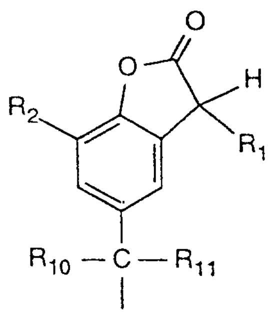 (II)

où

$R_1$ est défini comme donné ci-dessus pour n = 1,

$R_6$ et $R_7$ représentent, indépendamment l'un de l'autre, des groupes phénylène ou naphtylène non substitué ou substitué par des substituants alkyle en $C_1$-$C_4$,

$R_8$ représente un atome d'hydrogène ou un groupe alkyle en $C_1$-$C_8$,

$R_9$ représente un atome d'hydrogène, des groupes $[-O^{\theta}\ \frac{1}{r}\ M^{r+}]$, alkoxy en $C_1$-$C_{18}$ ou

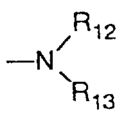

$R_{10}$ et $R_{11}$ représentent, indépendamment l'un de l'autre, des atomes d'hydrogène, des groupes $CF_3$, alkyle en $C_1$-$C_{12}$ ou phényle, ou $R_{10}$ et $R_{11}$ forment, conjointement avec l'atome de carbone auquel ils sont liés, un cycle cycloalkylidène en $C_5$-$C_8$ non substitué ou substitué par 1 à 3 substituants alkyle en $C_1$-$C_4$ ;

$R_{12}$ et $R_{13}$ représentent, indépendamment l'un de l'autre, des atomes d'hydrogène ou des groupes alkyle en $C_1$-$C_{18}$,

$R_{14}$ représente un atome d'hydrogène ou un groupe alkyle en $C_1$-$C_{18}$,

$R_{19}$, $R_{20}$, $R_{21}$, $R_{22}$ et $R_{23}$ représentent, indépendamment les uns des autres, des atomes d'hydrogène, des atomes d'halogène, des groupes hydroxy, alkyle en $C_1$-$C_{25}$, alkyle en $C_2$-$C_{25}$ interrompu par des atomes d'oxygène, de soufre ou des groupes $>N-R_8$; alkoxy en $C_1$-$C_{25}$, alkoxy en $C_2$-$C_{25}$, interrompu par des atomes d'oxygène, de soufre ou des groupes $>N-R_8$; (alkyl en $C_1$-$C_{25}$)thio, alcényle en $C_3$-$C_{25}$, alcényloxy en $C_3$-$C_{25}$, alcynyle en $C_3$-$C_{25}$, alcynyloxy en $C_3$-$C_{25}$, phénylalkyle en $C_7$-$C_9$, phénylalkoxy en $C_7$-$C_9$, phényle non substitué ou substitué par des substituants alkyle en $C_1$-$C_4$ ; phénoxy non substitué ou substitué par des substituants alkyle en $C_1$-$C_4$ ; cycloalkyle en $C_5$-$C_8$ non substitué ou substitué par des substituants alkyle en $C_1$-$C_4$ ; cycloalkoxy en $C_5$-$C_8$ non substitué ou substitué par des substituants alkyle en $C_1$-$C_4$ ; (alkyl en $C_1$-$C_4$)-amino, di-(alkyl en $C_1$-$C_4$)amino, alcanoyle en $C_1$-$C_{25}$, alcanoyle en $C_3$-$C_{25}$ interrompu par des atomes d'oxygène, de soufre ou des groupes $>N-R_8$; alcanoyloxy en $C_1$-$C_{25}$, alcanoyloxy en $C_3$-$C_{25}$ interrompu par des atomes d'oxygène, de soufre ou des groupes $>N-R_8$; (alcanoyl en $C_1$-$C_{25}$)-amino, alcénoyle en $C_3$-$C_{25}$, alcénoyle en $C_3$-$C_{25}$ interrompu par des atomes d'oxygène, de soufre ou des groupes $>N-R_8$; alcénoyloxy en $C_3$-$C_{25}$, alcénoyloxy en $C_3$-$C_{25}$ interrompu par des atomes d'oxygène, de soufre ou des groupes $>N-R_8$; cyclo(alkyl en $C_6$-$C_9$)-carbonyle, cyclo(alkyl en $C_6$-$C_9$)carbonyloxy, benzoyle ou benzoyle substitué par des substituants alkyle en $C_1$-$C_{12}$ ; benzoyloxy ou benzoyloxy substitué par des substituants alkyle en $C_1$-$C_{12}$ ; représentent

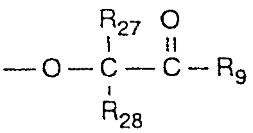 , 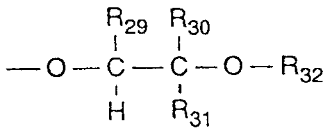

ou de plus, dans la formule V, les restes $R_{19}$ et $R_{20}$ ou les restes $R_{20}$ et $R_{21}$ forment, conjointement avec les atomes de carbone auxquels ils sont liés, un cycle benzo,

$R_{24}$ représente un atome d'hydrogène, des groupes alkyle en $C_1$-$C_4$ ou phényle non substitué ou substitué par des substituants alkyle en $C_1$-$C_4$,

$R_{25}$ et $R_{26}$ représentent des atomes d'hydrogène, des groupes alkyle en $C_1$-$C_4$ ou phényle, à condition qu'au moins un des restes $R_{25}$ et $R_{26}$ représente un atome d'hydrogène,

$R_{27}$ et $R_{28}$ représentent, indépendamment l'un de l'autre, des atomes d'hydrogène, des groupes alkyle en $C_1$-$C_4$ ou phényle,

$R_{29}$ représente un atome d'hydrogène ou un groupe alkyle en $C_1$-$C_4$,

$R_{30}$ représente un atome d'hydrogène ou des groupes phényle non substitué ou substitué par des substituants alkyle en $C_1$-$C_4$ ; alkyle en $C_1$-$C_{25}$, alkyle en $C_2$-$C_{25}$ interrompu par des atomes d'oxygène, de soufre ou des groupes $>N-R_8$; phénylalkyle en $C_7$-$C_9$ non substitué ou substitué sur le reste phényle par 1 à 3 substituants alkyle en $C_1$-$C_4$ ; phénylalkyle en $C_7$-$C_{25}$ non substitué interrompu par des atomes d'oxygène, de soufre ou des groupes $>N-R_8$ ou substitué sur le reste phényle par 1 à 3 substituants alkyle en $C_1$-$C_4$ ou, de plus, les restes $R_{29}$ et $R_{30}$ forment, conjointement avec les atomes de carbone auxquels ils sont liés, un cycle cycloalkylène en $C_5$-$C_{12}$ non substitué ou substitué par 1 à 3 substituants alkyle en $C_1$-$C_4$ ;

$R_{31}$ représente un atome d'hydrogène ou un groupe alkyle en $C_1$-$C_4$,

$R_{32}$ représente un atome d'hydrogène, des groupes alcanoyle en $C_1$-$C_{25}$, alcénoyle en $C_3$-$C_{25}$, alcanoyle en $C_3$-$C_{25}$ interrompu par des atomes d'oxygène, de soufre ou des groupes $>N-R_8$; alcanoyle en $C_2$-$C_{25}$ substitué par un groupe di(alkyl en $C_1$-$C_6$)-phosphonate ; cyclo(alkyl en $C_6$-$C_9$)carbonyle, thénoyle, furoyle, benzoyle ou benzoyle substitué par des substituants alkyle en $C_1$-$C_{12}$ ;

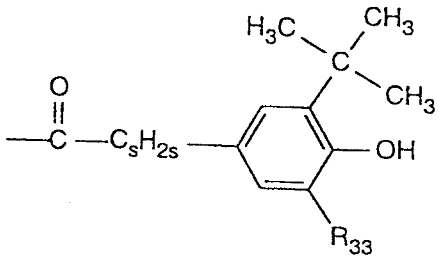 , 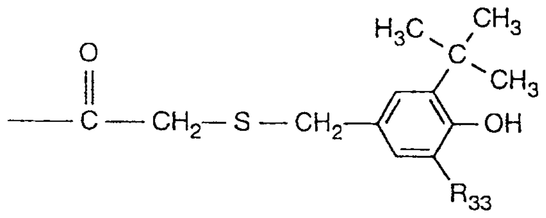 ,

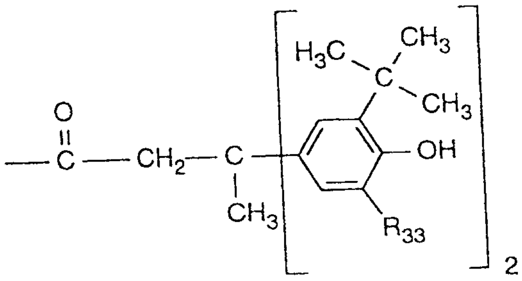 , 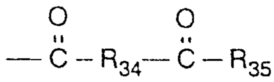 ou

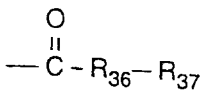

$R_{33}$ représente un atome d'hydrogène ou un groupe alkyle en $C_1$-$C_8$,

$R_{34}$ représente une liaison directe, des groupes alkylène en $C_1$-$C_{18}$, alkylène en $C_2$-$C_{18}$ interrompu par des atomes d'oxygène, de soufre ou des groupes $>N-R_8$; alcénylène en $C_2$-$C_{18}$, alkylidène en $C_2$-$C_{20}$, phénylalkylidène en $C_7$-$C_{20}$, cycloalkylène en $C_5$-$C_8$, bicycloalkylène en $C_7$-$C_8$, phénylène non substitué ou substitué par des substituants alkyle en $C_1$-$C_4$ ;

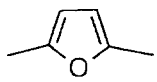 ou 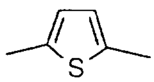

$R_{35}$ représente des groupes hydroxy, [$-O^{\theta} \frac{1}{r} M^{r+}$], alkoxy en $C_1$-$C_{18}$ ou

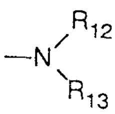

$R_{36}$ représente un atome d'oxygène, des groupes -NH- ou

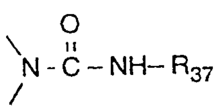

$R_{37}$ représente des groupes alkyle en $C_1$-$C_{18}$ ou phényle,

M est un cation métallique à fonctionnalité r,

X représente une liaison directe, un atome d'oxygène, un atome de soufre ou un groupe $-NR_{14}-$,

n vaut 1 ou 2,

p vaut 0, 1 ou 2,

q vaut 1, 2, 3, 4, 5 ou 6,

r vaut 1, 2 ou 3, et

s vaut 0, 1 ou 2,

caractérisé en ce qu'on fait réagir un composé de formule III

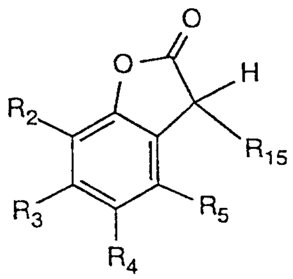

(III)

où

$R_{15}$ représente un atome d'halogène ou un groupe $-OR_{15}$,

$R_{15}$ représente un atome d'hydrogène, des groupes alcanoyle en $C_1$-$C_{25}$, alcénoyle en $C_3$-$C_{25}$, alcanoyle en $C_3$-$C_{25}$ interrompu par des atomes d'oxygène, de soufre ou des groupes $>N—R_8$; cyclo(alkyl en $C_6$-$C_9$)carbonyle, thénoyle, furoyle, benzoyle ou benzoyle substitué par des substituants alkyle en $C_1$-$C_{12}$ ; naphtoyle ou naphtoyle substitué par des substituants alkyle en $C_1$-$C_{12}$ ; (alcane en $C_1$-$C_{25}$)-sulfonyle, (alcane en $C_1$-$C_{25}$)sulfonyle substitué par des atomes de fluor ; phénylsulfonyle ou phénylsulfonyle substitué par des substituants alkyle en $C_1$-$C_{12}$ ; ou

$R_{16}$ représente une liaison directe, des groupes alkylène en $C_1$-$C_{18}$, alkylène en $C_2$-$C_{18}$ interrompu par des atomes d'oxygène, de soufre ou des groupes $>N—R_8$; alcénylène en $C_2$-$C_{18}$, alkylidène en $C_2$-$C_{20}$, phénylalkylidène en $C_7$-$C_{20}$, cycloalkylène en $C_5$-$C_8$, bicycloalkylène en $C_7$-$C_8$, phénylène non substitué ou substitué par un substituant alkyle en $C_1$-$C_4$ ;

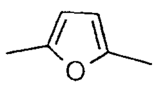 ou 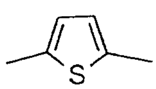

$R_{17}$ représente un atome d'oxygène, des groupes -NH- ou

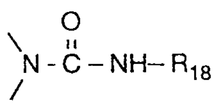 ,

et

$R_{18}$ représente des groupes alkyle en $C_1$-$C_{18}$ ou phényle,

avec un composé de formule IV

$$[H]_n\text{-}R_1 \qquad (IV).$$

**2.** Procédé selon la revendication 1, lorsque n vaut 2,

$R_1$ représente un groupe $-R_6$-X-$R_7$-,

$R_6$ et $R_7$ représentent un groupe phénylène,

X représente un atome d'oxygène ou un groupe $-NR_{14}-$, et

$R_{14}$ représente un groupe alkyle en $C_1$-$C_4$.

**3.** Procédé selon la revendication 1, où

$R_{19}$, $R_{20}$, $R_{21}$, $R_{22}$ et $R_{23}$ représentent, indépendamment les uns des autres, des atomes d'hydrogène, des atomes de chlore, des atomes de brome, des groupes hydroxy, alkyle en $C_1$-$C_{18}$, alkyle en $C_2$-$C_{18}$ interrompu par des atomes d'oxygène ou de soufre ; alkoxy en $C_1$-$C_{18}$, alkoxy en $C_2$-$C_{18}$ interrompu par des atomes d'oxygène ou de soufre ; (alkyl en $C_1$-$C_{18}$)-thio, alcényloxy en $C_3$-$C_{12}$, alcynyloxy en $C_3$-$C_{12}$, phénylalkyle en $C_7$-$C_9$, phénylalkoxy en $C_7$-$C_9$, phényle non substitué ou substitué par des substituants alkyle en $C_1$-$C_4$ ; phénoxy, cyclohexyle, cycloalkoxy en $C_5$-$C_8$ ; (alkyl en $C_1$-$C_4$)-amino, di-(alkyl en $C_1$-$C_4$)amino, alcanoyle en $C_1$-$C_{12}$, alcanoyle en $C_3$-$C_{12}$ interrompu par des atomes d'oxygène ou de soufre ; alcanoyloxy en $C_1$-$C_{12}$, alcanoyloxy en $C_3$-$C_{12}$ interrompu par des atomes d'oxygène ou de soufre ; (alcanoyl en $C_1$-$C_{12}$)-amino, alcénoyle en $C_3$-$C_{12}$, alcénoyloxy en $C_3$-$C_{12}$, cyclohexylcarbonyle, cyclohexylcarbonyloxy, benzoyle ou benzoyle substitué par des substituants alkyle en $C_1$-$C_4$ ; benzoyloxy ou benzoyloxy substitué par des substituant alkyle en $C_1$-$C_4$ ;

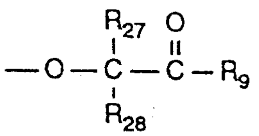

,

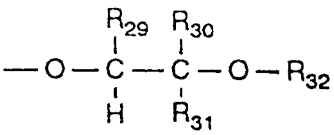

;

ou, de plus, dans la formule V, les restes $R_{19}$ et $R_{20}$ ou les restes $R_{20}$ et $R_{21}$ forment, conjointement avec les atomes de carbone auxquels ils sont liés, un cycle benzo,

$R_{24}$ représente un atome d'hydrogène ou un groupe alkyle en $C_1$-$C_4$,

$R_{25}$ et $R_{26}$ représentent des atomes d'hydrogène ou des groupes alkyle en $C_1$-$C_4$, à condition qu'au moins un des restes $R_{25}$ et $R_{26}$ représente un atome d'hydrogène,

$R_{27}$ et $R_{28}$ représentent, indépendamment l'un de l'autre, des atomes d'hydrogène ou des groupes alkyle en

$R_{29}$ représente un atome d'hydrogène,

$R_{30}$ représente un atome d'hydrogène, des groupes phényle, alkyle en $C_1$-$C_{18}$, alkyle en $C_2$-$C_{18}$ interrompu par des atomes d'oxygène ou de soufre ; phénylalkyle en $C_7$-$C_9$, phénylalkyle en $C_7$-$C_{18}$ non substitué interrompu par des atomes d'oxygène ou de soufre ou substitué sur le reste phényle par 1 à 3 substituants alkyle en $C_1$-$C_4$ ou, de plus, les restes $R_{29}$ et $R_{30}$ forment, conjointement avec les atomes de carbone auxquels ils sont liés, un cycle cyclohexylène non substitué ou substitué par 1 à 3 substituants alkyle en $C_1$-$C_4$ ;

$R_{31}$ représente un atome d'hydrogène ou un groupe alkyle en $C_1$-$C_4$,

$R_{32}$ représente un atome d'hydrogène, des groupes alcanoyle en $C_1$-$C_{18}$, alcénoyle en $C_3$-$C_{12}$, alcanoyle en $C_3$-$C_{12}$ interrompu par des atomes d'oxygène ou de soufre ; alcanoyle en $C_2$-$C_{12}$ substitué par un groupe di(alkyl en $C_1$-$C_6$)-phosphonate ; cyclo(alkyl en $C_6$-$C_9$)carbonyle, benzoyle,

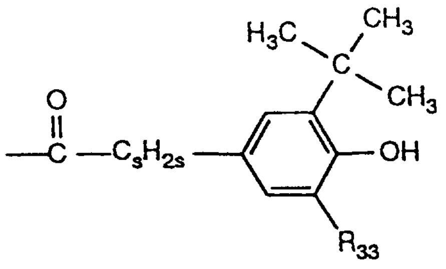 , 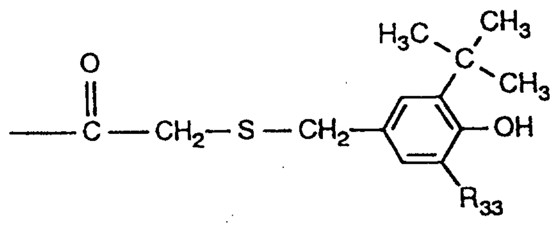 ,

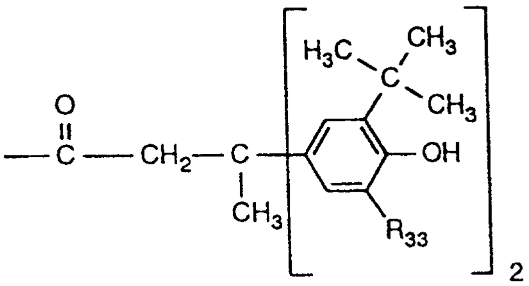 , 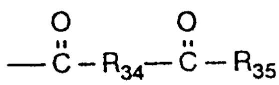 ou 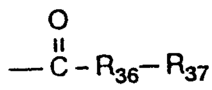

| | |
|---|---|
| $R_{33}$ | représente un atome d'hydrogène ou un groupe alkyle en $C_1$-$C_4$, |
| $R_{34}$ | représente des groupes alkylène en $C_1$-$C_{12}$, alcénylène en $C_2$-$C_8$, alkylidène en $C_2$-$C_8$, phénylalkylidène en $C_7$-$C_{12}$, cycloalkylène en $C_5$-$C_8$ ou phénylène, |
| $R_{35}$ | représents des groupes hydroxy, [$-O^{\theta} \frac{1}{r} M^{r+}$] ou alkoxy en $C_1$-$C_{18}$, |
| $R_{36}$ | représente un atome d'oxygène ou des groupes -NH-, |
| $R_{37}$ | représente des groupes alkyle en $C_1$-$C_8$ ou phényle et |
| s | vaut 0, 1 ou 2. |

**4.** Procédé selon la revendication 1, où

$R_1$ représente des groupes phénanthryle, thiényle, dibenzofuryle, carbazolyle non substitué ou susbtitué par des substituants alkyle en $C_1$-$C_4$ ; ou représente un groupe fluorényle, ou $R_1$ représente un reste de formule V

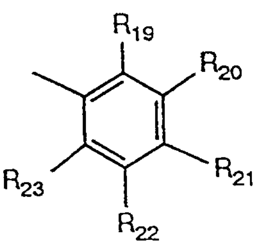 (V)

dans laquelle

$R_{19}$, $R_{20}$, $R_{21}$, $R_{22}$ et $R_{23}$ représentent, indépendamment les uns des autres, des atomes d'hydrogène, des atomes de chlore, des groupes hydroxy, alkyle en $C_1$-$C_{18}$, alkoxy en $C_1$-$C_{18}$, (alkyl en $C_1$-$C_{18}$)thio, alcénoyloxy en $C_3$-$C_4$, alcynyloxy en $C_3$-$C_4$, phényle benzoyle, benzoyloxy ou

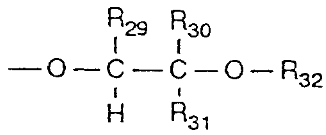

| | |
|---|---|
| $R_{29}$ | représente un atome d'hydrogène, |
| $R_{30}$ | représente un atome d'hydrogène, des groupes phényle ou alkyle en $C_1$-$C_{18}$ ou, de plus, les restes $R_{29}$ et $R_{30}$ forment, conjointement avec les atomes de carbone auxquels ils sont liés, un cycle cyclohexylène non substitué ou substitué par 1 à 3 substituants alkyle en $C_1$-$C_4$ ; |
| $R_{31}$ | représente un atome d'hydrogène ou un groupe alkyle en $C_1$-$C_4$, et |
| $R_{32}$ | représente un atome d'hydrogène, des groupes alcanoyle en $C_1$-$C_{12}$ ou benzoyle. |

**5.** Procédé selon la revendication 4, où

| | |
|---|---|
| $R_{19}$ | représente un atome d'hydrogène ou un groupe alkyle en $C_1$-$C_4$, |
| $R_{20}$ | représente un atome d'hydrogène ou un groupe alkyle en $C_1$-$C_4$, |
| $R_{21}$ | représente un atome d'hydrogène, un atome de chlore, des groupes hydroxy, alkyle en $C_1$-$C_{12}$, alkoxy en $C_1$-$C_4$, (alkyl en $C_1$-$C_4$)thio, phényle ou $-O-CH_2CH_2-O-R_{32}$, |
| $R_{22}$ | représente un atome d'hydrogène ou un groupe alkyle en $C_1$-$C_4$, |
| $R_{23}$ | représente un atome d'hydrogène ou un groupe alkyle en $C_1$-$C_4$, et |
| $R_{32}$ | représente un groupe alcanoyle en $C_1$-$C_4$. |

**6.** Procédé selon la revendication 1, où

$R_2$, $R_3$, $R_4$ et $R_5$ représentent, indépendamment les uns des autres, des atomes d'hydrogène, des atomes de chlore, des groupes hydroxy, alkyle en $C_1$-$C_{25}$, phénylalkyle en $C_7$-$C_9$, phényle non substitué ou substitué par des substituants alkyle en $C_1$-$C_4$, cycloalkyle en $C_5$-$C_8$ non substitué ou substitué par des substituants alkyle en $C_1$-$C_4$ ; alkoxy en $C_1$-$C_{12}$, (alkyl en $C_1$-$C_{12}$)thio, (alkyl en $C_1$-$C_4$)-amino, di-(alkyl en $C_1$-$C_4$)amino, alcanoyloxy en $C_1$-$C_{18}$, (alcanoyl en $C_1$-$C_{18}$)amino, alcénoyloxy en $C_3$-$C_{18}$, alcanoyloxy en $C_3$-$C_{18}$ interrompu par des atomes d'oxygène, de soufre ou des groupes $>N-R_8$; cyclo(alkyl en $C_6$-$C_9$)carbonyloxy, benzoyloxy ou benzoyloxy substitué par des substituants alkyle en $C_1$-$C_8$ ou, de plus, les restes $R_2$ et $R_3$ ou les restes $R_3$ et $R_4$ ou les restes $R_4$ et $R_5$ forment, conjointement avec les atomes de carbone auxquels ils sont liés, un cycle benzo, $R_4$ de plus représente un groupe $-(CH_2)_p-COR_9$ ou $-(CH_2)_qOH$ ou, lorsque $R_3$ et $R_5$ représentent des atomes d'hydrogène, $R_4$ représente de plus un reste de formule II

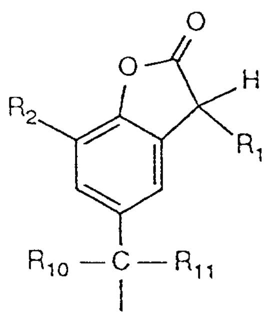

(II)

| | |
|---|---|
| $R_8$ | représente un atome d'hydrogène ou un groupe alkyle en $C_1$-$C_6$, |
| $R_9$ | représente des groupes hydroxy, alkoxy en $C_1$-$C_{18}$ ou |

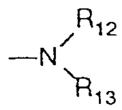

| | |
|---|---|
| $R_{10}$ et $R_{11}$ | représentent des groupes méthyle ou forment, conjointement avec l'atome de carbone auquel ils sont liés, un cycle cycloalkylidène en $C_5$-$C_8$ non substitué ou substitué par 1 à 3 groupes alkyle en $C_1$-$C_4$, |
| $R_{12}$ et $R_{13}$ | représentent, indépendamment l'un de l'autre, un atome d'hydrogène ou un groupe alkyle en $C_1$-$C_8$, et |
| q | vaut 2, 3, 4 ou 5. |

7. Procédé selon la revendication 1, où au moins deux des restes $R_2$, $R_3$, $R_4$ et $R_5$ représentent des atomes d'hydrogène.

8. Procédé selon la revendication 1, où $R_3$ et $R_5$ représentent des atomes d'hydrogène.

9. Procédé selon la revendication 1, où

| | |
|---|---|
| $R_2$, $R_3$, $R_4$ et $R_5$ | représentent, indépendamment les uns des autres, des atomes d'hydrogène, des atomes de chlore, des groupes hydroxy, alkyle en $C_1$-$C_{18}$, phénylalkyle en $C_7$-$C_9$, phényle, cycloalkyle en $C_5$-$C_8$, alkoxy en $C_1$-$C_6$, cyclohexylcarbonyloxy ou benzoyloxy ou, de plus, les restes $R_2$ et $R_3$ ou les restes $R_3$ et $R_4$ ou les restes $R_4$ et $R_5$ forment, conjointement avec les atomes de carbone auxquels ils sont liés, un cycle benzo, $R_4$ représente de plus un groupe $-(CH_2)_p-COR_9$, ou lorsque $R_3$ et $R_5$ représentent des atomes d'hydrogène, $R_4$ représente de plus un reste de formule II, |
| $R_9$ | représente des groupes hydroxy ou alkoxy en $C_1$-$C_{18}$ |
| $R_{10}$ et $R_{11}$ | représentent des groupes méthyle ou forment, conjointement avec l'atome de carbone auquel ils sont liés, un cycle cycloalkylidène en $C_5$-$C_8$. |

10. Procédé selon la revendication 1, où

| | |
|---|---|
| $R_2$ | représente des groupes alkyle en $C_1$-$C_{18}$ ou cyclohexyle, |
| $R_3$ | représente un atome d'hydrogène, |
| $R_4$ | représente des groupes alkyle en $C_1$-$C_4$, cyclohexyle ou un reste de formule II, |
| $R_5$ | un atome d'hydrogène, et |
| $R_{10}$ et $R_{11}$ | forment, conjointement avec l'atome de carbone auquel ils sont lié, un cycle cyclohexylidène. |

11. Procédé selon la revendication 1, où

$R_{15}$ représente un atome d'hydrogène, des groupes alcanoyle en $C_1$-$C_{18}$, alcénoyle en $C_3$-$C_{18}$, alcanoyle en $C_3$-$C_{18}$ interrompu par des atomes d'oxygène, de soufre ou des groupes $>N-R_8$; cyclo(alkyl en $C_6$-$C_9$)-carbonyle, thénoyle, furoyle, benzoyle ou benzoyle substitué par des substituants alkyle en $C_1$-$C_8$ ; naphtoyle ou naphtoyle substitué par des substituants alkyle en $C_1$-$C_8$ ; (alcane en $C_1$-$C_{18}$)-sulfonyle substitué par des atomes de fluor ; phénylsulfonyle ou phénylsulfonyle substitué par des substituants alkyle en $C_1$-$C_8$ ;

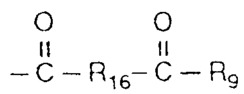 ou 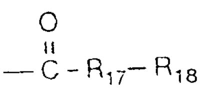

$R_{16}$ représente une liaison directe, des groupes alkylène en $C_1$-$C_{12}$, alkylène en $C_2$-$C_{12}$ interrompu par des atomes d'oxygène, de soufre ou des groupes $>N-R_8$; alcènylène en $C_2$-$C_{12}$, alkylidène en $C_2$-$C_{12}$, phé-

nylalkylidène en $C_7$-$C_{12}$, cycloalkylène en $C_5$-$C_8$, bicycloalkylène en $C_7$-$C_8$ ou phénylène,

$R_{17}$ représente un atome d'oxygène ou un groupe -NH-, et

$R_{18}$ représente des groupes alkyle en $C_1$-$C_{12}$ ou phényle.

**12.** Procédé selon la revendication 1, où

$R_{15}$ représente un atome de chlore, un atome de brome ou un groupe -OR'$_{15}$,

$R'_{15}$ représente un atome d'hydrogène, des groupes alcanoyle en $C_1$-$C_{12}$, alcanoyle en $C_3$-$C_{12}$ interrompu par des atomes d'oxygène, cyclohexylcarbonyle, benzoyle, naphtoyle, (alcane en $C_1$-$C_{12}$)-sulfonyle, (alcane en $C_1$-$C_{12}$)sulfonyle substitué par des atomes de fluor ; phénylsulfonyle ou phénylsulfonyle substitué par un substituant alkyle en $C_1$-$C_4$ ; ou

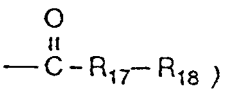

$R_{17}$ représente un groupe -NH-, et

$R_{18}$ représente des groupes alkyle en $C_1$-$C_8$ ou phényle.

**13.** Procédé selon la revendication 1, où

$R_{15}$ représente un groupe -OR$_{15}$,

$R'_{15}$ représente un atome d'hydrogène, des groupes alcanoyle en $C_1$-$C_4$ ou

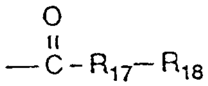

$R_{17}$ représente un groupe -NH-, et

$R_{18}$ représente un groupe alkyle en $C_1$-$C_4$.

**14.** Procédé selon la revendication 1, où la transformation est mise en oeuvre en présence d'un catalyseur.

**15.** Procédé selon la revendication 14, où le catalyseur est un acide protonique, un acide de Lewis, un silicate d'aluminium, une résine échangeuse d'ions, une zéolite, un phyllosilicate naturel ou un phyllosilicate modifié.

**16.** Procédé selon la revendication 14, où le catalyseur est un phyllosilicate naturel ou un phyllosilicate modifié.

**17.** Procédé selon la revendication 16, où le phyllosilicate naturel est un phyllosilicate du type Fulcat® ou Fulmont®.

**18.** Procédé selon la revendication 1, où, lorsque n vaut 1, le rapport molaire du composé de formule III au composé de formule IV est de 1:1 à 1:20 et lorsque n vaut 2, le rapport molaire du composé de formule III au composé de formule IV est de 3:1 à 2:1.

**19.** Procédé de préparation de composés de formule I

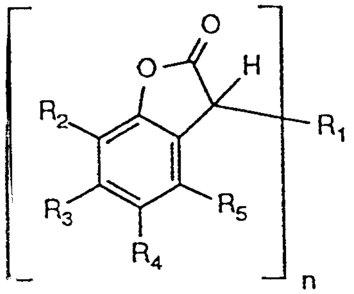

(I)

où les symboles généraux sont définis comme à la revendication 1, caractérisé en ce qu'on fait réagir 1 équivalent de phénol de formule VII

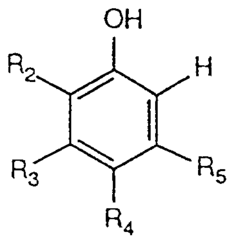

(VII)

où les symboles généraux sont définis comme à la revendication 1, avec 0,8 à 2,0 équivalents d'acide glyoxylique pour obtenir un composé de formule VIII

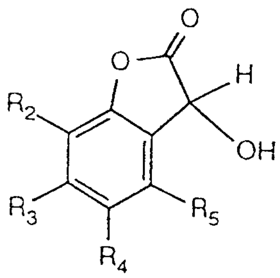

(VIII)

où les symboles généraux sont définis comme à la revendication 1, et ensuite, on fait réagir celui-ci sans isolement avec un composé de formule IV

$$[H]_n\text{-}R_1 \qquad (IV)$$

où $R_1$ et n sont définis comme à la revendication 1.

**20.** Procédé selon la revendication 19, où on fait régir le composé de formule VIII sans isolement, avant la transformation ultérieure avec un composé de formule IV, dans un autre stade de réaction, avec un hydracide halogéné, un halogénure d'un acide thio-oxygéné, un halogénure de l'acide phosphorique, un halogénure de l'acide phosphoreux, un acide de formule IX

$$R'_{15}\text{-OH} \qquad (IX)$$

un halogénure d'acide de formule X

$$R'_{15}\text{-Y} \qquad (X)$$

un ester de formule XI

$$R'_{15}\text{-O-}R_{38} \qquad \text{(XI)}$$

un anhydride symétrique ou asymétrique de formule XII

$$R'_{15}\text{-O-}R'_{15} \qquad \text{(XII)}$$

ou un isocyanate de formule XIII

$$R_{39}\text{-N=C=O} \qquad \text{(XIII)}$$

où $R_{15}$ possède la signification donnée ci-dessus, en ce que $R'_{15}$, dans les composés de formules IX, X, XI et XII, ne représente pas un atome d'hydrogène ;

$R_{38}$ représente un groupe alkyle en $C_1$-$C_8$,
$R_{39}$ représente un groupe alkyle en $C_1$-$C_{18}$ ou phényle, et
Y représente des atomes de fluor, de chlore, de brome ou d'iode, pour obtenir un composé de formule III

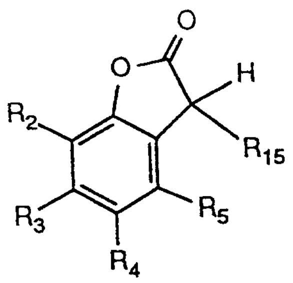

(III)

où les restes $R_2$, $R_3$, $R_4$, $R_5$ et $R_{15}$ sont définis comme à la revendication 1, dans le cas où $R_{15}$ = -OR'$_{15}$, $R'_{15}$ ne représentant pas un atome d'hydrogène.